# EUROPEAN PATENT APPLICATION

(11) **EP 4 417 686 A2**
(43) Date of publication of application: **21.08.2024**
(21) Application number: 24171802.2
(22) Date of filing: 13.02.2018
(51) Int. Cl.: C12N 5/077

(54) **POLYPEPTIDES, NUCLEIC ACID MOLECULES, CELLS, AND RELATED METHODS**

(30) Priority: 14.02.2017 US 201762458634 P
(62) Divisional of application: 18708011.4
(71) Applicant: Children's Hospital Medical Center, Cincinnati, OH 45229-3039 (US)
(72) Inventor: MILLAY, Douglas, Park Hills, KY, 41011 (US)
(74) Representative: Forresters IP LLP

(57) **Abstract**

Some embodiments of the invention include polypeptides comprising a myomerger polypeptide. Other embodiments of the invention include myomerger nucleic acid molecules encoding polypeptides comprising a myomerger polypeptide. Other embodiments of the invention include myomerger vectors comprising a myomerger nucleic acid molecule. Still other embodiments of the invention include modified cells comprising a myomerger nucleic acid molecule, a myomerger vector, or a myomerger polypeptide. Yet other embodiments of the invention include methods of making modified cells and methods of using modified cells. Additional embodiments of the invention are also discussed herein.

## Description

### CROSS-REFERENCE TO RELATED APPLICATIONS

This application claims the benefit of U.S. Provisional Application No. 62/458,634, filed February 14, 2017 entitled "INDUCTION OF CELL FUSION BY A NOVEL FUSION FACTOR" which is herein incorporated by reference in its entirety.

### GOVERNMENT RIGHTS

This invention was made with government support under NIH R01AR068286 awarded by the National Institutes of Health. The government has certain rights in the invention.

### BACKGROUND

The fusion of plasma membranes appears necessary for numerous biological processes from conception to the development of skeletal muscle, osteoclasts, trophoblasts, and giant cells. The molecular regulation of fusion is not as understood as it could be and the reconstitution of fusogenicity has not been achieved with mammalian proteins. Specifically, certain factors that participate in membrane coalescence have not been identified. Also, discoveries of specific fusion proteins and development of reconstitution systems have been historically helpful to decipher multiple types of membrane fusion; however, these systems are lacking for mammalian cellular fusion.

Myoblast fusion is a highly regulated process essential for muscle formation during development and regeneration. While numerous proteins have been shown to contribute to mammalian myoblast fusion, myomaker is the only known muscle-specific protein absolutely required for this process. Expression of myomaker in fibroblasts or mesenchymal stromal cells (MSCs) induces their fusion with muscle cells. Myomaker-expressing fibroblasts do not fuse to each other indicating that these cells harbor a competency to fuse, but only in the presence of a fusogenic cell (such as muscle cell). Thus, additional myocyte factors that confer fusogenicity appear to be required for reconstitution of fusion in myomaker+ fibroblasts.

Certain embodiments of the invention address one or more of the deficiencies described above. For example, some embodiments of the invention include polypeptides comprising a myomerger polypeptide. Other embodiments of the invention include myomerger nucleic acid molecules encoding polypeptides comprising a myomerger polypeptide. Other embodiments of the invention include myomerger vectors comprising a myomerger nucleic acid molecule. Still other embodiments of the invention include modified cells comprising a myomerger nucleic acid molecule, a myomerger vector, or a myomerger polypeptide. Yet other embodiments of the invention include methods of making modified cells and methods of using modified cells. Additional embodiments of the invention are also discussed herein.

### SUMMARY

Some embodiments of the invention include a polypeptide comprising a myomerger polypeptide. In certain embodiments, the polypeptide is not a wt-myomerger polypeptide or is a mutant-myomerger polypeptide. In other embodiments, the polypeptide comprises at least one amino acid modification relative to a wt-myomerger polypeptide. In yet other embodiments, the polypeptide comprises at least one amino acid modification relative to a wt-myomerger polypeptide and the at least one amino acid modification is an insertion, a deletion, or a substitution. In still other embodiments, the polypeptide is selected from SEQ ID Nos: 32-38. In other embodiments, the polypeptide is not a polypeptide is selected from SEQ ID Nos: 32-38. In certain embodiments, the wt-myomerger polypeptide is selected from SEQ ID Nos: 32-38. In other embodiments, the polypeptide sequence has at least an 80% sequence identity to a wt-myomerger polypeptide. In yet other embodiments, the polypeptide sequence has at least a 90% sequence identity to a wt-myomerger polypeptide.

Some embodiments of the invention include a myomerger nucleic acid molecule encoding an inventive polypeptide (e.g., myomerger polypeptide) disclosed herein. In certain embodiments, the myomerger nucleic acid sequence has at least an 80% identity to one or more sequences selected from SEQ ID Nos: 39-49. In other embodiments, the myomerger nucleic acid sequence encoding the polypeptide is selected from SEQ ID NO: 39-49. In still other embodiments, the myomerger nucleic acid sequence is a cDNA, or the myomerger nucleic acid sequence is not SEQ ID NO: 46, SEQ ID NO: 47, SEQ ID NO: 48, or SEQ ID NO: 49. In yet other embodiments, the myomerger nucleic acid molecule is in a cell, an insect cell, a mammalian cell, a human cell, or an sf9 insect cell. In some embodiments, the myomerger nucleic acid molecule is in a non-muscle cell, a muscle cell, a fibroblast, a mesenchymal stem cell (MSC), a hematopoietic stem cell, a blood cell, a bone marrow cell, or an adipose stem cell. In other embodiments, the myomerger nucleic acid molecule is in a modified cell. In yet other embodiments, the myomerger nucleic acid molecule is included in a vector, a viral vector, or a plasmid.

Some embodiments of the invention include a myomerger vector comprising a myomerger nucleic acid molecule disclosed herein.

Some embodiments of the invention include a modified cell comprising a myomerger nucleic acid molecule or a myomerger vector. In some embodiments, the myomerger nucleic acid molecule is exogenous. In other embodiments, the modified cell further comprises a myomaker nucleic acid molecule and at least one modification of the modified cell was the addition of a myomerger nucleic acid molecule or a myomerger vector. In other embodiments, the modified cell further comprises a myomaker nucleic acid molecule. In other embodiments, the modified cell further comprises a myomaker nucleic acid molecule, where optionally at least one modification of the modified cell was the addition of the myomaker nucleic acid molecule. In other embodiments, the modified cell further comprises a myomaker nucleic acid molecule, where at least one modification of the modified cell was the addition of the myomaker nucleic acid molecule. In certain embodiments, the modified cell is an insect cell, a mammalian cell, or a human cell. In yet other embodiments, the modified cell is a non-muscle cell, a muscle cell, a fibroblast, a mesenchymal stem cell (MSC), a hematopoietic stem cell, a blood cell, a bone marrow cell, or an adipose stem cell. In still other embodiments, at least part of the myomerger nucleic acid molecule is under control of a promoter. In certain embodiments, the promotor is a constitutive promoter, a synthetic promoter, an inducible promotor, a tissue specific promoter, a chemically regulated promotor, or a physically regulated promoter. In yet other embodiments, the modified cell comprises a myomerger polypeptide, a myomaker polypeptide, or both, and prior to modification the modified cell did not comprise a myomaker polypeptide, a myomerger polypeptide, or both. In some embodiments, the modified cell comprises a myomaker nucleic acid molecule. In other embodiments, the myomaker nucleic acid molecule is exogenous. In certain embodiments, the modification to the modified cell comprises one or more of (a) diminishing the effect of a first nucleic acid molecule, (b) addition of a second nucleic acid molecule encoding a myomaker polypeptide, or (c) addition of a third nucleic acid molecule encoding a myomerger polypeptide. In other embodiments, the modified cell (a) is a cell (e.g., a muscle cell or a non-muscle cell) that has a diminished effect of a first nucleic acid molecule, (b) a cell (e.g., a muscle cell or a non-muscle cell) that has a diminished effect of a first myomerger nucleic acid molecule, (c) a cell (e.g., a muscle cell or a non-muscle cell) that has a diminished effect of a first myomaker nucleic acid molecule, (d) a cell (e.g., a muscle cell, a non-muscle cell, or a fibroblast) that has an addition of a second myomerger nucleic acid molecule, or (e) a cell (e.g., a muscle cell, a non-muscle cell, or a fibroblast) that has an addition of a third myomerger nucleic acid molecule and that has an addition of a second myomaker nucleic acid molecule, or (f) combinations thereof.

Some embodiments of the invention include a method of preparing a modified cell (e.g., as disclosed herein) comprising adding a myomerger nucleic acid molecule to a first cell. In other embodiments, the first cell is a cell that has been previously modified.

Some embodiments of the invention include a composition comprising an inventive polypeptide (e.g., as disclosed herein), a myomerger nucleic acid molecule, or a modified cell. In other embodiments, the amount of the inventive polypeptide, the myomerger nucleic acid molecule, or the modified cell is from about 0.0001% (by weight total composition) to about 99%.

Some embodiments of the invention include a pharmaceutical composition comprising an inventive polypeptide (e.g., as disclosed here), a myomerger nucleic acid molecule, or a modified cell. In other embodiments, the amount of the inventive polypeptide, the myomerger nucleic acid molecule, or the modified cell is from about 0.0001% (by weight total composition) to about 50%.

Some embodiments of the invention include a method for fusing two or more cells comprising contacting a first cell with a second cell to form a third cell. In some embodiments, the first cell is a modified cell comprising a first myomerger polypeptide and a first myomaker polypeptide; the second cell comprises a second myomaker polypeptide and optionally comprises a second myomerger polypeptide; and the third cell is a multinucleated cell. In certain embodiments, the second cell comprises the second myomerger polypeptide. In other embodiments, the first cell is a non-muscle cell, the second cell is a non-muscle cell, or both. In yet other embodiments, the first cell is a non-muscle cell and the second cell is a muscle cell. In still other embodiments, the second cell is an isolated muscle cell. In some embodiments, the second cell is a myoblast. In yet other embodiments, the second cell is a muscle cell and is part of a muscle or muscle tissue. In certain embodiments, the contacting occurs *in vitro* or the contacting occurs *in vivo.*

Some embodiments of the invention include a method for delivering a gene of interest comprising contacting a first cell with a second cell, which fuse to form a third cell. In certain embodiments, the first cell is a modified cell comprising a first myomerger polypeptide, a first myomaker polypeptide, and a gene of interest; the second cell comprises a second myomaker polypeptide and optionally comprises a second myomerger polypeptide; and the third cell is a multinucleated cell and the gene of interest is delivered to the third cell upon fusion of the first cell with the second cell. In some embodiments, the second cell comprises the second myomerger polypeptide. In other embodiments, the first cell is a non-muscle cell, the second cell is a non-muscle cell, or both. In still other embodiments, the first cell is a non-muscle cell and the second cell is a muscle cell. In yet other embodiments, the second cell is an isolated muscle cell. In certain embodiments, the second cell is a myoblast. In other embodiments, the second cell is a muscle cell and is part of a muscle or muscle tissue. In still other embodiments, the contacting occurs *in vitro* or the contacting occurs *in vivo.* In some embodiments, the contacting occurs *ex vivo* and the method further comprises implanting the third cell in an animal. In other embodiments, the second cell underexpresses the gene of interest, does not express the gene of interest, or expresses a defective version of the gene of interest. In still other embodiments, the delivery comprises an injection or an intramuscular injection. In certain embodiments, the delivery comprises an injection comprising the first cell, the second cell, or both, or the delivery comprises an intramuscular injection comprising the first cell, the second cell, or both. In yet other embodiments, the delivery further comprises one or more of the contacting steps.

Other embodiments of the invention are also discussed herein.

### BRIEF DESCRIPTION OF THE DRAWINGS

The following drawings form part of the present specification and are included to further demonstrate certain aspects of the present invention. The invention may be better understood by reference to one or more of these drawings in combination with the description of specific embodiments presented herein.

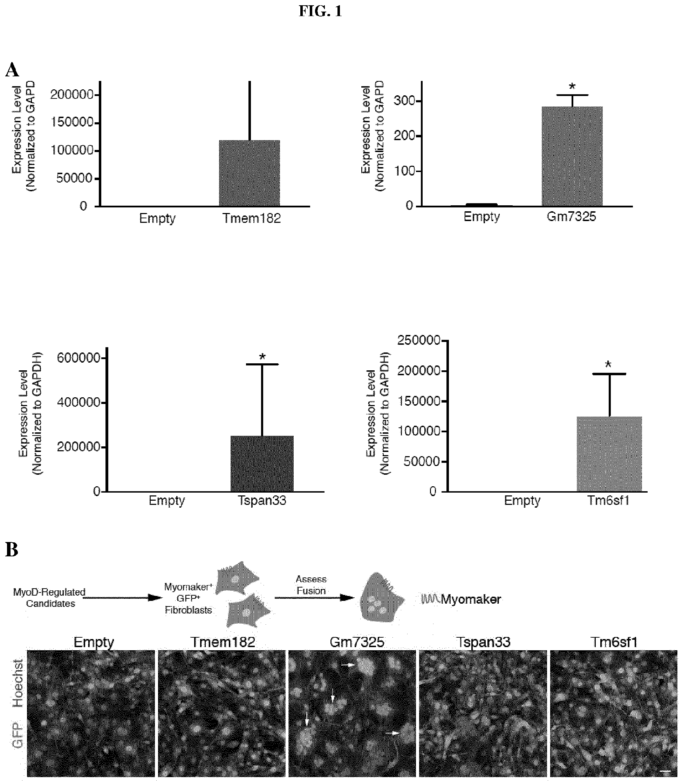

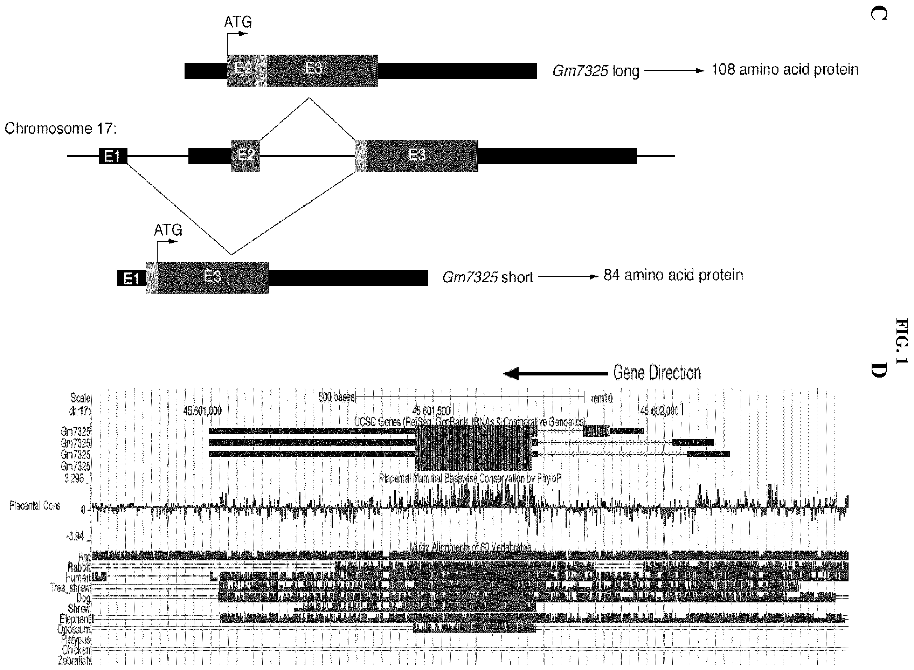

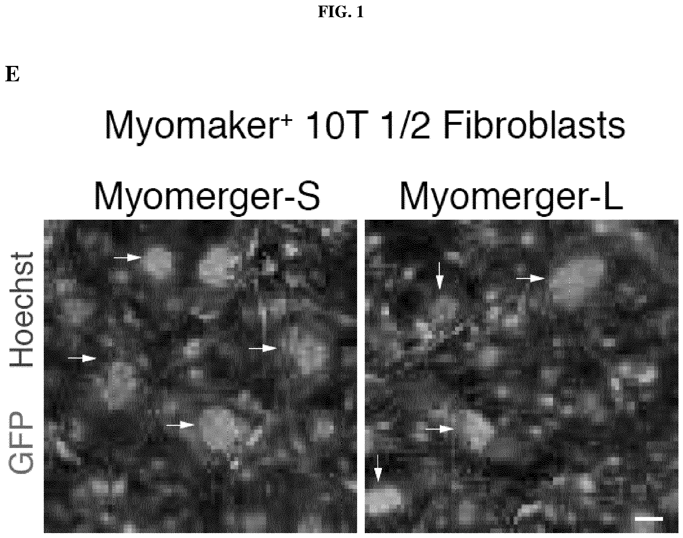

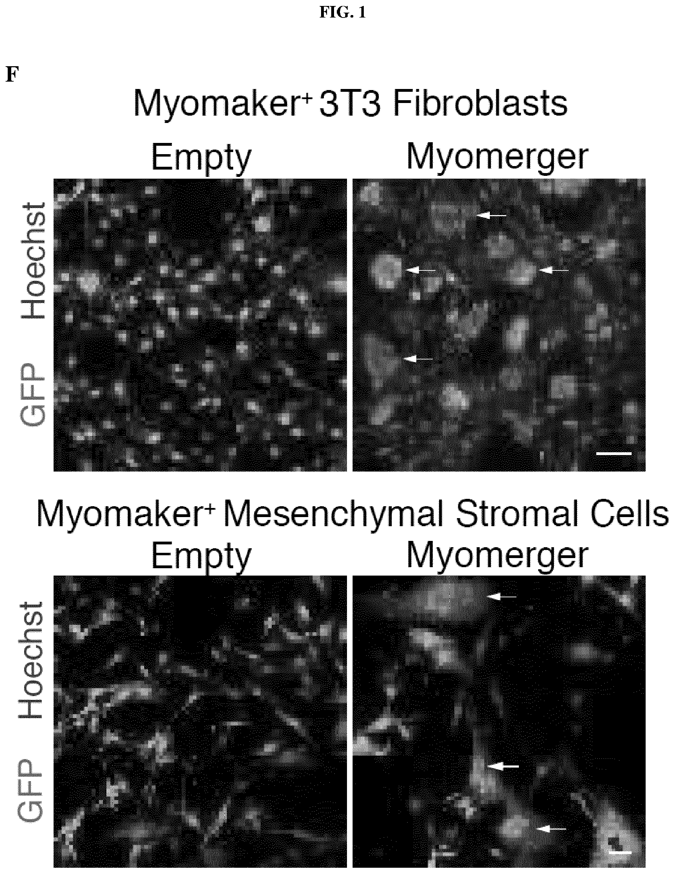

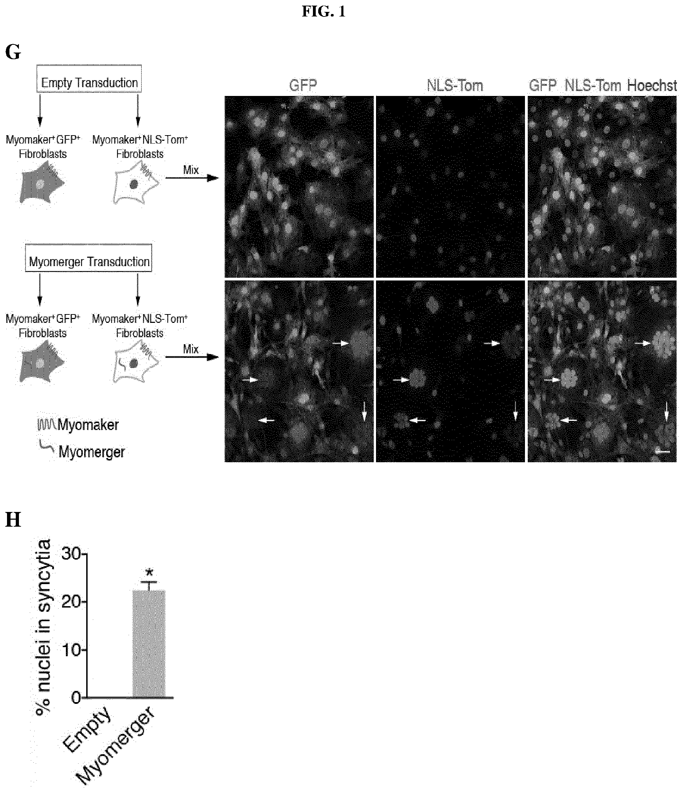

**FIG. 1****: Induction of fibroblast fusion by myomerger.** (A) Expression of MyoD-regulated genes in myomaker⁺ fibroblasts. qRT-PCR analysis for the indicated genes 72 hours after expression in fibroblasts. For *Gm7325,* we used primers specific for the long transcript. (B) Schematic showing a functional assay to screen for muscle genes that could activate fusion of GFP⁺ myomaker⁺ fibroblasts. Representative images of GFP⁺ cells and nuclei after expression of the indicated genes. Arrows depict cells with multiple nuclei. (C) Diagram showing the *Gm7325* locus on chromosome 17. The short transcript is generated by splicing of exon 1 (non-coding) with exon 3, leading to an 84 amino acid protein. The long transcript is produced by splicing of exon 2 with exon 3 and results in a 108 amino acid protein. (D) UCSC genome browser track showing multiple transcripts and conservation across vertebrate species. The short transcript is highly conserved in multiple species, including human, but not present in zebrafish. The upstream exon that produces the longer transcript is not highly conserved. Note that this annotation displays the gene on the reverse strand. (E) The short (S) or long (L) myomerger transcripts were expressed in myomaker⁺ 10T ½ fibroblasts and both induced fusion (n=3). (F) Myomerger also induced fusion of myomaker⁺ NIH/3T3 fibroblasts (n=3) and myomaker⁺ mesenchymal stromal cells (n=3). (G) Illustration of cell mixing approach to show fusion between the populations of fibroblasts. Co-localization of GFP and NLS-TdTomato (NLS-Tom) in the nucleus represents fusion. Representative images demonstrate fusion of myomaker⁺ myomerger⁺ fibroblasts but not empty-infected myomaker⁺ fibroblasts. Arrows indicate fusion between GFP⁺ and NLS-Tom⁺ fibroblasts. (H) The percentage of nuclei in syncytia after expression of empty or myomerger (*n*=3). Data are presented as mean ± SEM. **P*<0.05 compared to empty using an unpaired t-test. Arrows indicate fusion. Scale bars, 50 µm.

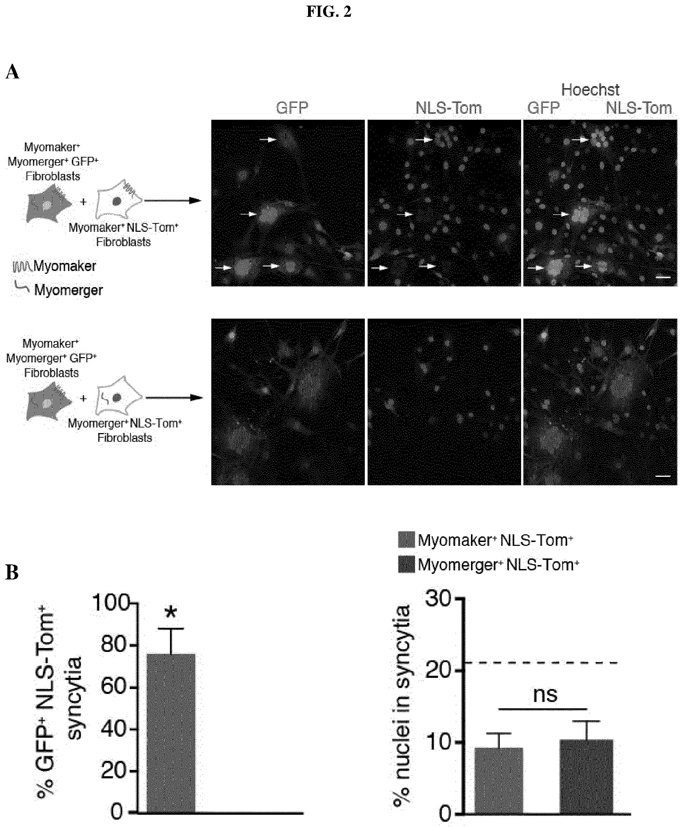

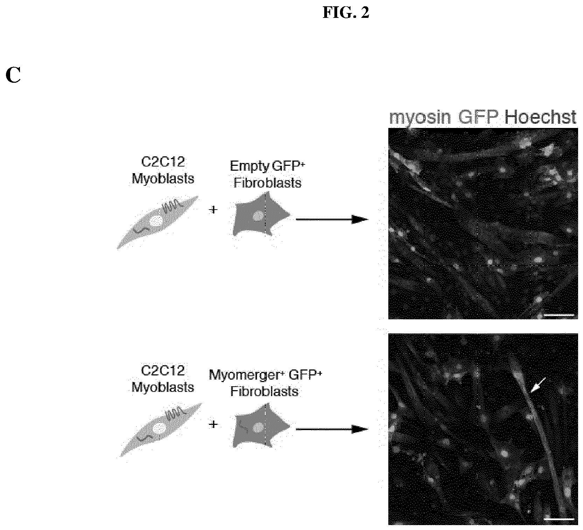

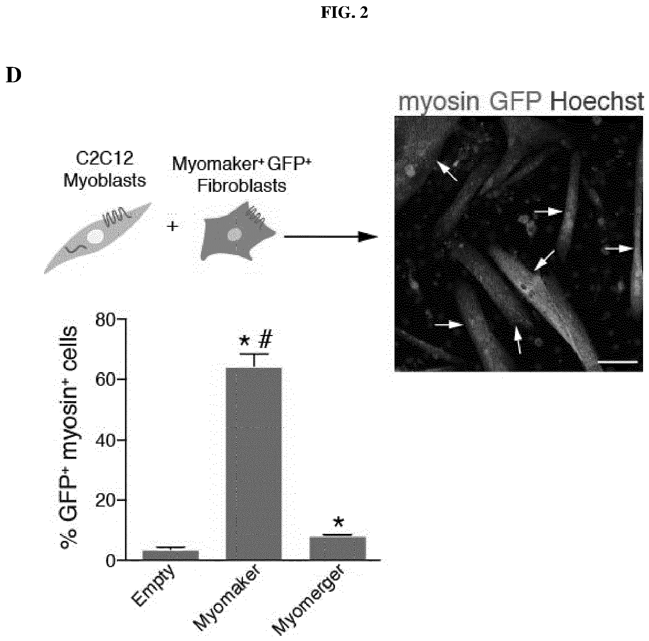

**FIG. 2****: Role of myomerger and myomaker in cell fusion.** (A) Diagram showing the cell mixing approach to assess fusion between the populations of fibroblasts. Co-localization of GFP and NLS-TdTomato (NLS-Tom) in the nucleus represents fusion (arrows). Representative images demonstrate fusion of myomaker⁺ myomerger⁺ GFP⁺ fibroblasts with myomaker⁺NLS-Tom⁺fibroblasts but not myomerger⁺NLS-Tom⁺fibroblasts. (B) Quantification of the percent of GFP⁺ NLS-Tom⁺ syncytia and the percent of nuclei in syncytia (*n*=3). Dotted line on right panel represents fusion achieved when both cells express both myomaker and myomerger (from FIG. 1B). (C & D) Heterologous fusion experiment between C2C12 myoblasts and GFP⁺ fibroblasts infected with either empty, myomaker, or myomerger. Representative immunofluorescent images to visualize co-localization of myosin and GFP (arrows), indicating fusion. Quantification of the percentage of GFP⁺ myosin⁺ cells (*n*=3). Data are presented as mean ± SEM. **P*<0.05 compared to myomerger⁺NLS-Tom⁺fibroblasts in (B) or empty in (C). ^{#}*P*<0.05 between myomaker and myomerger. An unpaired t-test was used to determine significance. Scale bars, 50 µm (A), 100 µm (C).

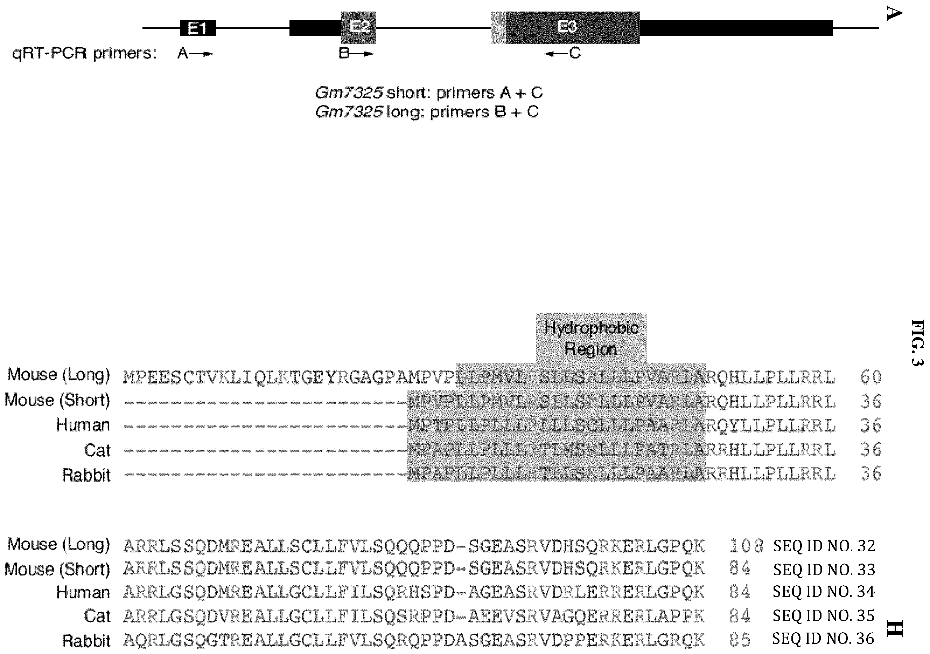

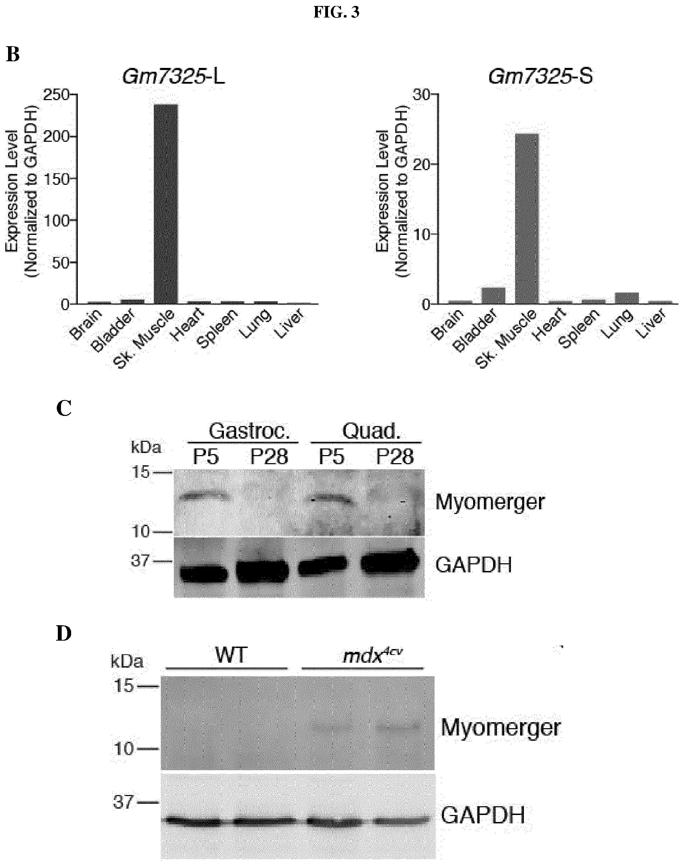

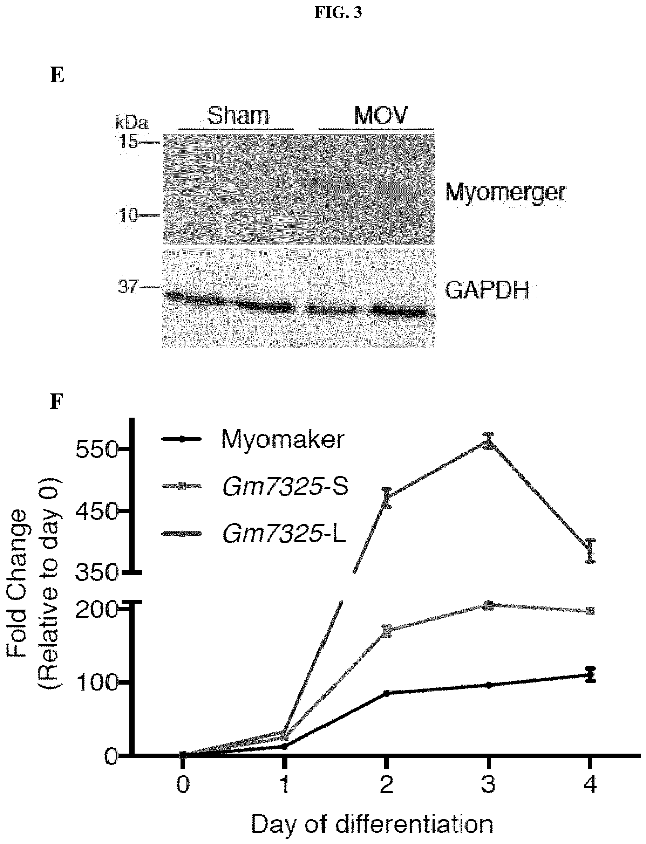

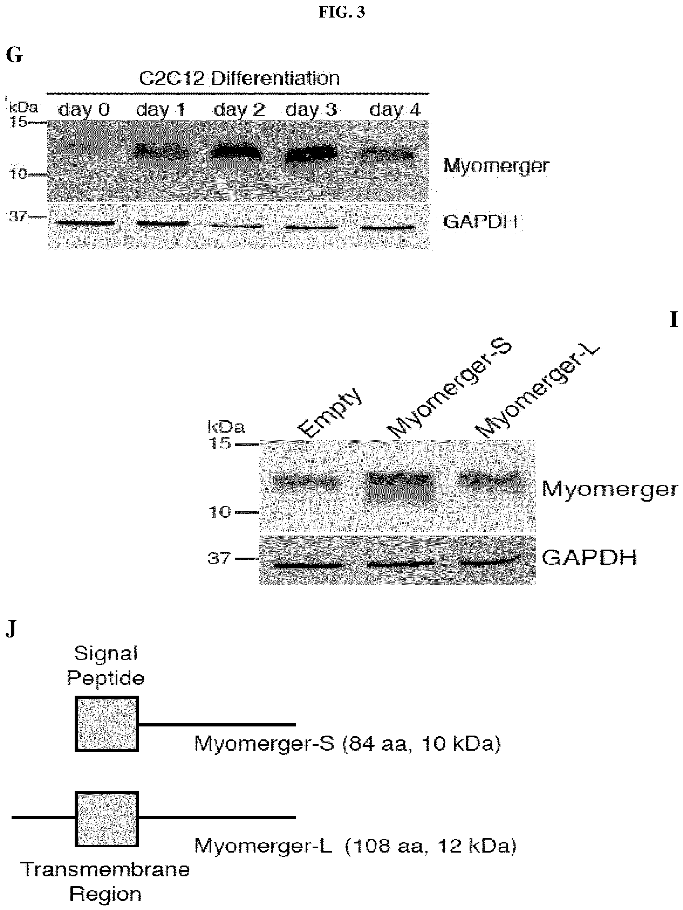

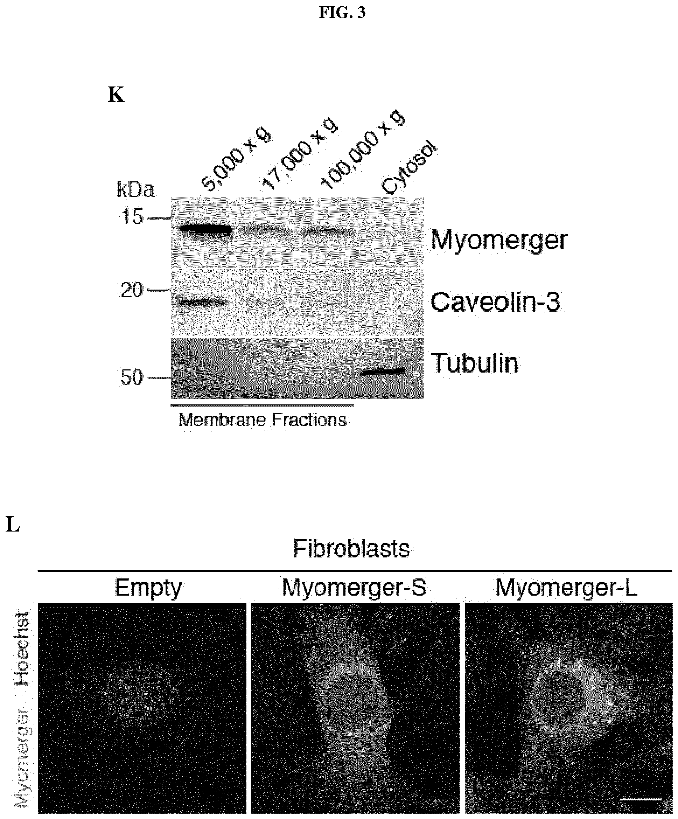

**FIG. 3****: Design of qRT-PCR primers and comparison of myomerger protein variants & Muscle-specific expression and regulation of myomerger.** (A) Schematic showing the location of primers to distinguish short and long transcripts. (B) qRT-PCR for both *Gm7325* long (L) and short (S) transcripts from various postnatal (P) day 5 tissues. (C) Immunoblotting for myomerger comparing P5 muscle to P28 muscle. (D) Immunoblotting for myomerger comparing WT to *mdx^{4cv}* diaphragms (8 weeks of age). (E) Immunoblotting for myomerger comparing sham plantaris to mechanically overloaded (MOV) plantaris (3 months of age). (F) qRT-PCR for *Gm7325* transcript variants and myomaker in C2C12 cells on the indicated days of differentiation (n=3 for each time point). (G) Immunoblotting for myomerger during C2C12 myoblast differentiation. GAPDH was used as a loading control. (H) Sequence alignment of both mouse myomerger protein products with multiple mammalian orthologs using Clustal Omega. A potential hydrophobic region is highlighted in gray. (I) Immunoblotting from C2C12 cells infected with either empty, myomerger-short (S), myomerger-long (L) on day 2 of differentiation. Myomerger migrates as a single band around 12 kDa when endogenously produced (empty). Over-expression of myomerger-S leads to an increase in the endogenous band and a lower band is also detected suggesting that myomerger transcripts may be subjected to intricate mRNA processing or post-translational modifications. (J) Graphic showing the regions of myomerger-S and myomerger-L as predicted by SignalP and Phobius. (K) Fractionation of C2C12 lysates on day 2 of differentiation followed by immunoblotting. (L) Representative immunostaining of fibroblasts infected with either empty, myomerger-short (S), or myomerger-long (L). Scale bar, 10 µm.

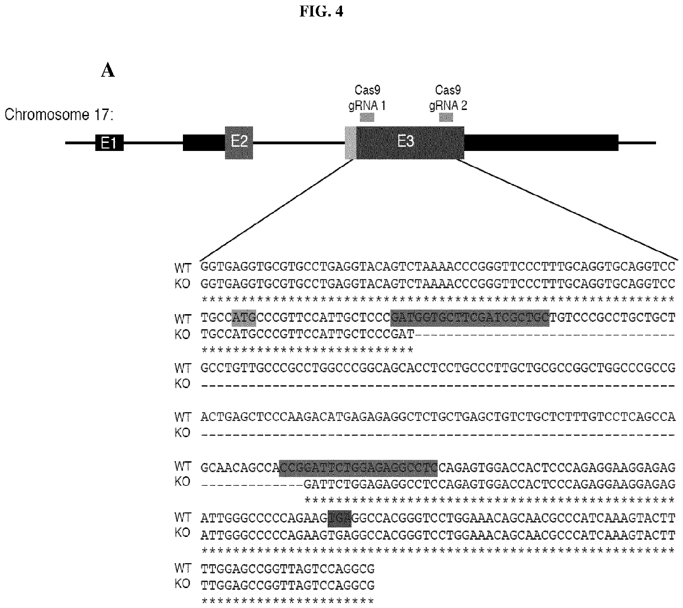

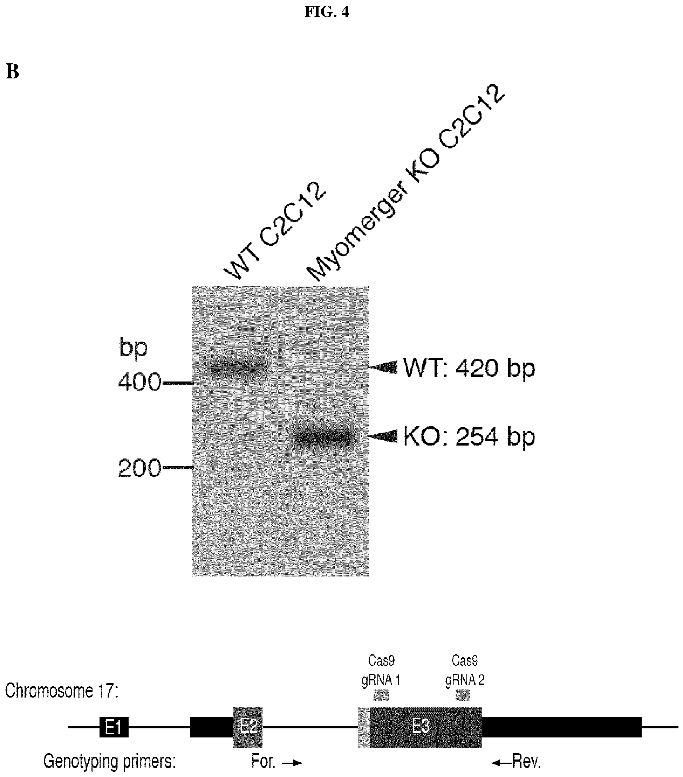

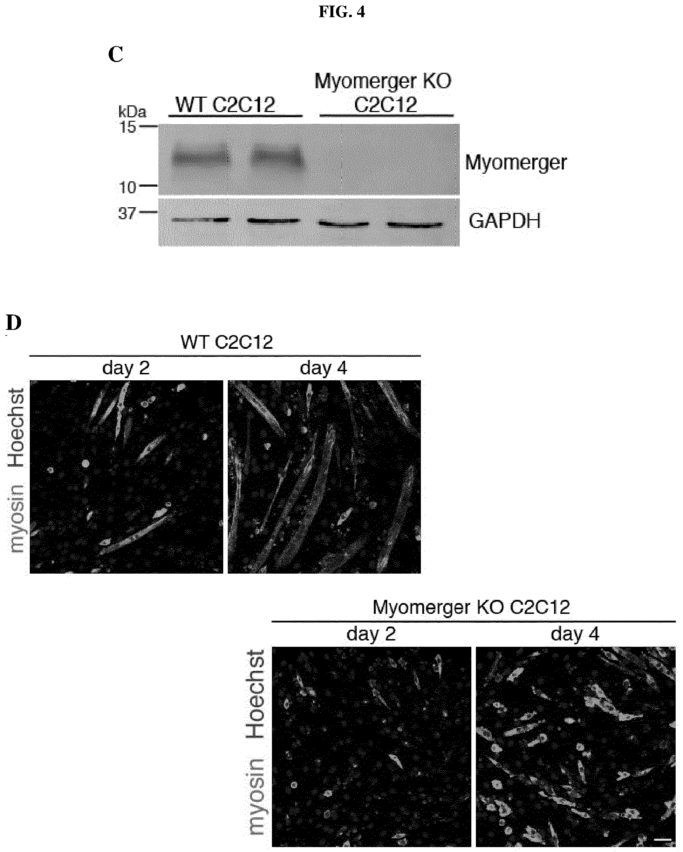

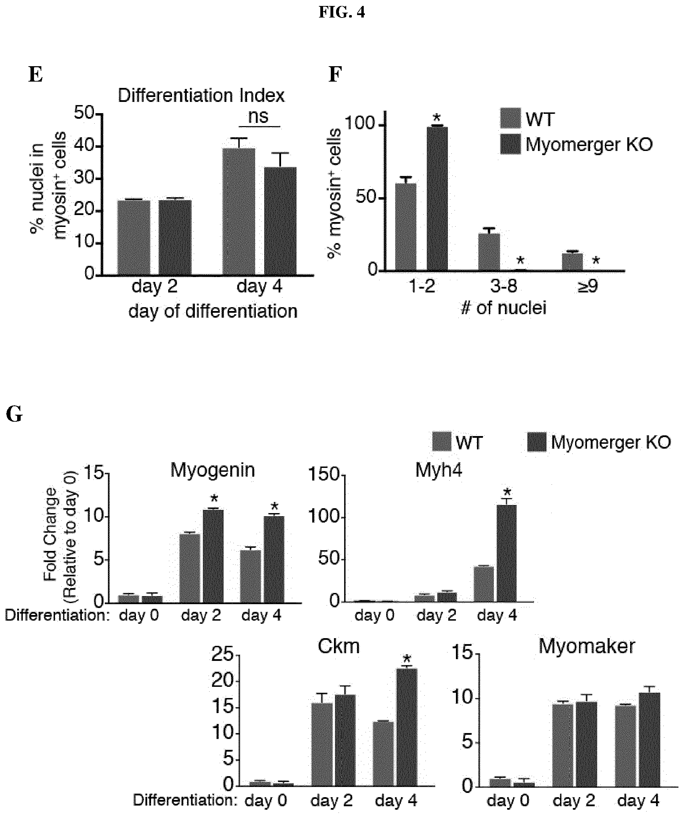

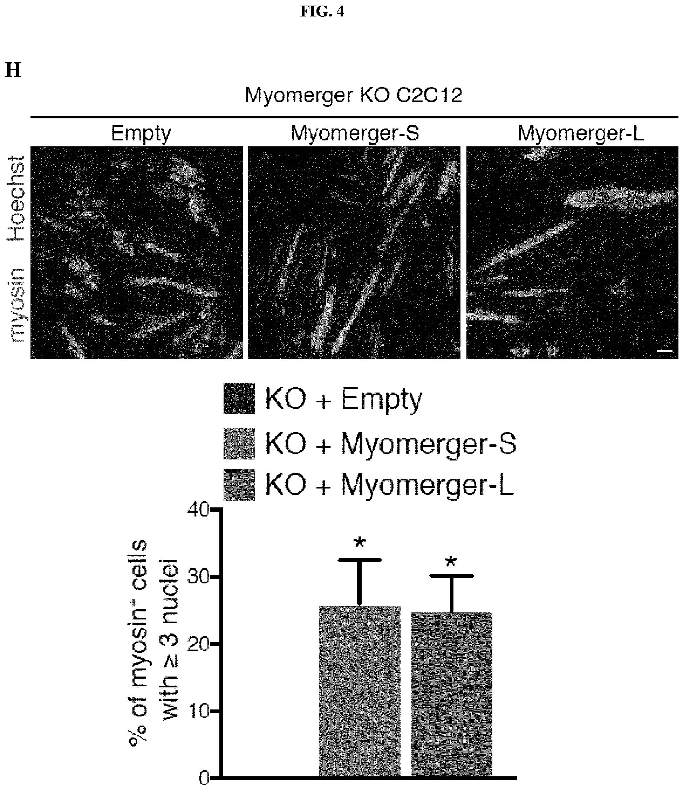

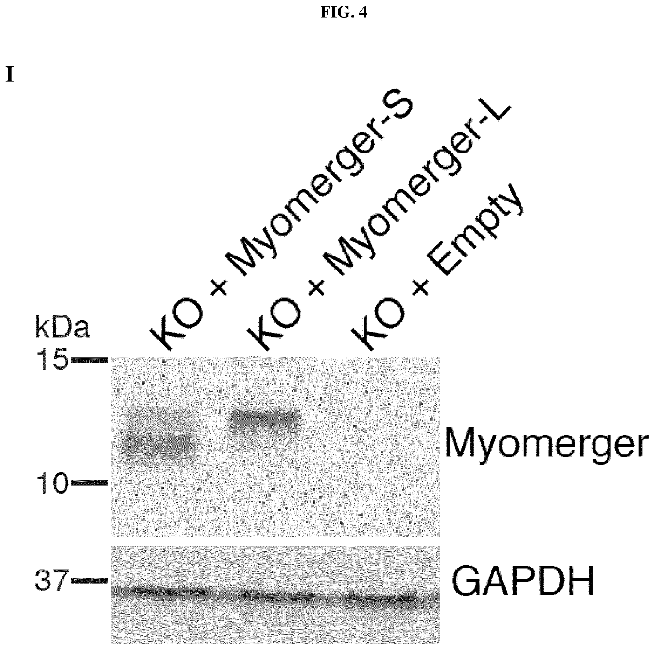

**FIG. 4****: CRISPR/Cas9 disruption of the *Gm7325* locus & Role of myomerger in myoblast fusion *in vitro.*** (A) Schematic showing the *Gm7325* locus and targeting of sgRNAs. (B) Genotyping strategy for myomerger KO C2C12 cells. WT and KO PCR products were sequenced and the result is shown in (A). The use of two sgRNAs results in reproducible cut sites leading to a 166 base pair deletion in both C2C12 cells and mice. The translational start site (ATG, green) for myomerger-S and stop site (TGA, red) for both myomerger-S and myomerger-L are noted. (C) Immunoblotting for myomerger in WT and myomerger KO C2C12 cells on day 2 of differentiation. GAPDH was used as a loading control. (D) Representative immunofluorescence images on day 2 and day 4 of differentiation for WT and myomerger KO C2C12 cells. Myomerger KO cells differentiate but fail to fuse. (E) Quantification of the differentiation index, the percentage of nuclei in myosin⁺ cells (*n*=4). ns, not significant. (F) The percentage of myosin⁺ cells that contain 1-2, 3-8, or ≥9 nuclei after 4 days of differentiation, as an indicator of fusogenicity (*n*=3). (G) qRT-PCR for the indicated myogenic transcripts (*n*=4). (H) Myomerger KO C2C12 cells were infected with either empty, myomerger-S, or myomerger-L and induced to differentiate. Both myomerger-S and myomerger-L rescued the lack of fusion in myomerger KO cells. Quantification of the fusion index, calculated as the percentage of myosin⁺ cells with ≥ 3 nuclei. Data are presented as mean ± SEM. **P*<0.05 compared to Empty using an unpaired t-test. (I) Immunoblotting for myomerger shows appropriate expression after transduction of myomerger KO cells. Data are presented as mean ± SEM. **P*<0.05 compared to WT using an unpaired t-test. Scale bar, 50 µm.

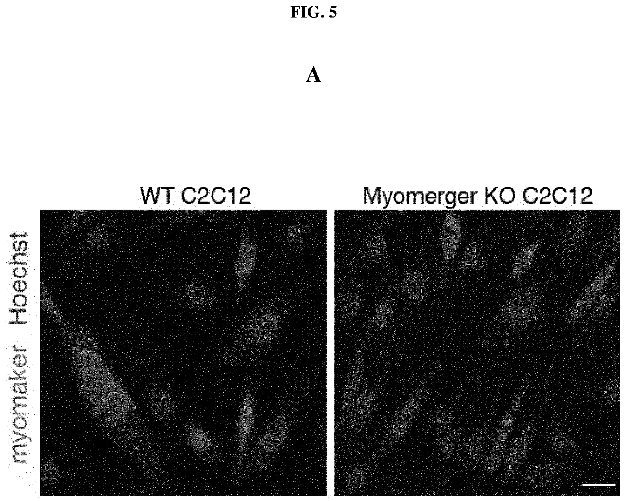

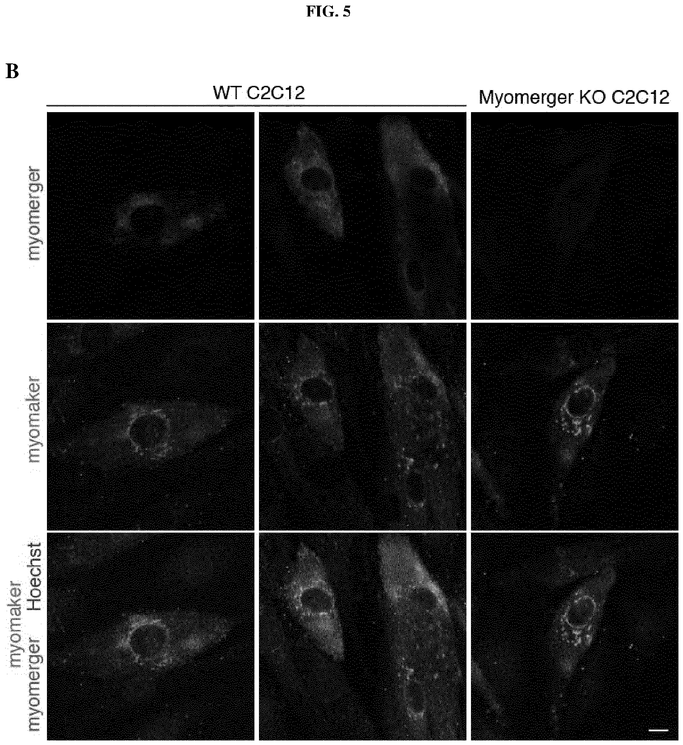

**FIG. 5****: Analysis of myomaker and myomerger co-localization.** (A) Representative immunofluorescence images from WT and myomerger KO C2C12 cells on day 2 of differentiation indicating that loss of myomerger does not alter myomaker expression or localization. (B) Immunofluorescence for myomerger and myomaker on the indicated cells on day 2 of differentiation. These two fusion proteins exhibit different localization patterns. Scale bars, 10 µm A, 5 µm B.

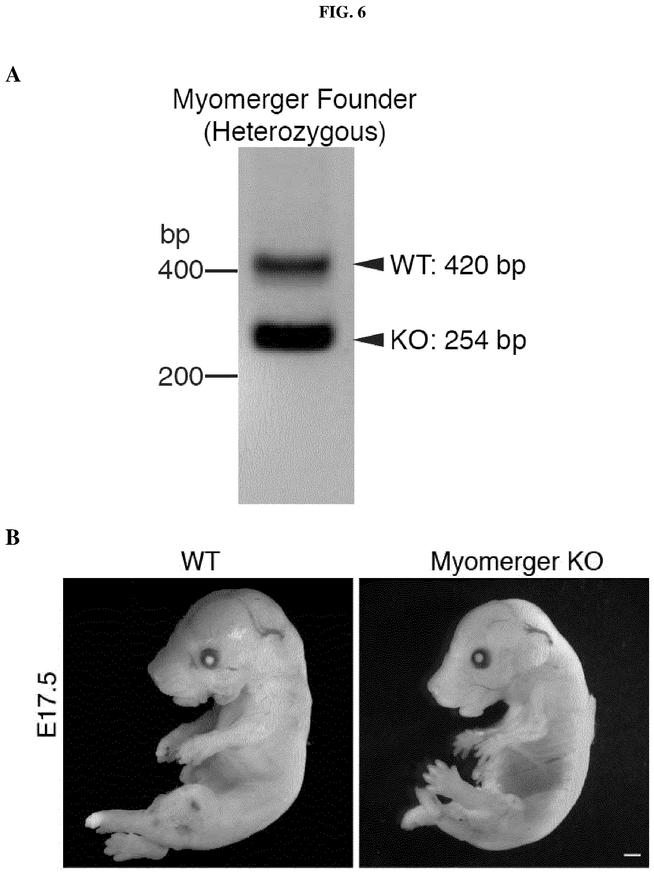

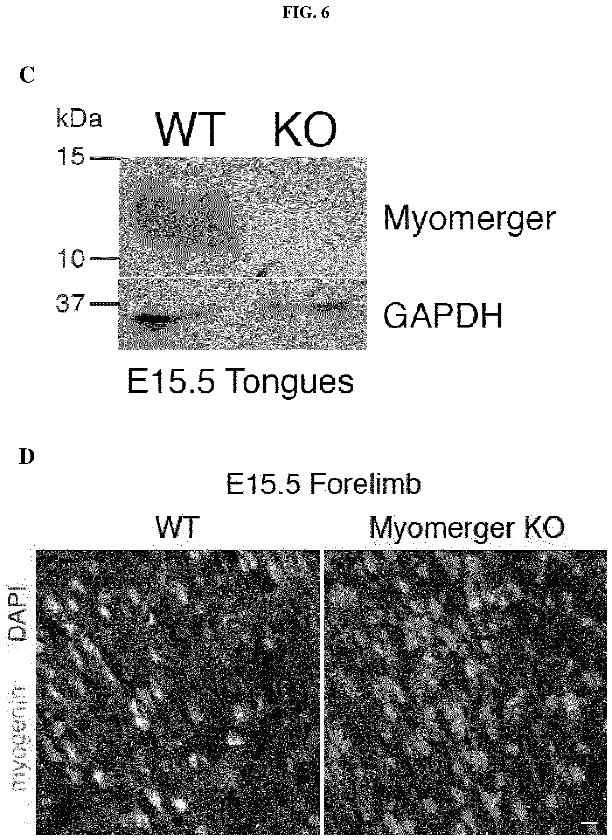

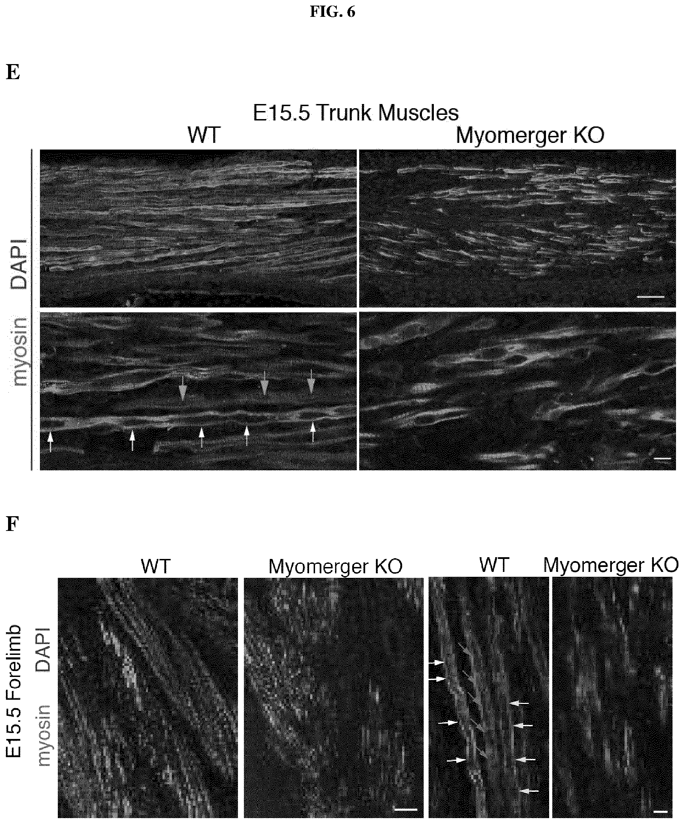

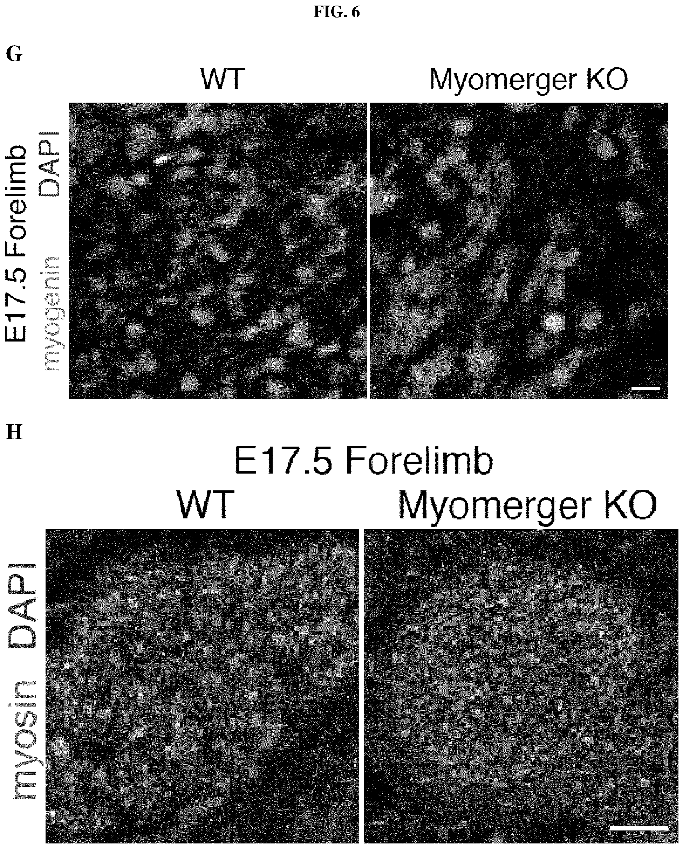

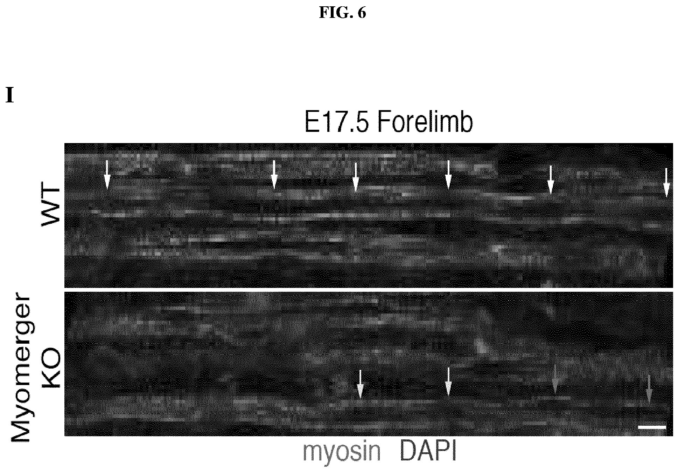

**FIG. 6****: Examination of myomerger KO muscle & Role of myomerger in myoblast fusion and muscle formation during embryonic development.** (A) Genotyping of the one founder harboring the *Gm7325* mutation generated through Cas9-mutagensis. (B) Representative whole-mount images of WT and myomerger KO E17.5 embryos showing improper skeletal muscle formation in KO embryos (*n*=4). (C) Immunoblotting on tongue lysates from WT and myomerger KO mice showing lack of myomerger in KO samples. GAPDH was used as a loading control. (D) Immunofluorescence images for myogenin from WT and myomerger KO E15.5 forelimbs demonstrating that myomerger does not appear to be required for myogenic activation (*n*=3). (E) Myosin immunofluorescence on the indicated E15.5 trunk muscles (*n*=3). Multi-nucleated myofibers (arrows of same color show nuclei within one myofiber) were observed in WT sections. Myomerger KO myocytes were myosin⁺ with sarcomeres but remained mono-nucleated. (F) E15.5 forelimbs (n=3) immunostained with a myosin antibody demonstrates that myomerger KO myoblasts differentiate but are unable to fuse. Arrows of same color show nuclei within same fiber (G, H & I) E17.5 forelimbs from WT and myomerger KO mice were evaluated for myogenin and myosin expression, and multi-nucleation. Arrows of same color in (I) show nuclei within one myofiber. We observed myocytes in myomerger KO samples that contained two nuclei (arrows). The nuclei labeled by the yellow and pink arrows are within different myofibers. Scale bars - 1 mm: B; 50 µm: E top panels, F left panels, H; 10 µm: D, E bottom panels, F right panels, G, I.

### DETAILED DESCRIPTION

While embodiments encompassing the general inventive concepts may take diverse forms, various embodiments will be described herein, with the understanding that the present disclosure is to be considered merely exemplary, and the general inventive concepts are not intended to be limited to the disclosed embodiments.

Some embodiments of the invention include polypeptides comprising a myomerger polypeptide. Other embodiments of the invention include myomerger nucleic acid molecules encoding polypeptides comprising a myomerger polypeptide. Other embodiments of the invention include myomerger vectors comprising a myomerger nucleic acid molecule. Still other embodiments of the invention include modified cells comprising a myomerger nucleic acid molecule, a myomerger vector, or a myomerger polypeptide. Yet other embodiments of the invention include methods of making modified cells and methods of using modified cells. Additional embodiments of the invention are also discussed herein.

### Inventive Polypeptides, Nucleic Acid Molecules, and Compositions

Some embodiments of the invention include inventive polypeptides comprising a myomerger polypeptide. In some embodiments, the myomerger polypeptide can be defined as a polypeptide that (a) induces fusogenicity (e.g., by inducing the fusion of myomaker-expressing fibroblasts), (b) can confer fusogenic activity to normally non-fusogenic cells, (c) is expressed during developmental myogenesis, (d) is expressed during regenerative myogenesis, (e) is expressed only during developmental myogenesis, (f) is expressed only during regenerative myogenesis, or (g) combinations thereof. The term "myomerger polypeptide" encompasses "wt-myomerger polypeptides" (i.e., myomerger polypeptides found in nature without any purposely human-made modification) and "mutant myomerger polypeptides" (e.g., with one or more modifications made to a wt-myomerger polypeptide). Nonlimiting examples of wt-myomerger polypeptides are found in Table 1A. In other embodiments, the myomerger polypeptide has at least one amino acid modification relative to a wt-myomerger polypeptide. A wt-myomerger polypeptide can, in some embodiments, be a myomerger polypeptide from any animal including but not limited to a mammal, a rat, a cat, a rabbit, a human, a cow, a chicken, a turkey, a monkey, a tree shrew, a dog, a pig, a shrew, an elephant, or an opossum. Table 1A provides nonlimiting examples of wt-myomerger polypeptides and Tables 1B and 1C provide nonlimiting examples of related nucleic acid sequences (including start and stop codons).

**Table 1A**

| **Source** | **Polypeptide sequence** |
|---|---|
| Mouse (long) | MPEESCTVKLIQLKTGEYRGAGPAMPVPLLPMVLRSLLSRLLLPVARLA RQHLLPLLRRLARRLSSQDMREALLSCLLFVLSQQQPPDSGEASRVDHS QRKERLGPQK (SEQ ID NO: 32) |
| Mouse (short) | MPVPLLPMVLRSLLSRLLLPVARLARQHLLPLLRRLARRLSSQDMREAL LSCLLFVLSQQQPPDSGEASRVDHSQRKERLGPQK (SEQ ID NO: 33) |
| Human | MPTPLLPLLLRLLLSCLLLPAARLARQYLLPLLRRLARRLGSQDMREAL LGCLLFILSQRHSPDAGEASRVDRLERRERLGPQK (SEQ ID NO: 34) |
| Cat | MPAPLLPLLLRTLMSRLLLPATRLARRHLLPLLRRLARRLGSQDVREAL LGCLLFILSQSRPPDAEEVSRVAGQERRERLAPPK (SEQ ID NO: 35) |
| Rabbit | MPAPLLPLLLRTLLSRLLLPAARLARRHLLPLLRRLAQRLGSQGTREALL GCLLFVLSQRQPPDASGEASRVDPPERKERLGRQK (SEQ ID NO: 36) |
| Dog | MPAPLLPLLLRTLVSRLLLPAARLARRHLLPLLRGLARRLGSQEVREAL LGCLLFILSQRHPPDAEEASRVAGQERKERLAPPK (SEQ ID NO: 37) |
| Elephant | MPVPLLSLLLRALLSRLLLPAARLARQHLLPLLRRLARRLGSQDMRQAL LGCLLFVLSQQHPPDAGEASREALSERRGRLAPQK (SEQ ID NO: 38) |

**Table 1B**

| **Source** | **cDNA nucleic acid sequence** |
|---|---|
| Mouse (long) | atgcc agaagaaagc tgcactgtaa aactaatcca gttgaaaact ggggagtaca gaggtgcagg tcctgccatg cccgttccat tgctcccgat ggtgcttcga tcgctgctgt cccgcctgct gctgcctgtt gcccgcctgg cccggcagca cctcctgccc ttgctgcgcc ggctggcccg ccgactgagc tcccaagaca tgagagaggc tctgctgagc tgtctgctct ttgtcctcag ccagcaacag ccaccggatt ctggagaggc ctccagagtg gaccactccc agaggaagga gagattgggc ccccagaagt ga (SEQ ID NO: 39) |
| Mouse (short) | atgcccg ttccattgct cccgatggtg cttcgatcgc tgctgtcccg cctgctgctg cctgttgccc gcctggcccg gcagcacctc ctgcccttgc tgcgccggct ggcccgccga ctgagctccc aagacatgag agaggctctg ctgagctgtc tgctctttgt cctcagccag caacagccac cggattctgg agaggcctcc agagtggacc actcccagag gaaggagaga ttgggccccc agaagtga (SEQ ID NO: 40) |
| Human | atgcccac gccactgctc ccgctgctgc ttcgattgct gctgtcctgc ctgctgctgc ctgctgcccg cctggcccgc caatacctcc tgcccctgct gcgccgattg gcccgccgcc tgggctccca ggacatgcga gaggctttgc tgggctgtct gctgttcatt ctcagccagc gacactcgcc agacgctggg gaggcctcaa gagtggaccg cctggagagg agggagaggt taggccccca aaagtga (SEQ ID NO: 41) |
| Cat | atgcccgc tccactgctc ccactgctgc ttcgaaccct gatgtcccgc ttgctgctgc ctgccacccg cctggcccgc cggcacctcc tgcccctcct gcgccgactg gcccgccgcc tgggctcgca ggatgttcga gaagctttgc tgggctgtct gttgttcatc ctcagccaga gccgcccgcc cgacgctgag gaggtctcca gagtggctgg ccaggagagg agggagaggc tagctccccc aaaatga (SEQ ID NO: 42) |
| Rabbit | atgcc tgcccccctg ctgccgctgc tgctgcgaac gctgctgtcc cgtctgctgc tgcccgctgc ccgcctggcc cgccggcacc tcctgcccct gctgcgccga ctggctcaac gcctgggctc ccagggcacg cgcgaggctt tgctgggctg tttgctgttt gtcctcagcc agagacagcc gccagatgcc tctggggagg cctccagagt ggacccaccg gagaggaagg agaggttagg ccgccaaaag tga (SEQ ID NO: 43) |
| Dog | atgc ctgctccact gctcccactg ctgctgcgaa cgctggtgtc tcgcctgctg ctgcctgctg cccgcctggc ccggcggcac ctcctgcccc tgctgcgtgg actggcccgc cgcctaggct cgcaggaggt tcgagaggct ttgctgggct gtctgttgtt catcctcagc cagagacatc cgccggacgc cgaggaggcc tccagagtgg ctggccagga gaggaaggag aggctagctc cccccaaatg a (SEQ ID NO: 44) |
| Elephant | atgcccgtcc cgctgctctc gctgctgctg cgcgcgctgc tgtcccgcct gctgctgcct gctgcccgcc tggcccgcca gcacctcctg cccctcctgc gccgacttgc tcgccgcctg ggctcccagg acatgcgaca ggctctcttg ggatgtctgc tctttgtcct cagccagcaa cacccgccgg acgctggtga ggcctccaga gaggccctct cagagaggag agggaggcta gccccccaaa agtga (SEQ ID NO: 45) |

**Table 1C (exons in lowercase)**

| **Source** | **Genomic nucleic acid sequence** |
|---|---|
| Human (+ strand) - start codon is bold & underlined; stop codon is bold and italicized | ctgcccggtgagagctgccgtggattggtggggGTAGGGGACTGAGAGGTCAGGGAGTGT CAGGTCAGGGTGGATCAGGAGCCCCAAAAGAAAAATTGAGAATTGCCTGGAGAAGAACTC CTGCTAGACTGAGGGAGAAGGGTTAGGGAACTCCAGGGGCATTGAGGCTGTGCAAGAGGA GGGGGTGACTAGAGGAAGGGAGGGGCCAGGGAGCAGTAGGAATGCCTGGAGCTGGGAACG GCAAGCTGTAGGTCTTGGTTTACTCTTGCCTTGGTTCAGTCTCCCCATCTGTGCTATGGT GAGAACCTTCCTGCCTCAGCTGCCTTGCCAAGAGAAAGGGCTTCATGAAAGCAAAAATGA CCTACAAATTGAGGTCAGGAGCAGGAAGGTGTAAACTGAAGGGAGGGGGAACTCCTGCCC ACCCCATGTCCTTGCCAGGTGAGGCAGAACCAGGACATGCAAGCCTAAAGTCTGTGTTGT CTTCCCAGgcactgactcactggccctgccatgcccacgccactgctcccgctgctgctt cgattgctgctgtcctgcctgctgctgcctgctgcccgcctggcccgccaatacctcctg cccctgctgcgccgattggcccgccgcctgggctcccaggacatgcgagaggctttgctg ggctgtctgctgttcattctcagccagcgacactcgccagacgctggggaggcctcaaga gtggaccgcctggagaggagggagaggttaggcccccaaaagtgaggccacaagtcctgg cagcagctgtatccacaaaatgctttcttttggagtaggataatcctggcaccagcactg accgaagcctgcccagtggacagaagatatagtgagggttgtgcatgagagggatctgcc acagacatgcctctccactcccaacagaaatgtctttctggaagaatgccttgcatctag cacaaaactgattattgcccctctgtcctccagcagttcctcccaaagaccactcctaat cacctctggcctcaggcgggaggggaactaacacccacccacccctgccctccctgcaaa tgggaacatcaaggttcccagtgcttaactgagggacaagtgacaatttagcagagaggc aagatttgaatccagactgtcttccagactcaggacctaccttaaaataatatctgagtt gcttatggaggcagacctgcctgcaaagcccagcactcagcaagtgctcaataaatattt gatttgaattctttc (SEQ ID NO: 46) |
| Human (- strand, reverse complement) | gaaagaattcaaatcaaatatttattgagcacttgctgagtgctgggctttgcaggcagg tctgcctccataagcaactcagatattattttaaggtaggtcctgagtctggaagacagt ctggattcaaatcttgcctctctgctaaattgtcacttgtccctcagttaagcactggga accttgatgttcccatttgcagggagggcaggggtgggtgggtgttagttcccctcccgc ctgaggccagaggtgattaggagtggtctttgggaggaactgctggaggacagaggggca ataatcagttttgtgctagatgcaaggcattcttccagaaagacatttctgttgggagtg gagaggcatgtctgtggcagatccctctcatgcacaaccctcactatatcttctgtccac tgggcaggcttcggtcagtgctggtgccaggattatcctactccaaaagaaagcattttg tggatacagctgctgccaggacttgtggcctcacttttgggggcctaacctctccctcct ctccaggcggtccactcttgaggcctccccagcgtctggcgagtgtcgctggctgagaat gaacagcagacagcccagcaaagcctctcgcatgtcctgggagcccaggcggcgggccaa tcggcgcagcaggggcaggaggtattggcgggccaggcgggcagcaggcagcagcaggca ggacagcagcaatcgaagcagcagcgggagcagtggcgtgggcatggcagggccagtgag tcagtgcCTGGGAAGACAACACAGACTTTAGGCTTGCATGTCCTGGTTCTGCCTCACCTG GCAAGGACATGGGGTGGGCAGGAGTTCCCCCTCCCTTCAGTTTACACCTTCCTGCTCCTG ACCTCAATTTGTAGGTCATTTTTGCTTTCATGAAGCCCTTTCTCTTGGCAAGGCAGCTGA GGCAGGAAGGTTCTCACCATAGCACAGATGGGGAGACTGAACCAAGGCAAGAGTAAACCA AGACCTACAGCTTGCCGTTCCCAGCTCCAGGCATTCCTACTGCTCCCTGGCCCCTCCCTT CCTCTAGTCACCCCCTCCTCTTGCACAGCCTCAATGCCCCTGGAGTTCCCTAACCCTTCT CCCTCAGTCTAGCAGGAGTTCTTCTC ATCCACCCTGACCTGACACTCCCTGA |
| Mouse (+ strand) | ccaataacaacacactgtcctcgtttattgactacctgctgcgtaccaagctttgaaagt actcattctttaacgggaagcaagggcttataattttaaggtagacgggacagtttggat ttaaataccacctcttagctaaattgtcttgagtctaagtgaaacatcatctcttaactg accttgatacccgcatttgcaggtccaccctggaggccagagataaggcagagggagctg cagagaggaagggtcaatcaacacaatctgtagcctgctaggagctaggggagtgggaac tgttcaggtcagagccctcttgcactcagcccggactgtcttcgcccactgggcagtctg ccgtccatgcccgtgcgtgcggaccgacgcctggactaaccggctccaaaagtactttga tgggcgttgctgtttccaggacccgtggcctcacttctgggggcccaatctctccttcct ctgggagtggtccactctggaggcctctccagaatccggtggctgttgctggctgaggac aaagagcagacagctcagcagagcctctctcatgtcttgggagctcagtcggcgggccag ccggcgcagcaagggcaggaggtgctgccgggccaggcgggcaacaggcagcagcaggcg ggacagcagcgatcgaagcaccatcgggagcaatggaacgggcatggcaggacctgcacC TGCAAAGGGAACCCGGGTTTTAGACTGTACCTCAGGCACGCACCTCACCTGGCAAAGCAG GGTGCGGGGGTGTGGAGTCCTCCCTTCAGCTTATACctctgtactccccagttttcaact ggattagttttacagtgcagctttcttctggcatgaaagctggttaaggagttcactcac tgttatcacagatgggaagggagcccagggctggaaggtggtggggactGAGGCTAGGGC CTTTTCCAGAACCCACTTCCTTTAATCCCTCCCTCCCTTTGCATACTCTGACctgaagcc tgaacttcttgccctcctgctcaccagttctaaccggccagtggcagctctcaccagtca gaactgctcagaatcaatttcaggatgcttttgcctgcggtggattcagcatcact (SEQ ID NO: 48) |
| Mouse (-strand, reverse complement) - start codon is bold & underlined; stop codon is bold and italicized | agtgatgctgaatccaccgcaggcaaaagcatcctgaaattgattctgagcagttctgac tggtgagagctgccactggccggttagaactggtgagcaggagggcaagaagttcaggct tcagGTCAGAGTATGCAAAGGGAGGGAGGGATTAAAGGAAGTGGGTTCTGGAAAAGGCCC TAGCCTCagtccccaccaccttccagccctgggctcccttcccatctgtgataacagtga gtgaactccttaaccagctttcatgccagaagaaagctgcactgtaaaactaatccagtt gaaaactggggagtacagagGTATAAGCTGAAGGGAGGACTCCACACCCCCGCACCCTGC TTTGCCAGGTGAGGTGCGTGCCTGAGGTACAGTCTAAAACCCGGGTTCCCTTTGCAGgtg caggtcctgccatgcccgttccattgctcccgatggtgcttcgatcgctgctgtcccgcc tgctgctgcctgttgcccgcctggcccggcagcacctcctgcccttgctgcgccggctgg cccgccgactgagctcccaagacatgagagaggctctgctgagctgtctgctctttgtcc tcagccagcaacagccaccggattctggagaggcctccagagtggaccactcccagagga aggagagattgggcccccagaagtgaggccacgggtcctggaaacagcaacgcccatcaa agtacttttggagccggttagtccaggcgtcggtccgcacgcacgggcatggacggcaga ctgcccagtgggcgaagacagtccgggctgagtgcaagagggctctgacctgaacagttc ccactcccctagctcctagcaggctacagattgtgttgattgacccttcctctctgcagc tccctctgccttatctctggcctccagggtggacctgcaaatgcgggtatcaaggtcagt taagagatgatgtttcacttagactcaagacaatttagctaagaggtggtatttaaatcc aaactgtcccgtctaccttaaaattataagcccttgcttcccgttaaagaatgagtactt tcaaagcttggtacgcagcaggtagtcaataaacgaggacagtgtgttgttattgg (SEQ ID NO: 49) |

One or more modifications, in some instances, can include an insertion, a deletion, a substitution, or combinations thereof. In certain embodiments, one or more modifications to a wt-myomerger polypeptide can comprise an insertion, such, but not limited to an insertion at the C-terminus or at the N-terminus of the wt-myomerger polypeptide. In some examples of the embodiments, an insertion can include (e.g., at the C-terminus, at the N-terminus, or at another place in the polypeptide) about 1, about 2, about 3, about 4, about 5, about 6, about 7, about 8, about 9, about 10, about 11, about 12, about 13, about 14, about 15, about 16, about 17, about 18, about 19, or about 20 amino acids (e.g., natural amino acids, or modified or unusual amino acids).

In some embodiments, the inventive polypeptide does not encompass one or more naturally occurring polypeptides (e.g., does not encompass one or more of the wt-myomerger polypeptides). In other embodiments, the inventive polypeptide does not encompass any of the wt-myomerger polypeptides. In some embodiments, the inventive polypeptide does not encompass any naturally occurring polypeptide (e.g., does not encompass any of the wt-myomerger polypeptides or any other naturally occurring polypeptide).

In some embodiments, one or more modifications to a wt-myomerger polypeptide can include one or more substitutions, one or more insertions, or one or more deletions (or combinations thereof) to one or more amino acids in a hydrophobic region of a wt-myomerger polypeptide, in a signal region of a wt-myomerger polypeptide, in a transmembrane region of a wt-myomerger polypeptide, or in a combination thereof. In some embodiments, one or more modifications to a wt-myomerger polypeptide can include one or more substitutions or one or more deletions (or combinations thereof) to one or more amino acids in a hydrophobic region of a wt-myomerger polypeptide, in a signal region of a wt-myomerger polypeptide, in a transmembrane region of a wt-myomerger polypeptide, or in a combination thereof.

In some embodiments, the inventive polypeptide can have a polypeptide sequence with an amino acid sequence identity to a wt-myomerger polypeptide (e.g., SEQ ID NO:32, SEQ ID NO:33, SEQ ID NO:34, and SEQ ID NO:36) of about 70%, about 75%, about 80%, about 85%, about 90%, about 91%, about 92%, about 93%, about 94%, about 95%, about 96%, about 97%, about 98%, about 99%, about 99.1%, about 99.2%, about 99.3%, about 99.4%, about 99.5%, about 99.6%, about 99.7%, about 99.8%, about 99.9%, about 99.95%, about 99.99%, less than about 100%, at least about 70%, at least about 80%, at least about 90%, at least about 95%, at least about 99%, or at least about 99.5%. In some embodiments, the inventive polypeptide sequence has an amino acid sequence identity to SEQ ID NO:32, SEQ ID NO:33, SEQ ID NO:34, or SEQ ID NO:36 of about 70%, about 75%, about 80%, about 85%, about 90%, about 91%, about 92%, about 93%, about 94%, about 95%, about 96%, about 97%, about 98%, about 99%, about 99.1%, about 99.2%, about 99.3%, about 99.4%, about 99.5%, about 99.6%, about 99.7%, about 99.8%, about 99.9%, about 99.95%, about 99.99%, less than about 100%, at least about 70%, at least about 80%, at least about 90%, at least about 95%, at least about 99%, or at least about 99.5%. The amino acid sequence identity (e.g., percent identity) can be determined by any suitable method, such as using BLAST, BLAST-2, ALIGN, ALIGN-2, Clustal Omega, or Megalign software. Unless otherwise indicated, the amino acid sequence identity (e.g., percent identity) is determined using BLAST-2.

In some embodiments, the inventive polypeptide has (e.g., as compared to a wt-myomerger polypeptide or as compared to the absence of a myomerger polypeptide) an increased ability to activate fusion, a decreased ability to activate fusion, an increased ability to confer fusogenicity, a decreased ability to confer fusogenicity, an increased level of expression during embryonic development, a decreased level of expression during embryonic development, an increased level of expression during myogenesis in adult organisms (e.g., older than embryonic), a decreased level of expression during myogenesis in adult organisms (e.g., older than embryonic), an increased level of induction of myogenesis in adult organisms (e.g., older than embryonic), a decreased of induction of myogenesis in adult organisms (e.g., older than embryonic), an increased affinity for membranes, a decreased affinity for membranes, an increased level of association with membrane compartment, a decreased level association with membrane compartment, or combinations thereof. In other embodiments, the inventive polypeptide has (e.g., as compared to a wt-myomerger polypeptide or as compared to the absence of a myomerger polypeptide) an increased ability to activate fusion, an increased ability to confer fusogenicity, an increased level of expression during embryonic development, an increased level of expression during myogenesis in adult organisms (e.g., older than embryonic), an increased level of induction of myogenesis in adult organisms (e.g., older than embryonic), an increased affinity for membranes, an increased level of association with membrane compartment, or combinations thereof.

Some embodiments of the invention include nucleic acid molecules that can encode for the inventive polypeptide ("myomerger nucleic acid molecules"). In certain embodiments, the myomerger nucleic acid molecule is included in a vector (e.g., a viral vector, a retroviral vector, a lentiviral vector, an adenoviral vector, an adeno-associated viral vector, a herpesviral vector, a chimeric viral vector, a plasmid, a cosmid, an artificial chromosome, a bacteriophage, an animal virus, a plant virus, an expression vector, a conjugative vector, or a nonconjugative vector). In certain embodiments, the myomerger nucleic acid molecule is in a cell, such as an insect cell (e.g., an Sf9 cell) or a mammalian cell (e.g., a human cell, a rat cell a mouse cell, a muscle cell, a non-muscle cell, a myoblast, a fibroblast, a C2C12 cell, a 10T ½ fibroblast, an NIH/3T3 cell, a CHO cell, a mesenchymal stem cell (MSC), a hematopoietic stem cell, a blood cell, a bone marrow cell, or an adipose stem cell).

In other embodiments, the myomerger nucleic acid molecule comprises one or more nucleic acid sequences that are not used to encode for the inventive polypeptide (e.g., one or more introns). For example, the myomerger nucleic acid molecule can comprise a nucleic acid sequence as found in nature (e.g., including introns). In certain embodiments, the myomerger nucleic acid molecule differs from the one or more nucleic acid molecules in nature because the myomerger nucleic acid molecule does not include one or more introns. In some embodiments, the myomerger nucleic acid molecule is a cDNA molecule ("myomerger cDNA molecule"). In certain embodiments, the myomerger cDNA molecule is identical to a nucleic acid molecule found in nature. In other embodiments, the myomerger cDNA molecule is not identical to a nucleic acid molecule found in nature (e.g., due to the myomerger cDNA molecule not including one or more introns in the nucleic acid molecule found in nature).

In some embodiments, the myomerger nucleic acid molecule sequence has a sequence identity to a nucleic acid molecule encoding a wt-myomerger polypeptide (e.g., SEQ ID NO:39, SEQ ID NO:40, SEQ ID NO:41, SEQ ID NO:43, SEQ ID NO:46, SEQ ID NO:47, SEQ ID NO:48, or SEQ ID NO:49) of about 90%, about 91%, about 92%, about 93%, about 94%, about 95%, about 96%, about 97%, about 98%, about 99%, about 99.1%, about 99.2%, about 99.3%, about 99.4%, about 99.5%, about 99.6%, about 99.7%, about 99.8%, about 99.9%, about 99.95%, about 99.99%, less than about 100%, at least about 90%, at least about 95%, at least about 99%, or at least about 99.5%. In some embodiments, the myomerger nucleic acid molecule sequence has a sequence identity to SEQ ID NO:39, SEQ ID NO:40, SEQ ID NO:41, SEQ ID NO:43, SEQ ID NO:46, SEQ ID NO:47, SEQ ID NO:48, or SEQ ID NO:49 of about 90%, about 91%, about 92%, about 93%, about 94%, about 95%, about 96%, about 97%, about 98%, about 99%, about 99.1%, about 99.2%, about 99.3%, about 99.4%, about 99.5%, about 99.6%, about 99.7%, about 99.8%, about 99.9%, about 99.95%, about 99.99%, less than about 100%, at least about 90%, at least about 95%, at least about 99%, or at least about 99.5%. Nonlimiting examples of wt-myomerger polypeptides and wt-myomerger nucleic acid molecules can be found in Table 1. The nucleic acid sequence identity (e.g., percent identity) can be determined by any suitable method, such as using BLAST, BLAST-2, ALIGN, ALIGN-2, Clustal Omega, or Megalign software. Unless otherwise indicated, the nucleic acid sequence identity (e.g., percent identity) is determined using BLAST-2.

In some embodiments, the myomerger nucleic acid molecule encodes for an inventive polypeptide that has one or more modifications to wt-myomerger polypeptide in a hydrophobic region, in a signal region, in a transmembrane region, or in a combination thereof.

The myomerger nucleic acid molecule can be made using any suitable technique, such as but not limited to, chemical synthesis, enzymatic production or biological production. Chemical synthesis of a nucleic acid molecule can include, for example, a nucleic acid molecule made by *in vitro* chemical synthesis using phosphotriester, phosphite or phosphoramidite chemistry and solid phase techniques, or via deoxynucleoside H-phosphonate intermediates. Enzymatically produced nucleic acid molecules can be accomplished using any suitable method including but not limited to Polymerase Chain Reaction (PCR). Biologically produced nucleic acid molecules can be accomplished using any suitable method including but not limited to a recombinant nucleic acid produced (i.e., replicated) in a living cell, such as a recombinant DNA vector replicated in bacteria.

Modifications or changes made in the structure of the nucleic acid molecules and/or polypeptides of the present invention are encompassed within some embodiments of the present invention. In certain embodiments, a polypeptide can be modified (e.g., by one or more insertions, one or more deletions, or one or more substitutions (e.g., conservative substitutions)). In some embodiments, the polypeptide which was modified does not have an appreciable loss (e.g., a decrease in a function of less than about 1%, less than about 5%, less than about 10%, less than about 25%, less than about 50%, less than about 75%, less than about 90%, less than about 95%, less than about 99%, or less than about 100%) of one or more chosen functions of the unmodified polypeptide such as, for example, the ability to form a pore in a cell (e.g., in a cell membrane), the ability to make changes to the cytoskeleton of the cell (e.g., reorganizing the cytoskeleton, rearranging the cytoskeleton, making changes to the cytoskeleton to allow the cell to fuse), the ability to activate fusion of two cells, the ability to make a cell fusion capable (e.g., a protein confers fusion capable properties to a cell if upon adding the protein, the cell is capable of fusing to another cell if that other cell comprises myomaker and myomerger), the ability to confer fusogenicity to a cell (e.g., a protein confers fusogenic properties to a cell if upon adding the protein, the cell will fuse with another cell if that other cell comprises myomaker), the level of expression during embryonic development, the level of expression during myogenesis in adult organisms (e.g., older than embryonic), the level of induction of myogenesis in adult organisms (e.g., older than embryonic), the affinity for membranes, or the level of association with membrane compartment. In some embodiments, the polypeptide which was modified retains desired levels (e.g., at least about 20%, at least about 40%, at least about 50%, at least about 75%, at least about 80%, at least about 90%, at least about 95%, or at least about 99%) of one or more functions of the unmodified polypeptide, such as, for example, the ability to form a pore in a cell (e.g., in a cell membrane), the ability to make changes to the cytoskeleton of the cell (e.g., reorganizing the cytoskeleton, rearranging the cytoskeleton, making changes to the cytoskeleton to allow the cell to fuse), the ability to activate fusion of two cells, the ability to make a cell fusion capable (e.g., a protein confers fusion capable properties to a cell if upon adding the protein, the cell is capable of fusing to another cell if that other cell comprises myomaker and myomerger), the ability to confer fusogenicity to a cell (e.g., a protein confers fusogenic properties to a cell if upon adding the protein, the cell will fuse with another cell if that other cell comprises myomaker), the level of expression during embryonic development, the level of expression during myogenesis in adult organisms (e.g., older than embryonic), the level of induction of myogenesis in adult organisms (e.g., older than embryonic), the affinity for membranes, or the level of association with membrane compartment. In some embodiments, the polypeptide after modification has an increased level of one or more functions as compared to the unmodified polypeptide. Nucleic acid molecules can be designed to encode for such a modified polypeptide, and such nucleic acid molecules are encompassed by the present invention.

A "functional polypeptide" is defined as a polypeptide (e.g., a myomerger polypeptide or a modified polypeptide) that has desired levels (e.g., at least about 20%, at least about 40%, at least about 50%, at least about 75%, at least about 80%, at least about 90%, at least about 95%, or at least about 99%, as compared to another polypeptide, such as a naturally occurring polypeptide) of one or more functions such as, for example, the ability to form a pore in a cell (e.g., in a cell membrane), the ability to make changes to the cytoskeleton of the cell (e.g., reorganizing the cytoskeleton, rearranging the cytoskeleton, making changes to the cytoskeleton to allow the cell to fuse), the ability to activate fusion of two cells, the ability to make a cell fusion capable (e.g., a protein confers fusion capable properties to a cell if upon adding the protein, the cell is capable of fusing to another cell if that other cell comprises myomaker and myomerger), the ability to confer fusogenicity to a cell (e.g., a protein confers fusogenic properties to a cell if upon adding the protein, the cell will fuse with another cell if that other cell comprises myomaker), the level of expression during embryonic development, the level of expression during myogenesis in adult organisms (e.g., older than embryonic), the level of induction of myogenesis in adult organisms (e.g., older than embryonic), the affinity for membranes, or the level of association with membrane compartment. In some embodiments, the function polypeptide has an increased level of one or more functions as compared to another polypeptide (e.g., a naturally occurring polypeptide). Nucleic acid molecules can be designed to encode for functional polypeptides, and such nucleic acid molecules are encompassed by the present invention.

A "functionally equivalent" polypeptide (e.g., a myomerger polypeptide) is defined as a polypeptide that has been modified (e.g., by one or more insertions, one or more deletions, or one or more substitutions (e.g., conservative substitutions)) from an original polypeptide (e.g., a wt-myomerger plypeptide) and that modified polypeptide retains desired levels (e.g., at least about 20%, at least about 40%, at least about 50%, at least about 75%, at least about 80%, at least about 90%, at least about 95%, or at least about 99%) of one or more functions of the original polypeptide, such as, for example, the ability to form a pore in a cell (e.g., in a cell membrane), the ability to make changes to the cytoskeleton of the cell (e.g., reorganizing the cytoskeleton, rearranging the cytoskeleton, making changes to the cytoskeleton to allow the cell to fuse), the ability to activate fusion of two cells, the ability to make a cell fusion capable (e.g., a protein confers fusion capable properties to a cell if upon adding the protein, the cell is capable of fusing to another cell if that other cell comprises myomaker and myomerger), the ability to confer fusogenicity to a cell (e.g., a protein confers fusogenic properties to a cell if upon adding the protein, the cell will fuse with another cell if that other cell comprises myomaker), the level of expression during embryonic development, the level of expression during myogenesis in adult organisms (e.g., older than embryonic), the level of induction of myogenesis in adult organisms (e.g., older than embryonic), the affinity for membranes, or the level of association with membrane compartment. In some embodiments, the functionally equivalent polypeptide has an increased level of one or more functions compared to the original polypeptide. Nucleic acid molecules can be designed to encode for functionally equivalent polypeptides, and such nucleic acid molecules are encompassed by the present invention.

In certain embodiments, the shorter the length of a polypeptide, the fewer the modifications (e.g., substitutions) that can be made within the polypeptide while retaining, for example, a desired level of a chosen function. In some instances, longer domains can have a greater number of such changes while retaining, for example, a desired level of a chosen function. In other embodiments, a full-length polypeptide can have more tolerance for a fixed number of changes while retaining, for example, a desired level of a chosen function, as compared to a shorter length of that polypeptide.

The design of substitutions can take many forms, including but not limited to those described herein. In some embodiments, the hydropathic index of amino acids may be considered in designing substitutions. In the hydropathic index, each amino acid is assigned a hydropathic index on the basis of their hydrophobicity or charge characteristics, as follows: isoleucine (+4.5); valine (+4.2); Leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); or arginine (-4.5). In some instances, certain amino acids may be substituted for other amino acids having a similar hydropathic index. In making changes based upon the hydropathic index, the substitution of amino acids with hydropathic indices can be made with amino acids that have an index difference of no more than ±2, no more than ±1, or no more than ±0.5.

In some embodiments, substitutions can also be made based on hydrophilicity values. As detailed in U.S. Patent 4,554,101, the following hydrophilicity values have been assigned to amino acid residues: arginine (+3.0); lysine (+3.0); aspartate (+3.0 ± 1); glutamate (+3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). In making changes based upon similar hydrophilicity values, the substitution of amino acids with hydrophilicity values can be made with amino acids that have a value of no more than ±2, no more than ±1, or no more than ±0.5.

A "conservative substitution" in an amino acid sequence or polypeptide indicates that a given amino acid residue is replaced by a residue having similar physiochemical characteristics (e.g., no more than ±1 when based on hydropathic index or no more than ±1 when base on hydrophilicity values). Examples of conservative substitutions include (a) substitution of one aliphatic residue for another with an aliphatic residue, (b) substitution of one of Ile, Val, Leu, or Ala for one another of Ile, Val, Leu, or Ala, (c) substitution of one of Gly, Ile, Val, Leu, or Ala for one another of Gly, Ile, Val, Leu, or Ala, (d) substitution of one polar residue for another polar residue, (e) substitution of one of Lys and Arg with another of Lys and Arg, (f) substitution of one of Glu and Asp with another of Glu and Asp, (g) substitution of one of Gln and Asn with another of Gln and Asn, (h) substitution of one hydroxyl or sulfur containing residue with another hydroxyl or sulfur containing residue, (i) substitution of one of Ser, Cys, Thr, or Met with another of Ser, Cys, Thr, or Met, (j) substitution of one aromatic residue for another with an aromatic residue, (k) substitution of one of Phe, Tyr, or Trp with another of Phe, Tyr, or Trp, (l) substitution of one basic residue for another basic residue, (m) substitution of one of His, Lys, or Arg with another of His, Lys, or Arg, (n) substitution of an acidic/amide residue with another acidic/amide residue, (o) substitution of one of Asp, Glu, Asn, or Gln with another of Asp, Glu, Asn, or Gln, (p) substitution of a residue with another residue of a similar size, and (q) substitution of one of Ala, Gly, or Ser with another of Ala, Gly, or Ser. In some embodiments, each amino acid in a hydrophobic region of a polypeptide can be substituted with conservative substitutions (e.g., any combination of conservative substitutions relating to hydrophobic residues) .

While discussion has focused on amino acid changes, it will be appreciated that these changes may occur by alteration of the encoding DNA; taking into consideration also that the genetic code is degenerate and that two or more codons may code for the same amino acid. A table of amino acids and their codons is presented below for use in such embodiments, as well as for other uses, such as in the design of probes and primers and the like.

**Tables A and B. Amino acid designations and codon table**

| **Table A - Amino Acid Designations** | | | **Table B - Codons for Amino Acids** |
|---|---|---|---|
| Alanine | Ala | A | GCA GCC GCG GCU |
| Cysteine | Cys | C | UGC UGU |
| Aspartic acid | Asp | D | GAC GAU |
| Glutamic acid | Glu | E | GAA GAG |
| Phenylalanine | Phe | F | UUC UUU |
| Glycine | Gly | G | GGA GGC GGG GGU |
| Histidine | His | H | CAC CAU |
| Isoleucine | Ile | I | AUA AUC AUU |
| Lysine | Lys | K | AAA AAG |
| Leucine | Leu | L | UUA UUG CUA CUC CUG CUU |
| Methionine | Met | M | AUG |
| Asparagine | Asn | N | AAC AAU |
| Proline | Pro | P | CCA CCC CCG CCU |
| Glutamine | Gln | Q | CAA CAG |
| Arginine | Arg | R | AGA AGG CGA CGC CGG CGU |
| Serine | Ser | S | AGC AGU UCA UCC UCG UCU |
| Threonine | Thr | T | ACA ACC ACG ACU |
| Valine | Val | V | GUA GUC GUG GUU |
| Tryptophan | Trp | W | UGG |
| Tyrosine | Tyr | Y | UAC UAU |

The term "functionally equivalent codon" is used herein to refer to codons that encode the same amino acid, such as the six codons for arginine or serine.

In certain instances, the nucleic acid molecule can be engineered to contain distinct sequences while at the same time retaining the capacity to encode a desired inventive polypeptide. In some embodiments, this can be accomplished owing to the degeneracy of the genetic code (i.e., the presence of multiple codons) which encode for the same amino acids. In other instances, it can be accomplished by including, adding, or excluding introns in the nucleic acid molecule.

In certain embodiments, a restriction enzyme recognition sequence can be introduced into a nucleic acid sequence while maintaining the ability of that nucleic acid molecule to encode a desired polypeptide. In other embodiments, a CRISPR system (e.g., a CRISPR system comprising one or more of guide RNA, crRNA, tracrRNA, sgRNA, DNA repair template, and Cas protein, such as but not limited to CRISPR/Cas9) can be used to introduce a nucleic acid molecule while maintaining the ability of that nucleic acid molecule to encode a desired polypeptide.

It will also be understood that amino acid sequences (e.g., polypeptides) and nucleic acid sequences may include additional residues, such as additional N- or C-terminal amino acids or 5' or 3' sequences, and yet still be essentially as set forth in one of the sequences disclosed herein, so long as the sequence meets the criteria set forth above, such as including the maintenance of biological activity where polypeptide expression is concerned. The addition of terminal sequences particularly applies to nucleic acid sequences that may, for example, include various non-coding sequences flanking either of the 5' or 3' portions of the coding region or may include various internal sequences, (i.e., introns) which can occur within genes.

Some embodiments of the present invention rely on or use synthesis of polypeptides *in cyto,* via transcription and translation of appropriate nucleic acid molecules (e.g., nucleic acid sequences as discussed herein). These polypeptides will include the twenty "natural" amino acids, and post-translational modifications thereof. *In vitro* peptide synthesis permits the use of modified or unusual amino acids. In some embodiments, the inventive polypeptide encompasses modifications (e.g., one or more substitutions or one or more insertions) that include one or more modified or unusual amino acids. A table of exemplary, but not limiting, modified or unusual amino acids is provided in Table C.

| **Table C - Modified or Unusual Amino Acids** | | | |
|---|---|---|---|
| Abbr. | Amino Acid | Abbr. | Amino Acid |
| Aad | 2-Aminoadipic acid | EtAsn | N-Ethylasparagine |
| BAad | 3- Aminoadipic acid | Hyl | Hydroxylysine |
| BAla | beta-alanine, beta-Amino-propionic acid | AHyl | allo-Hydroxylysine |
| Abu | 2-Aminobutyric acid | 3Hyp | 3-Hydroxyproline |
| 4Abu | 4- Aminobutyric acid, piperidinic acid | 4Hyp | 4-Hydroxyproline |
| Acp | 6-Aminocaproic acid | Ide | Isodesmosine |
| Ahe | 2-Aminoheptanoic acid | Aile | allo-Isoleucine |
| Aib | 2-Aminoisobutyric acid | MeGly | N-Methylglycine, sarcosine |
| BAib | 3-Aminoisobutyric acid | MeIle | N-Methylisoleucine |
| Apm | 2-Aminopimelic acid | MeLys | 6-N-Methyllysine |
| Dbu | 2,4-Diaminobutyric acid | MeVal | N-Methylvaline |
| Des | Desmosine | Nva | Norvaline |
| Dpm | 2,2'-Diaminopimelic acid | Nle | Norleucine |
| Dpr | 2,3-Diaminopropionic acid | Orn | Ornithine |
| EtGly | N-Ethylglycine | | |

The presently disclosed subject matter further includes a method of producing an inventive polypeptide (e.g., a mutant myomerger polypeptide or a wt-myomeger polypeptide). Any suitable method can used to make the inventive polypeptides including but not limited to expression through any suitable molecular biological technique (e.g., using a prokaryotic or eukaryotic expression system), isolation from a source in nature, or chemical synthesis. Eukaryotic expression systems include plant-based systems; insect cell systems via recombinant baculoviruses; whole insect systems via recombinant baculoviruses; genetically engineered yeast systems, including but not limited to *Saccharomyces sp.* and *Picchia spp*.; and mammalian cell systems, including but not limited to C2C12 cells, 10T 1/2 fibroblasts, NIH/3T3 fibroblasts, mesenchymal stem cells (MSCs), hematopoietic stem cells, Chinese hamster ovary cells or other cell lines commonly used for industrial scale expression of recombinant proteins. In some embodiments, useful plant-based expression systems can include transgenic plant systems. In some embodiments, useful plant-based expression systems can include transplastomic plant systems.

In some embodiments, a method of producing the inventive polypeptide includes providing a host cell comprising a nucleic acid molecule, as disclosed herein, operatively linked to a promoter operable under conditions whereby the encoded polypeptide is expressed; and recovering the polypeptide from the host cell.

### Myomaker Polypeptides and Myomaker Nucleic Acid Molecules

Some embodiments of the invention include compositions comprising the myomaker polypeptide, the myomaker nucleic acid molecule, or both, cells comprising the myomaker polypeptide, the myomaker nucleic acid molecule, or both, or using the myomaker polypeptide, the myomaker nucleic acid molecule, or both. In certain embodiments, the myomaker polypeptide, the myomaker nucleic acid molecule, or both, are used or part of a composition or a cell, with a myomerger polypeptide, a myomerger nucleic acid molecule, or both. In some embodiments, the myomaker polypeptide is the myomaker protein disclosed in WO 2014/210448 A1, which is herein incorporated by reference in its entirety. In other embodiments, myomaker polypeptide is the myomaker protein disclosed in Table 10A of WO 2014/210448 A1. The term "myomaker polypeptide" encompasses "wt-myomaker polypeptides" (i.e., myomaker polypeptides found in nature without any purposely human-made modification) and "mutant myomaker polypeptides" (e.g., with one or more modifications made to a wt-myomaker polypeptide). Nonlimiting examples of wt-myomaker polypeptides are found in Table 10A of WO 2014/210448 A1 or in Table 2A. In other embodiments, the myomaker polypeptide has at least one amino acid modification relative to a wt-myomaker polypeptide. A wt-myomaker polypeptide can, in some embodiments, be a myomaker polypeptide from any animal including but not limited to a mammal, a rat, a cat, a rabbit, a human, a cow, a chicken, a turkey, a monkey, a tree shrew, a dog, a pig, a shrew, an elephant, or an opossum. Table 2A provides nonlimiting examples of wt-myomaker polypeptides and Tables 2B and 2C provide nonlimiting examples of related nucleic acid sequences (including start and stop codons).

**Table 2A**

| **Source** | **Polypeptide sequence** |
|---|---|
| Human | MGTLVAKLLLPTLSSLAFLPTVSIAAKRRFHMEAMVYLFTLFFVALHH ACNGPGLSVLCFMRHDILEYFSVYGTALSMWVSLMALADFDEPKRST FVMFGVLTIAVRIYHDRWGYGVYSGPIGTAILIIAAKWLQKMKEKKG LYPDKSVYTQQIGPGLCFGALALMLRFFFEDWDYTYVHSFYHCALAM SFVLLLPKVNKKAGSPGTPAKLDCSTLCCACV (SEQ ID NO: 50) |
| Dog | MGTLAAKLLLPTLSSLAFLPTVSIAAKRRFHMEAMVYLFTMFFVALH HACNGPGLSVLCFMRHDVLEYFSVYGTALSMWVSLMALADFDEPKR STFVMFGVLTIAVRIYHDRWGYGVYSGPIGTAVLIIATKWLQQMKEK KSLYPDKSVYTQQIGPGLCFGALALMLRFFFEDWDYTYVHSFYHCAL AMSFVLLLPKVNKKAGSAGPPAKLDCSTLCCACI (SEQ ID NO: 51) |
| Pig | MGTVMAKLLLPTLSSLAFLPTVSIAAKRRFHMEAMVYLFTTFFVAFY HACHGPGLAMICFLRLDILEYFSVYGTALSMWVSLMALADFDEPKRS TFVMFGVLTIAVRIYHDRWGYGVYSGPIGTAALIIAAKWLQQMKDQR RLYPDKSVYTQQIGPGLCFGALALMLRFFFEEWDYTYVHSFYHCALA MSFVLLLPKANKKAGSAGPPAKLDCSTLCCACI (SEQ ID NO: 52) |
| Mouse | MGTVVAKLLLPTLSSLAFLPTVSIATKRRFYMEAMVYLFTMFFVAFSH ACDGPGLSVLCFMRRDILEYFSIYGTALSMWVSLMALADFDEPQRSTF TMLGVLTIAVRTFHDRWGYGVYSGPIGTATLIIAVKWLKKMKEKKGL YPDKSIYTQQIGPGLCFGALALMLRFFFEEWDYTYVHSFYHCALAMSF VLLLPKVNKKAGNAGAPAKLTFSTLCCTCV (SEQ ID NO: 53) |
| Opossum | MGTLVTKLLLPTISSLAFLPTISIAAKRRFHMEAMVYLFTMFFIAIYHA CDGPGLSVLCFMRYDILEYFSIYGTALSMWVSLMALAEFDEPKRSTFV MFGVLTIAVRIYQDRWGYGVYSGPIGTAVLIIATKWLQKMKEKKGLY PDKSVYTQQIGPGFCFGALALMLRFFFQEWDYTYVHSFYHCSLAMSF VLLLPKVNKKAGNAGTPAKLDCSTLCCACI (SEQ ID NO: 54) |
| Zebrafish | MGAFIAKMLLPTISSLVFVPAASVAAKRGFHMEAMVYFFTMFFTAIY HACDGPGLSILCFMKYDILEYFSVYGTAISMWVTLLALGDFDEPKRSS LTMFGVLTAAVRIYQDRLGYGIYSGPIGTAVFMITVKWLQKMKEKKG LYPDKSVYTQQVGPGCCFGALALMLRFYFEEWDYAYVHSFYHVSLA MSFILLLPKKNRYAGTGRNAAKLNCYTLCCCV (SEQ ID NO: 55) |

**Table 2B**

| **Source** | **cDNA nucleic acid sequence** |
|---|---|
| Human | atggggac gctggtggcc aagctgctcc tgcccaccct cagcagcctg gccttcctcc ccactgtcag catcgcggcc aagaggcggt tccacatgga ggccatggtc tacctcttca ccctgttctt cgtggcgctc caccatgcct gcaatggacc cggcttgtct gtgctgtgct tcatgcgtca cgacatcctg gagtatttca gtgtctacgg gacagccctg agcatgtggg tctcgctgat ggcactggcc gacttcgacg aacccaagag gtcaacattt gtgatgttcg gcgtcctgac cattgctgtg cggatctacc atgaccgatg gggctacggg gtgtactcgg gccccatcgg cacagccatc ctcatcatcg cggcaaagtg gctacagaag atgaaggaga agaagggcct gtacccagac aagagcgtct acacccagca gataggcccc ggcctctgct tcggggcgct ggccctgatg ctacgcttct tctttgagga ctgggactac acttatgtcc acagcttcta ccactgtgcc ctggctatgt cctttgttct gctgctgccc aaggtcaaca agaaggctgg atccccgggg accccggcca agctggactg ctccaccctg tgctgtgctt gtgtctga (SEQ ID NO: 56) |
| Dog | atgggga cgctcgcggc gaagctgctc ctgcccaccc tcagcagcct ggccttcctc cccaccgtca gcatcgccgc caagcggcgg ttccacatgg aggccatggt ctacctcttc accatgttct tcgtggcact ccaccacgcg tgcaacgggc ccgggctatc ggtgctctgc ttcatgcgcc acgacgtcct ggagtacttc agcgtctatg ggacggcact gagcatgtgg gtctcgctga tggcactggc tgacttcgac gaacccaaga ggtcgacttt tgtgatgttt ggcgtcctga ccatcgccgt gcggatctac catgaccgct ggggctacgg ggtgtactcg ggccccattg gcacggctgt cctcatcatc gccacaaagt ggctgcagca gatgaaggag aagaagagtc tgtacccgga caagagtgtc tacacccagc agataggccc tggcctctgt tttggggcac tggcccttat gctgcgcttc ttttttgagg actgggatta cacctatgtc cacagcttct accactgtgc cctggccatg tccttcgtcc tcctgctccc caaggtcaac aagaaggctg gaagcgcggg gccccctgcc aagctagact gctctaccct ttgctgtgct tgcatctga (SEQ ID NO: 57) |
| Pig | atgg ggaccgtcat ggccaaactg ctgctaccca cgctgagcag cctggccttc ctccccacgg tcagcatcgc tgccaagcgg cggttccaca tggaggccat ggtctatctc ttcaccacgt tcttcgtggc gttctaccac gcctgccacg ggccgggcct ggctatgatc tgctttctgc gccttgacat cctggagtat ttcagcgtct acggaaccgc cctgagcatg tgggtctcgc tgatggcgct ggctgacttc gacgagccca agaggtcgac tttcgtgatg tttggcgtcc tgaccatcgc cgtgcggatc taccacgacc gctggggcta cggcgtgtac tcgggcccca tcggcacggc cgccctcatc atcgcggcca agtggctgca gcagatgaag gaccaacggc gcctgtatcc agacaagagc gtgtacacac agcagatagg ccccggcctc tgcttcgggg cgctggccct catgctgcgc tttttcttcg aggagtggga ttatacctac gtccacagct tctaccactg cgccctggcc atgtccttcg tcctgctgct gcccaaggcc aacaagaagg ctggaagcgc agggccaccc gccaagctgg actgctccac cctctgctgt gcttgtatct ga (SEQ ID NO: 58) |
| Mouse | atgg ggacagttgt agccaaactg ctcctgccta ccctcagcag cctggccttc ctcccgacag tgagcatcgc taccaagagg cgtttctaca tggaggccat ggtctacctc ttcaccatgt tctttgtggc gttctcccat gcctgtgatg ggcctggttt gtctgtgctg tgcttcatgc gccgtgacat tctggagtac ttcagcatct atggaacagc cctgagcatg tgggtctccc tgatggcact ggccgacttt gatgaacccc agagatcgac cttcacaatg cttggcgtcc ttaccatcgc tgtgcggact tttcatgacc gctggggtta cggggtatac tccggtccca taggcacggc caccctcatc attgctgtaa agtggctgaa gaagatgaaa gagaagaagg gcctgtaccc cgacaagagc atctacaccc agcagatagg ccccggcctg tgctttgggg ccctggccct gatgcttcga ttcttctttg aggaatggga ttacacctac gtccacagct tctaccactg tgccctggcc atgtcctttg tcctgctgct gcccaaggtc aacaagaagg ctgggaacgc aggggccccc gccaagctga ccttctccac cctctgctgc acttgtgtct ga (SEQ ID NO: 59) |
| Opossum | atggg gactcttgtt accaagttgc ttcttcccac aatcagcagc ctcgcctttc tccccaccat cagcatcgct gctaagagga gattccacat ggaagccatg gtctacctct tcaccatgtt cttcatagca atatatcatg catgtgacgg gccaggctta tcagtgctat gcttcatgcg ctatgacata ctggagtatt tcagcatcta tgggacagca ctgagcatgt gggtgtcatt aatggcactg gcagagttcg atgaaccaaa aaggtcaacc tttgtaatgt ttggcgtgtt gactattgcc gtgaggatct accaagaccg gtggggatat ggggtatact cggggcctat tggcacagct gtccttatca ttgcaacaaa atggctgcaa aagatgaaag agaagaaggg tctgtaccct gacaagagtg tgtacaccca acagataggc cctggtttct gttttggagc gttagcactg atgctgcgtt tctttttcca ggagtgggat tacacctatg ttcacagctt ctaccactgt tcactagcca tgtcctttgt cttgctgctg cccaaggtaa acaagaaagc tgggaatgct gggacacctg ccaaattgga ctgttctaca ctctgctgtg cttgcatctg a (SEQ ID NO: 60) |
| Zebrafish | atgggag cgtttatcgc caagatgttg ctgcccacta ttagcagttt ggtgtttgtg cctgcagcca gcgtggctgc aaagaggggc ttccacatgg aggccatggt ctatttcttc acaatgttct tcaccgcgat ttaccacgca tgtgacggtc cgggcttgtc cattctctgt ttcatgaagt atgacattct ggagtacttc agcgtgtacg ggacagccat ctccatgtgg gtcacgctac tggcgcttgg ggatttcgat gagcccaaac gctcttcgct caccatgttt ggggtgttga ccgcagctgt gaggatttac caggaccgac tgggctacgg catttactcc ggccccatcg ggacagctgt ctttatgatc acagtcaaat ggttacagaa aatgaaggaa aagaaaggcc tttatccaga caaaagtgtt tacactcaac aagtgggccc agggtgctgc ttcggtgctc ttgctttgat gcttcgcttc tattttgagg agtgggacta cgcttatgtt cacagtttct accacgtgtc tctggccatg tcctttattc tgctgctgcc |
| | caagaagaac cgttatgctg gaacgggacg taacgcagcc aaactcaact gctacaccct ctgctgctgt gtatga (SEQ ID NO: 61) |

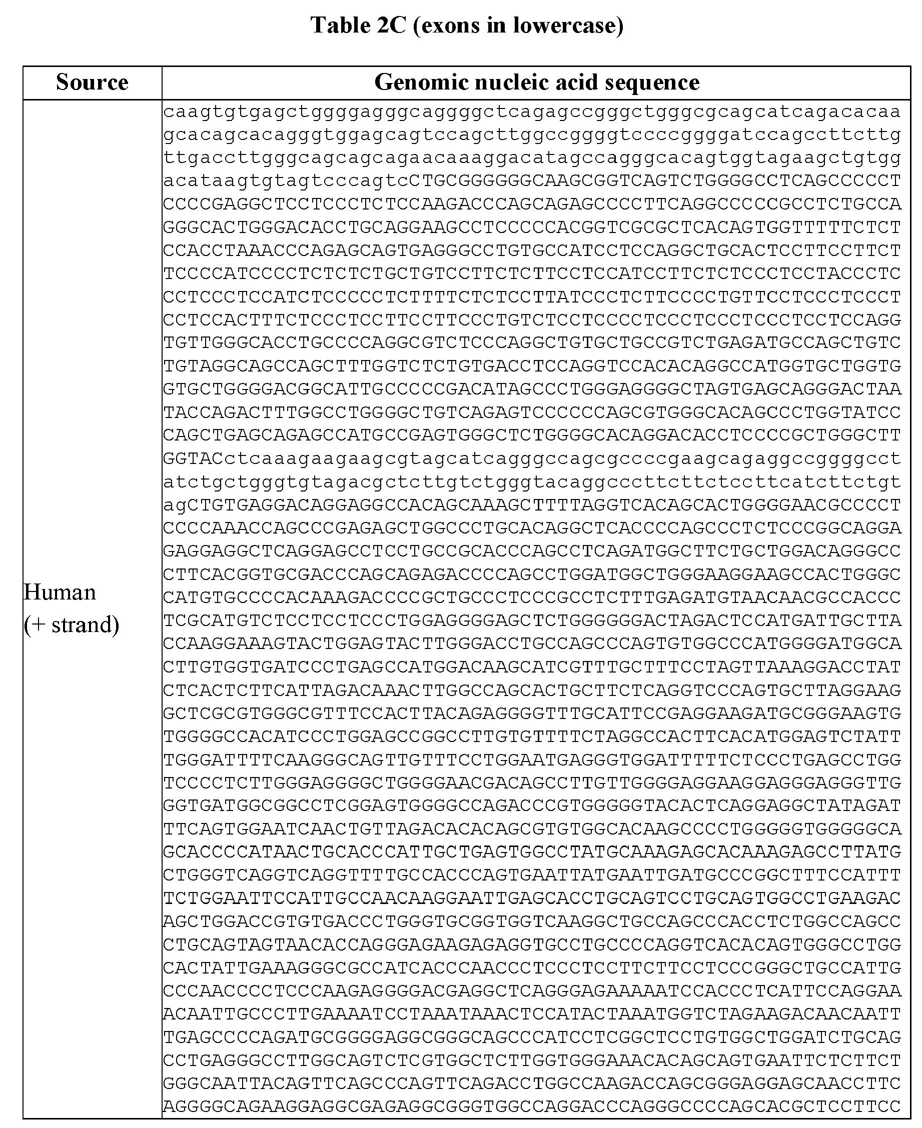

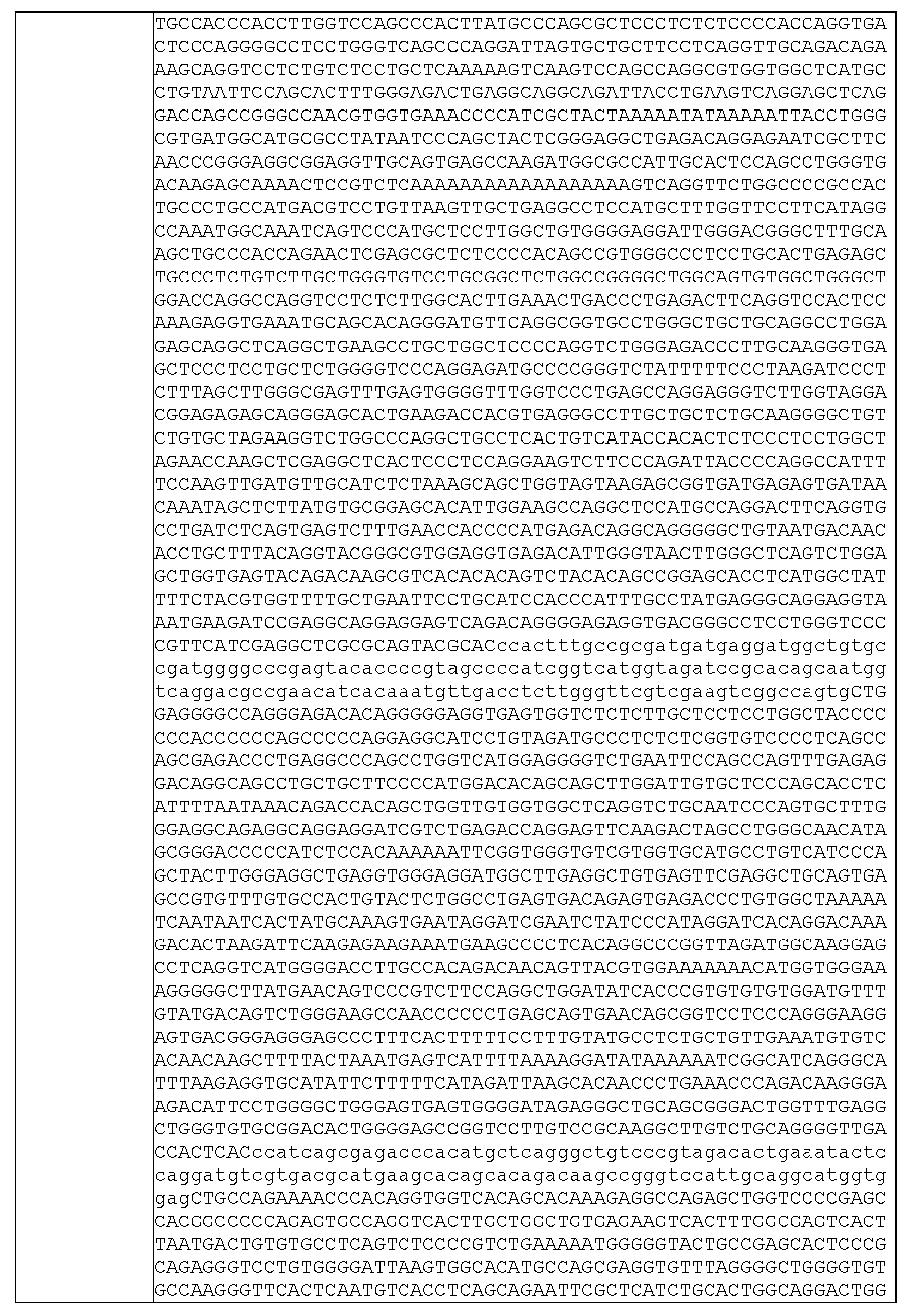

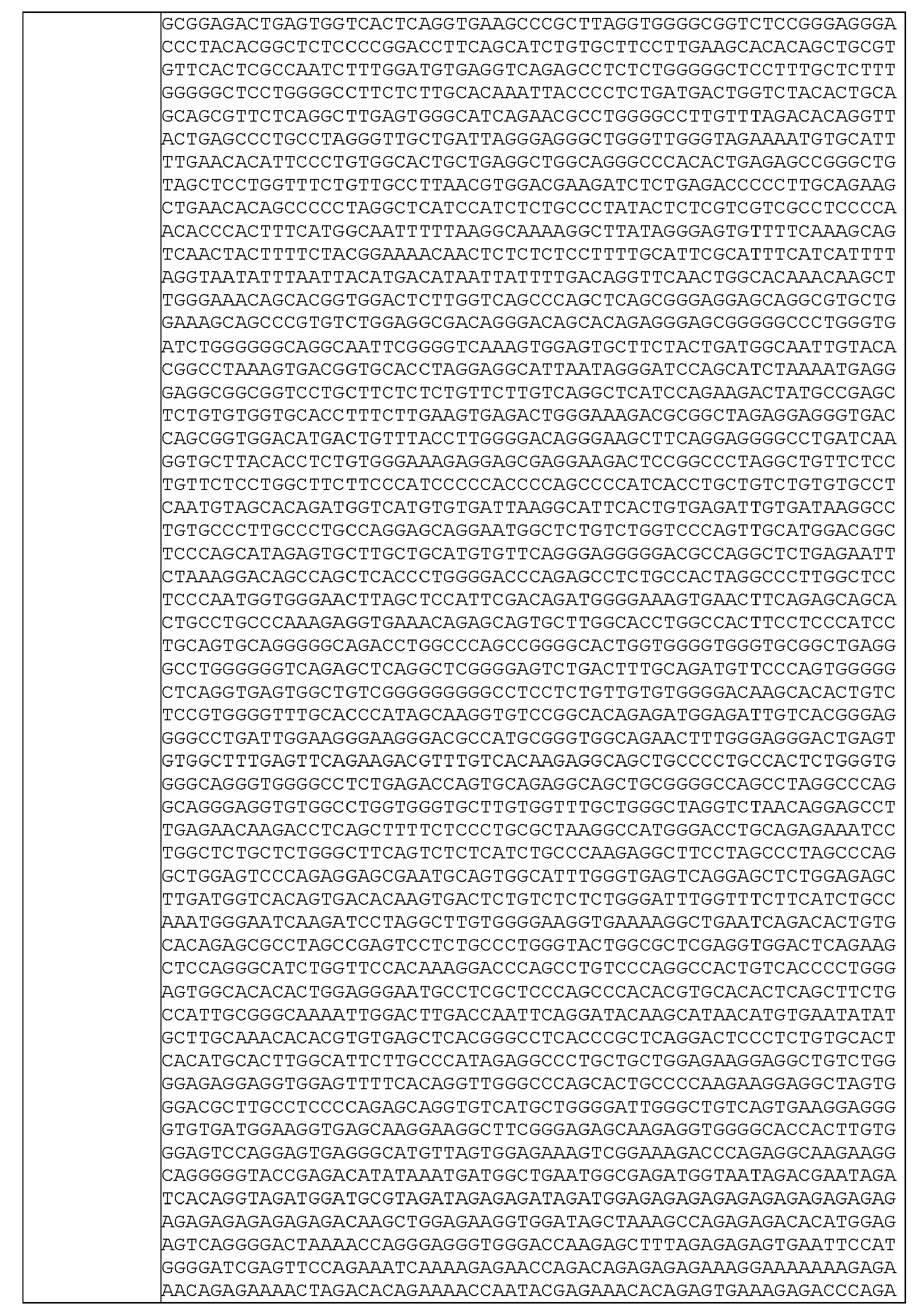

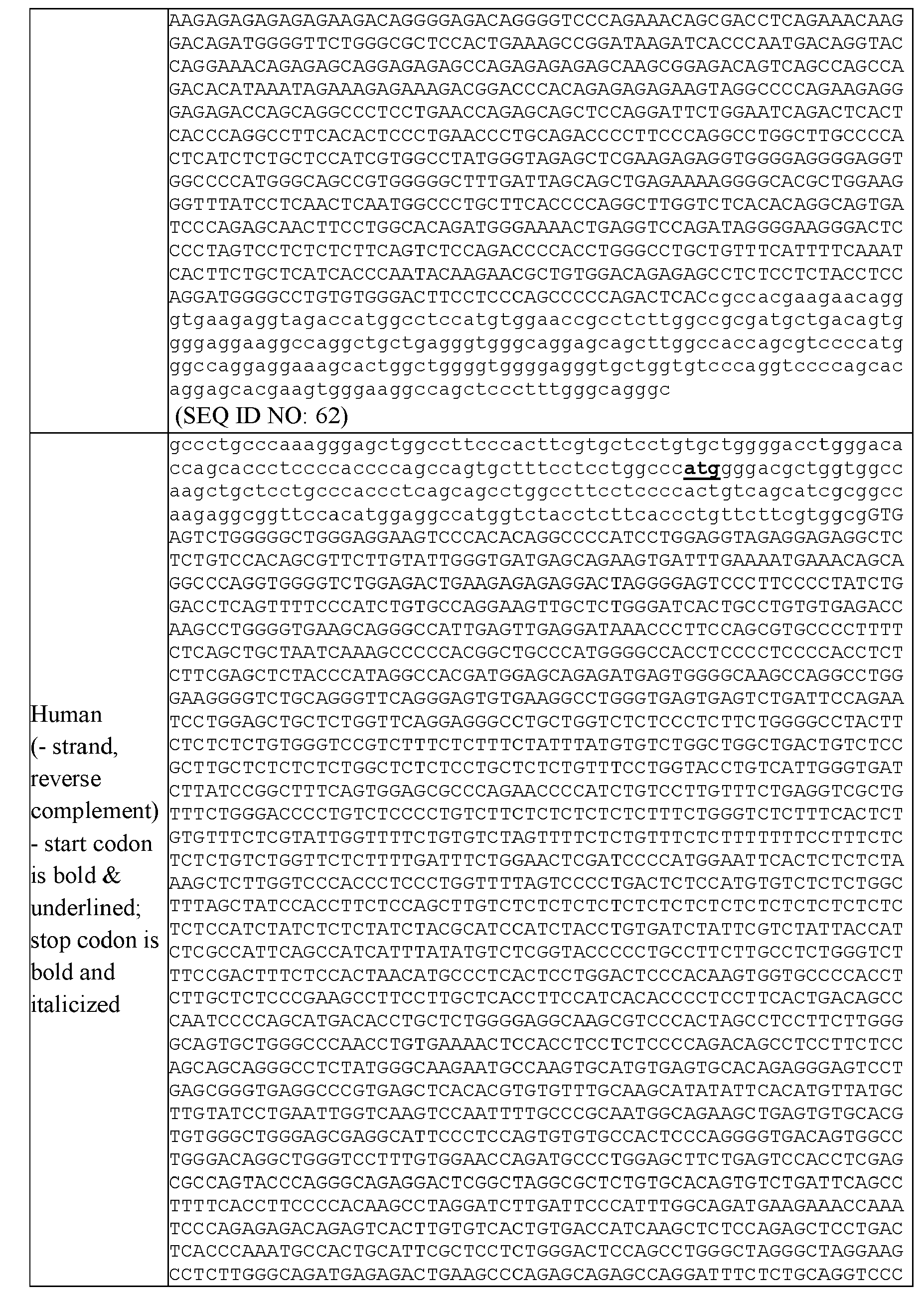

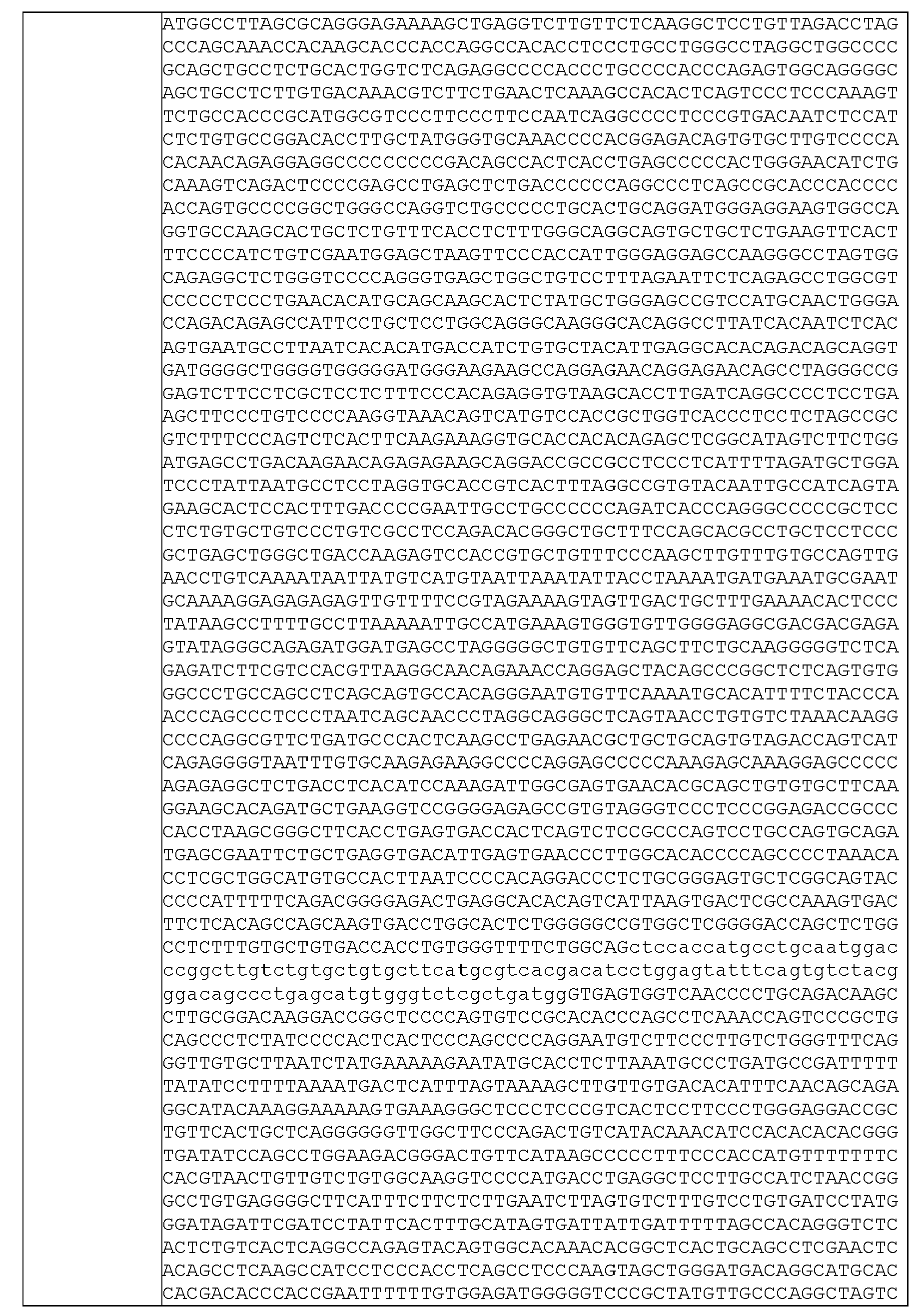

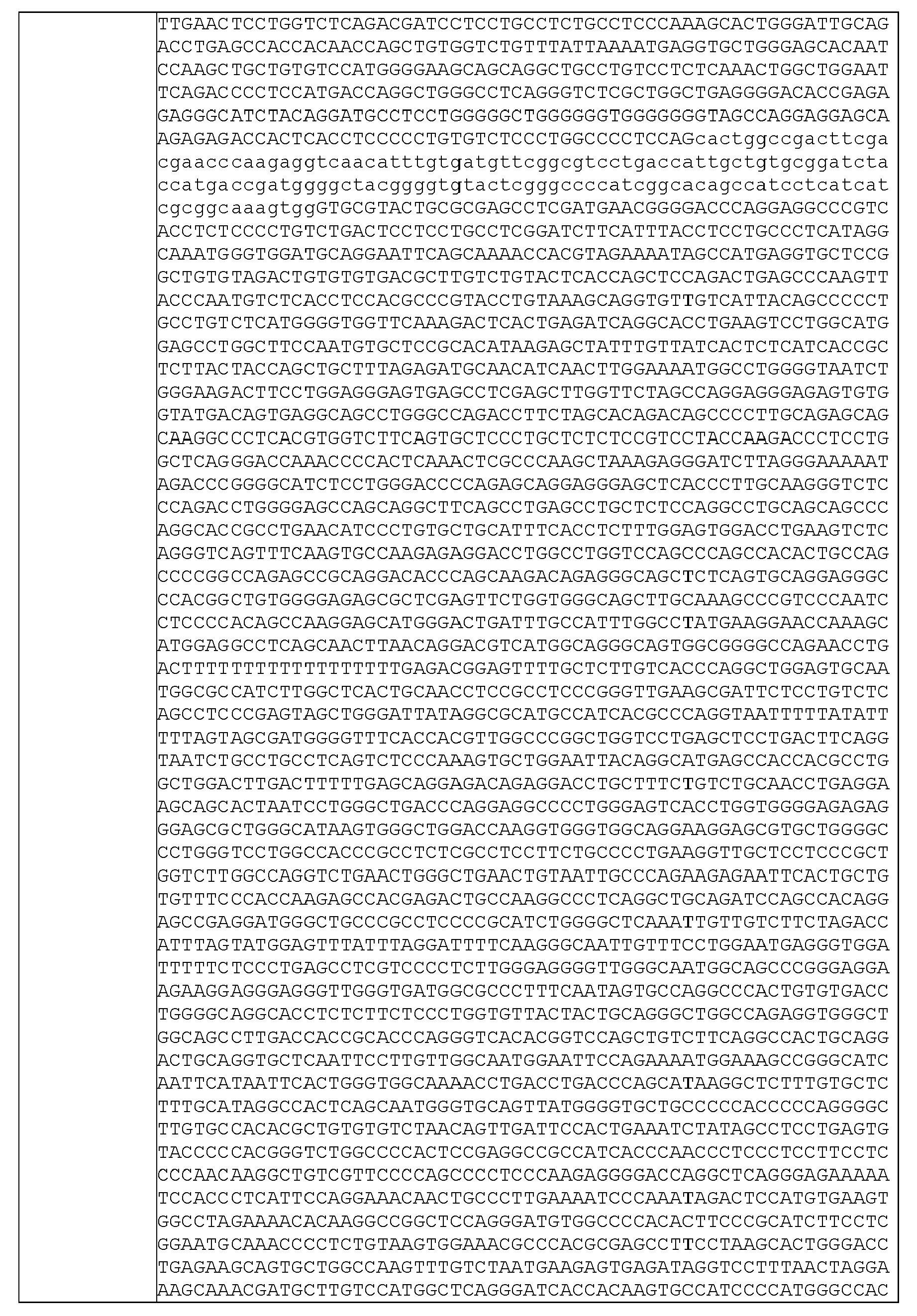

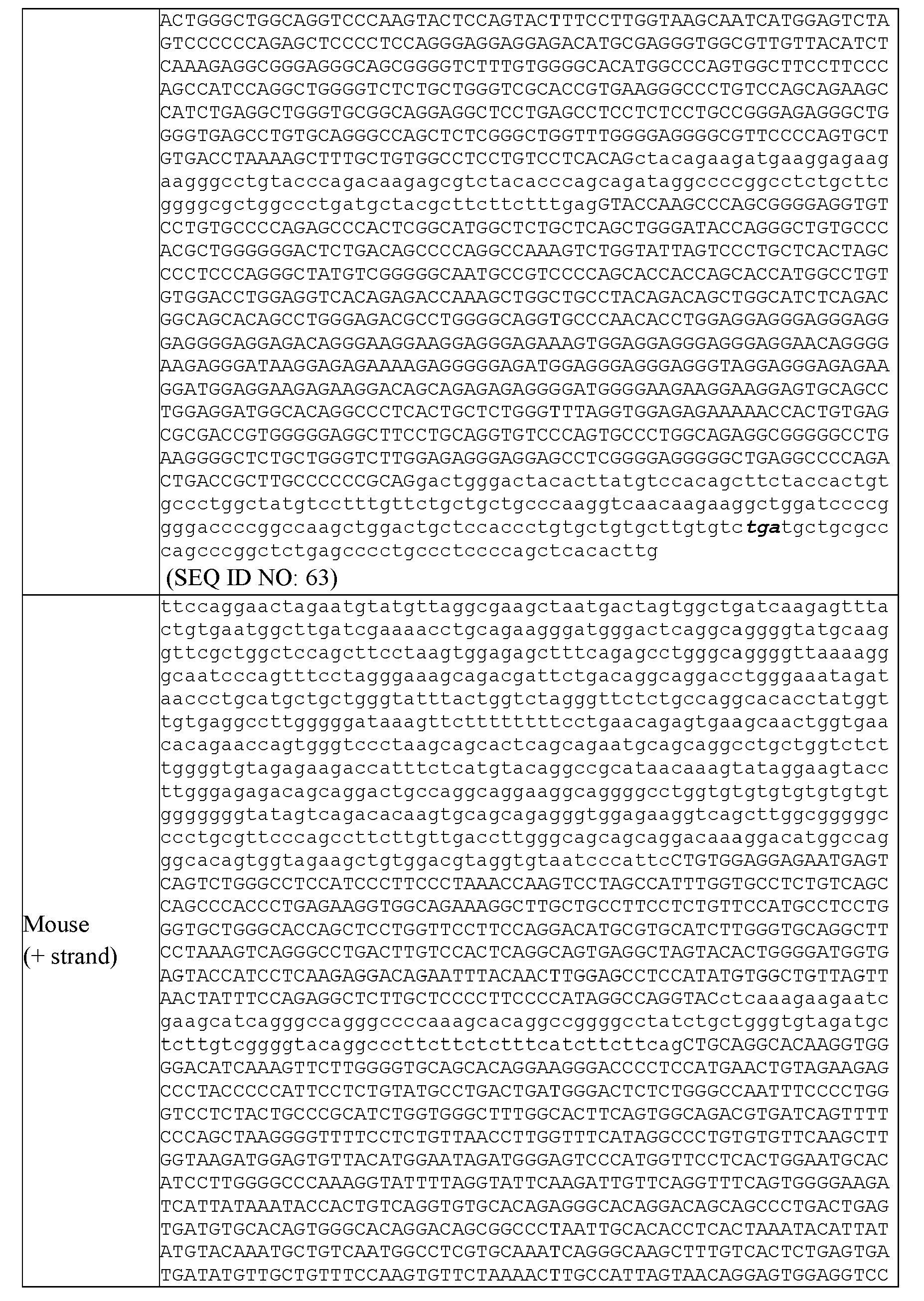

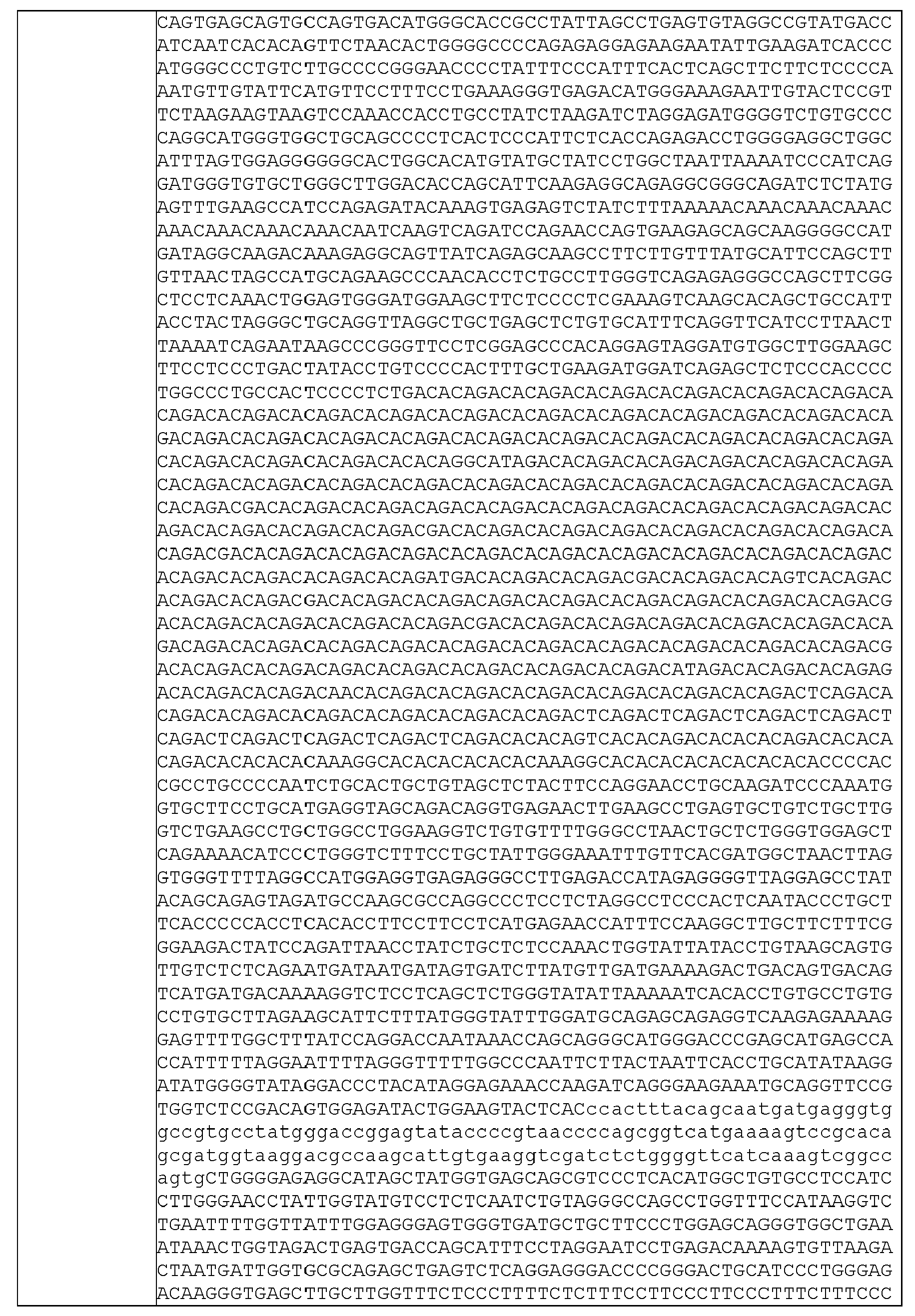

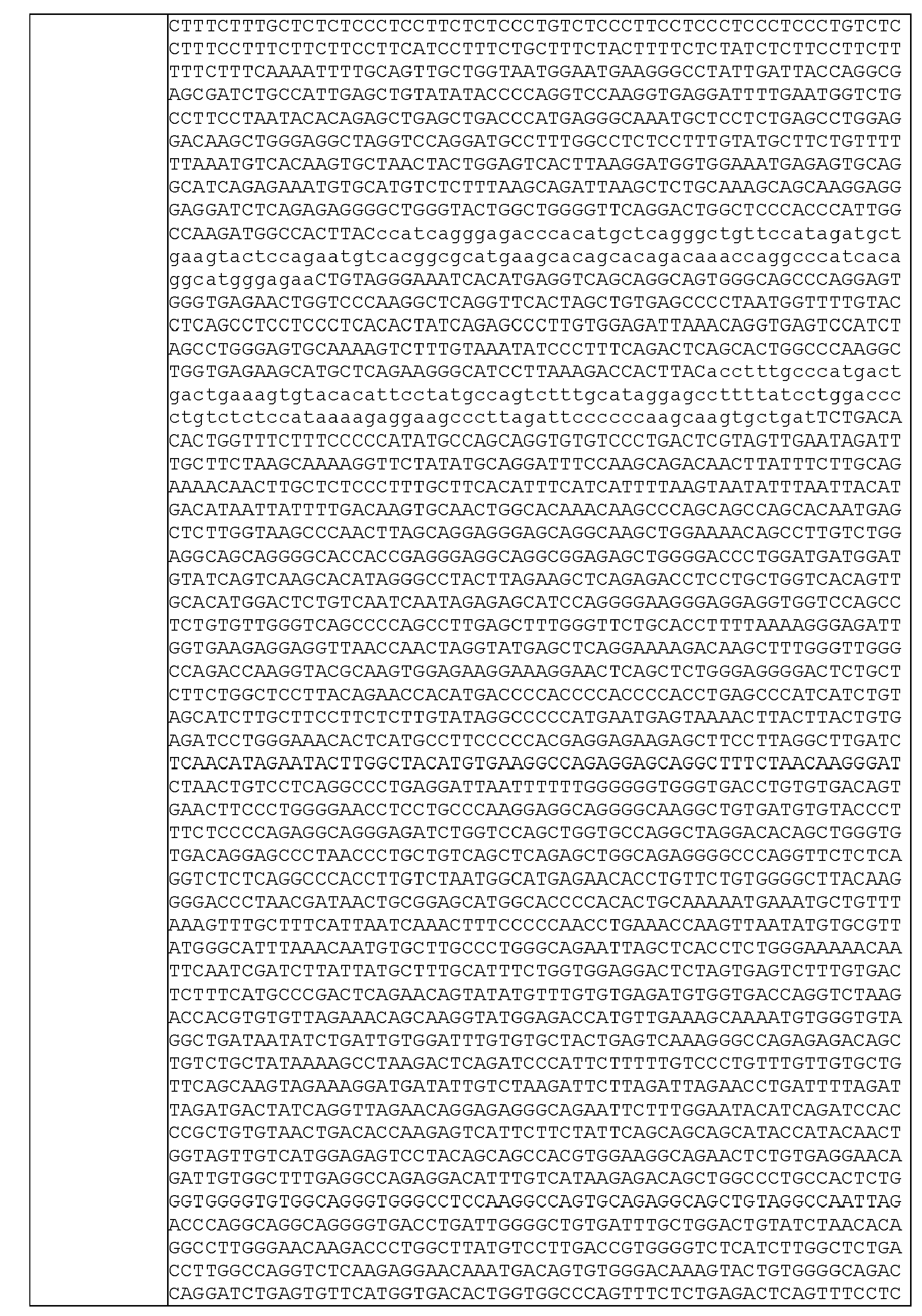

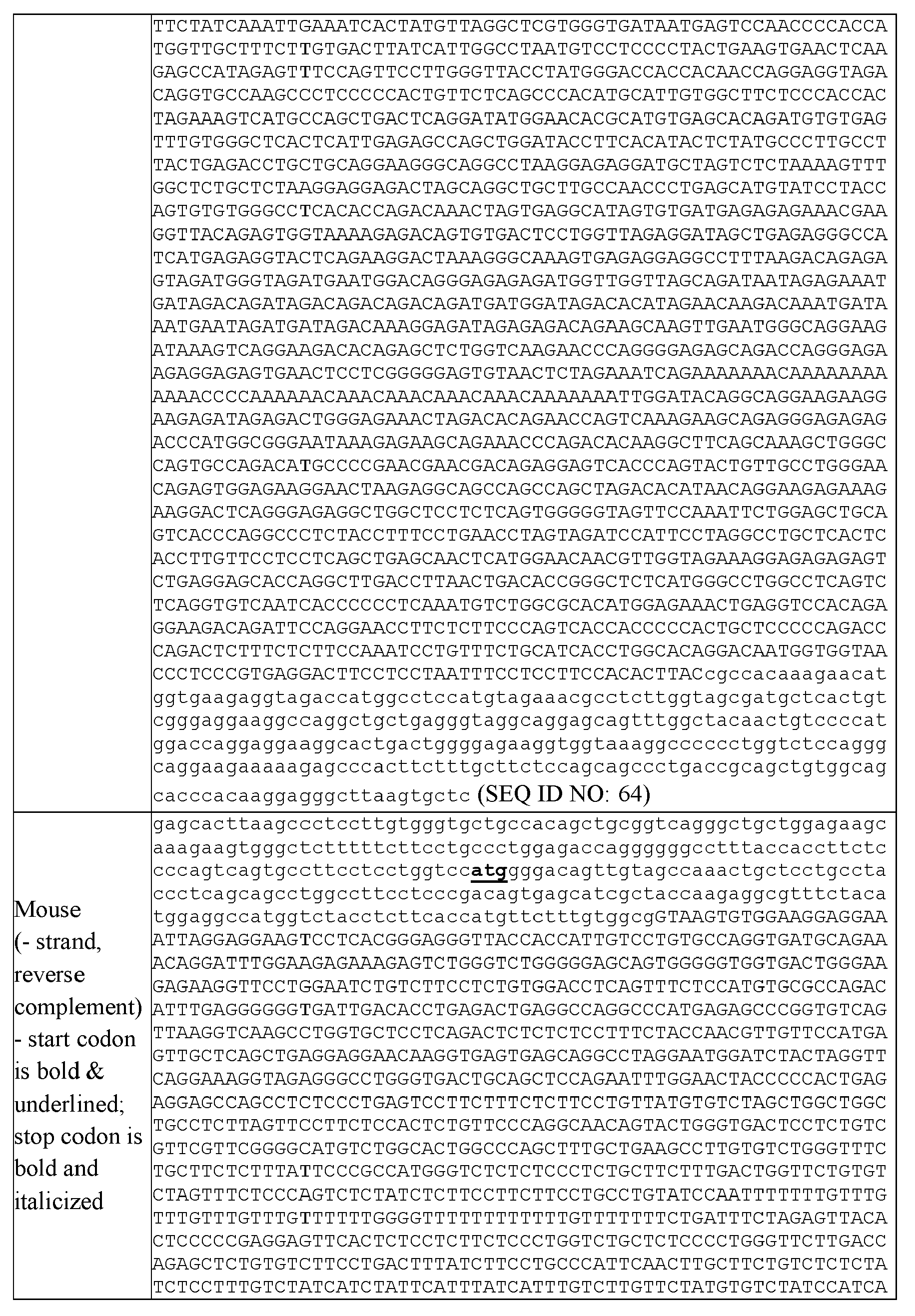

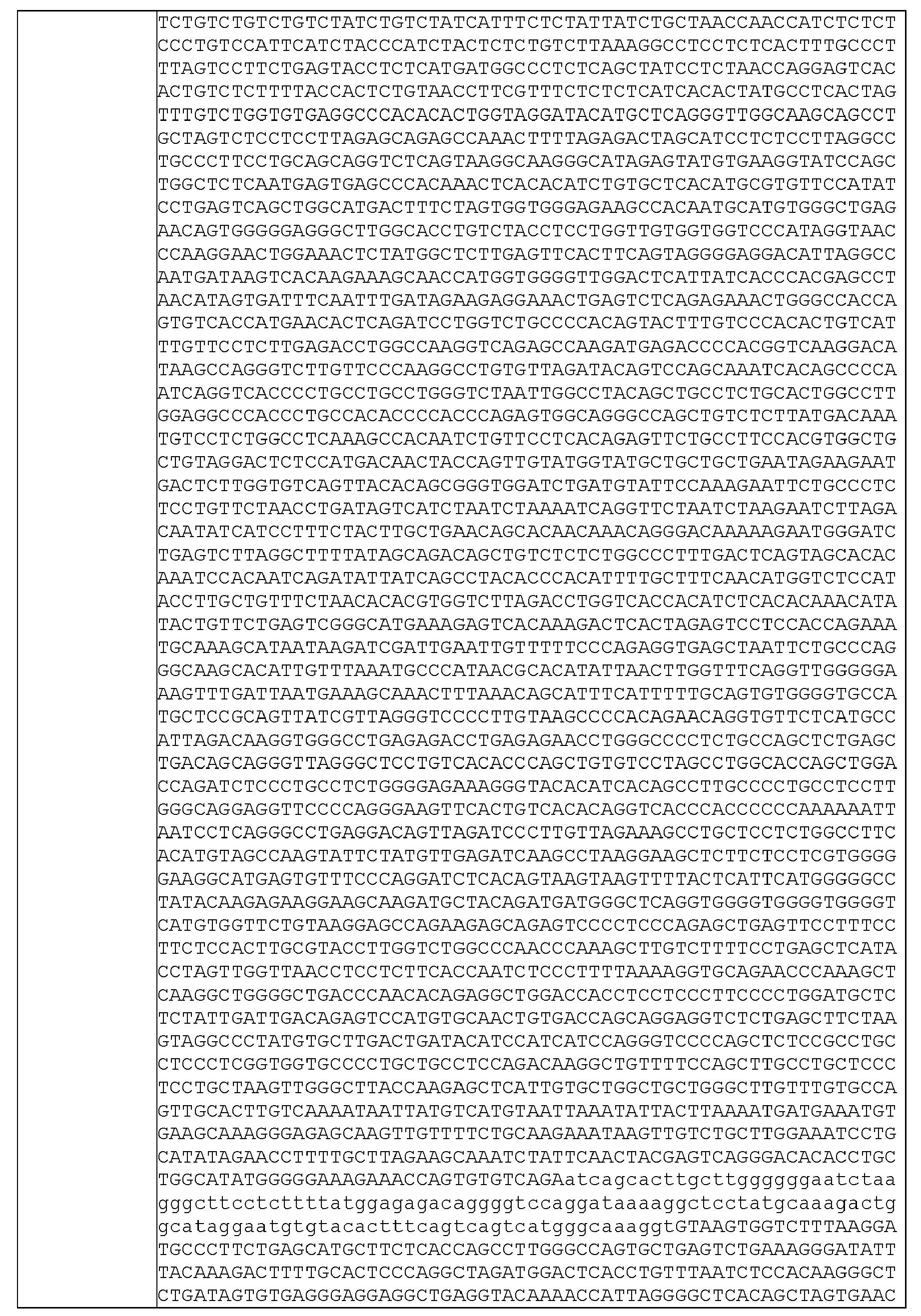

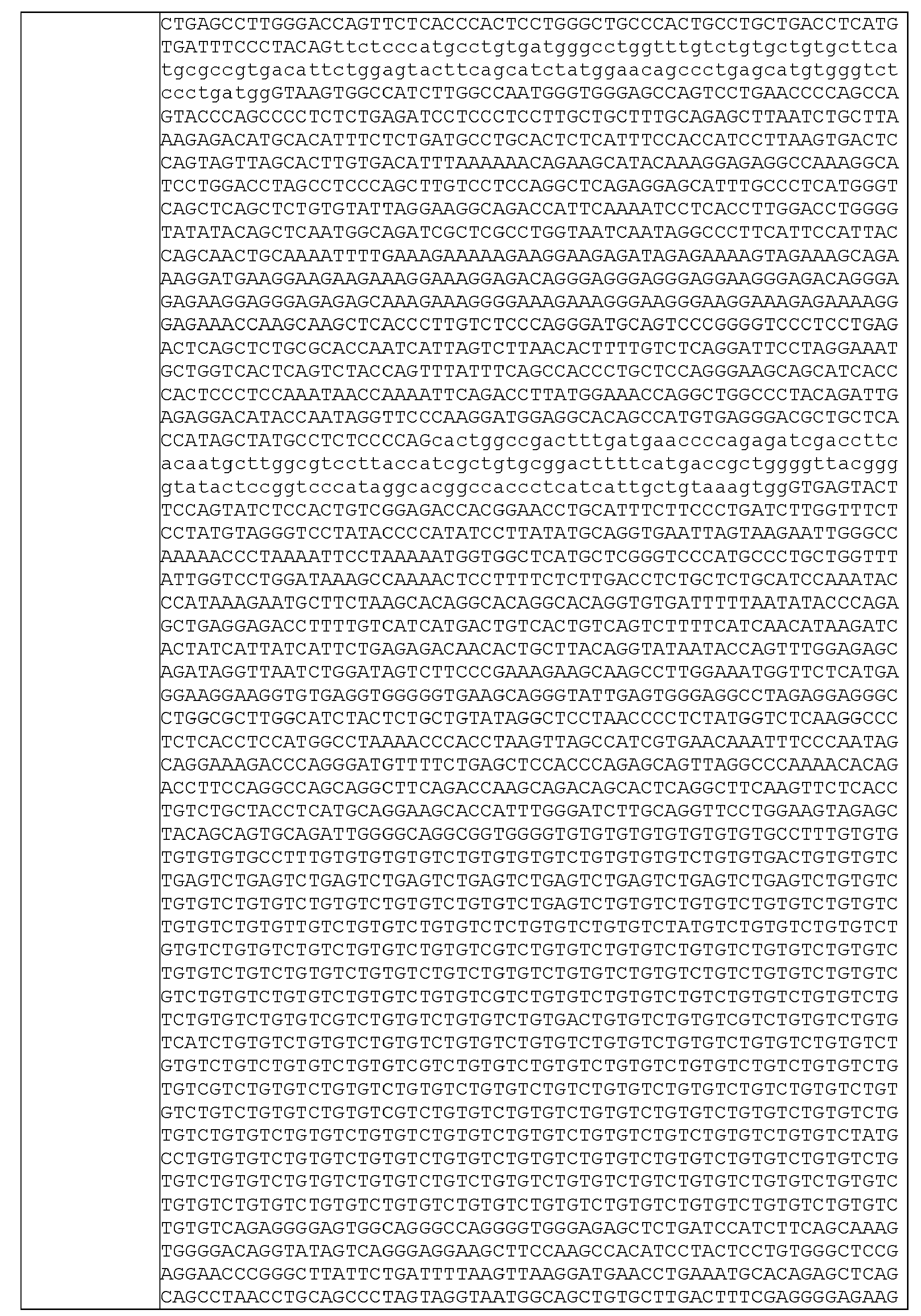

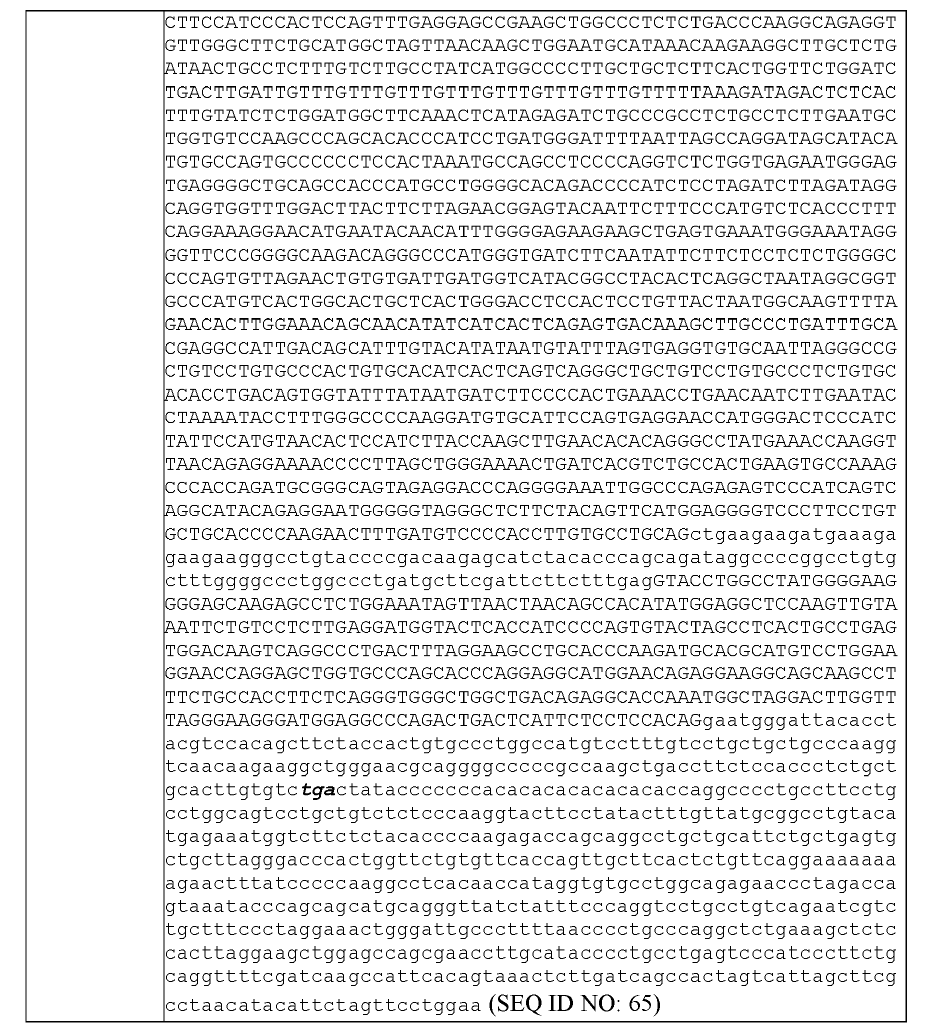

One or more modifications, in some instances, can include an insertion, a deletion, a substitution, or combinations thereof. In some embodiments, the inventive polypeptide does not encompass one or more naturally occurring polypeptides (e.g., does not encompass one or more of the wt-myomaker polypeptides). In other embodiments, the inventive polypeptide does not encompass any of the wt-myomaker polypeptides. In some embodiments, the inventive polypeptide does not encompass any naturally occurring polypeptide (e.g., does not encompass any of the wt-myomaker polypeptides or any other naturally occurring polypeptide).

In some embodiments, one or more modifications to a wt-myomaker polypeptide can include one or more substitutions, one or more insertions, or one or more deletions (or combinations thereof) to one or more amino acids in a hydrophobic region of a wt-myomaker polypeptide, to one or more amino acids in a hydrophilic region of a wt-myomaker polypeptide, or in a combination thereof. In some embodiments, one or more modifications to a wt-myomaker polypeptide can include one or more substitutions or one or more deletions (or combinations thereof) to one or more amino acids in a hydrophobic region of a wt-myomaker polypeptide, to one or more amino acids in a hydrophilic region of a wt-myomaker polypeptide, or in a combination thereof.

In some embodiments, the myomaker polypeptide can have a polypeptide sequence with an amino acid sequence identity to a wt-myomaker polypeptide (e.g., SEQ ID NO:50 or SEQ ID NO:53) of about 70%, about 75%, about 80%, about 85%, about 90%, about 91%, about 92%, about 93%, about 94%, about 95%, about 96%, about 97%, about 98%, about 99%, about 99.1%, about 99.2%, about 99.3%, about 99.4%, about 99.5%, about 99.6%, about 99.7%, about 99.8%, about 99.9%, about 99.95%, about 99.99%, less than about 100%, at least about 70%, at least about 80%, at least about 90%, at least about 95%, at least about 99%, or at least about 99.5%. In some embodiments, the myomaker polypeptide sequence has an amino acid sequence identity to SEQ ID

NO:50 or SEQ ID NO: 53 of about 70%, about 75%, about 80%, about 85%, about 90%, about 91%, about 92%, about 93%, about 94%, about 95%, about 96%, about 97%, about 98%, about 99%, about 99.1%, about 99.2%, about 99.3%, about 99.4%, about 99.5%, about 99.6%, about 99.7%, about 99.8%, about 99.9%, about 99.95%, about 99.99%, less than about 100%, at least about 70%, at least about 80%, at least about 90%, at least about 95%, at least about 99%, or at least about 99.5%. The amino acid sequence identity (e.g., percent identity) can be determined by any suitable method, such as using BLAST, BLAST-2, ALIGN, ALIGN-2, Clustal Omega, or Megalign software. Unless otherwise indicated, the amino acid sequence identity (e.g., percent identity) is determined using BLAST-2.

Nucleic acid molecules that encode for the myomaker polypeptide are termed "myomaker nucleic acid molecules." In certain embodiments, the myomaker nucleic acid molecule is included in a vector (e.g., a viral vector, a retroviral vector, a lentiviral vector, an adenoviral vector, an adeno-associated viral vector, a herpesviral vector, a chimeric viral vector, a plasmid, an expression vector, a conjugative vector, or a nonconjugative vector). In certain embodiments, the myomerger nucleic acid molecule is in the same vector as the myomaker nucleic acid molecule. In certain embodiments, the myomaker nucleic acid molecule is in a cell, such as an insect cell (e.g., an Sf9 cell) or mammalian cell (e.g., a human cell, a rat cell a mouse cell, a muscle cell, a non-muscle cell, a myoblast, a fibroblast, a C2C12 cell, a 10T ½ fibroblast, a NIH/3T3 cell, a CHO cell, a mesenchymal stem cell (MSC), a hematopoietic stem cell, a blood cell, a bone marrow cell, or an adipose stem cell). In certain embodiments, the myomerger nucleic acid molecule is in the same cell as the myomaker nucleic acid molecule.

In other embodiments, the myomaker nucleic acid molecule comprises one or more nucleic acid sequences that are not used to encode for the myomaker polypeptide (e.g., one or more introns). For example, the myomaker nucleic acid molecule can include one or more nucleic acid molecules as found in nature (e.g., including introns). In certain embodiments, the myomaker nucleic acid molecule differs from the one or more nucleic acid molecules in nature because the myomaker nucleic acid molecule does not include one or more introns. In some embodiments, the myomaker nucleic acid molecule is a cDNA molecule ("myomaker cDNA molecule"). In certain embodiments, the myomaker cDNA molecule is identical to a nucleic acid molecule found in nature. In other embodiments, the myomaker cDNA molecule is not identical to a nucleic acid molecule found in nature (e.g., due to the myomaker cDNA molecule not including one or more introns in the nucleic acid molecule found in nature).

In some embodiments, the myomaker nucleic acid molecule sequence has a sequence identity to a nucleic acid molecule encoding a wt-myomaker polypeptide (e.g., SEQ ID NO:56, SEQ ID NO:59, SEQ ID NO:62, SEQ ID NO:63, SEQ ID NO:64, or SEQ ID NO:65) of about 90%, about 91%, about 92%, about 93%, about 94%, about 95%, about 96%, about 97%, about 98%, about 99%, about 99.1%, about 99.2%, about 99.3%, about 99.4%, about 99.5%, about 99.6%, about 99.7%, about 99.8%, about 99.9%, about 99.95%, about 99.99%, less than about 100%, at least about 90%, at least about 95%, at least about 99%, or at least about 99.5%. In some embodiments, the myomaker nucleic acid molecule sequence has a sequence identity to SEQ ID NO: 56, SEQ ID NO:59, SEQ ID NO:62, SEQ ID NO:63, SEQ ID NO:64, or SEQ ID NO:65 of about 90%, about 91%, about 92%, about 93%, about 94%, about 95%, about 96%, about 97%, about 98%, about 99%, about 99.1%, about 99.2%, about 99.3%, about 99.4%, about 99.5%, about 99.6%, about 99.7%, about 99.8%, about 99.9%, about 99.95%, about 99.99%, less than about 100%, at least about 90%, at least about 95%, at least about 99%, or at least about 99.5%. Nonlimiting examples of wt-myomaker polypeptides and wt-myomaker nucleic acid molecules can be found in Table 2. The nucleic acid sequence identity (e.g., percent identity) can be determined by any suitable method, such as using BLAST, BLAST-2, ALIGN, ALIGN-2, Clustal Omega, CRISPor Megalign software. Unless otherwise indicated, the nucleic acid sequence identity (e.g., percent identity) is determined using BLAST-2.

In some embodiments, the myomaker nucleic acid molecule encodes for a myomaker polypeptide that has one or more modifications to wt-myomaker polypeptide in a hydrophobic region, in a hydrophilic region, or in a combination thereof.

The myomaker nucleic acid molecule can be made using any suitable technique, such as but not limited to, those found in WO 2014/210448 A1, chemical synthesis, enzymatic production or biological production. Chemical synthesis of a nucleic acid molecule can include, for example, a nucleic acid molecule made by *in vitro* chemical synthesis using phosphotriester, phosphite or phosphoramidite chemistry and solid phase techniques, or via deoxynucleoside H-phosphonate intermediates. Enzymatically produced nucleic acid molecules can be accomplished using any suitable method including but not limited to Polymerase Chain Reaction (PCR). Biologically produced nucleic acid molecules can be accomplished using any suitable method including but not limited to a recombinant nucleic acid produced (i.e., replicated) in a living cell, such as a recombinant DNA vector replicated in bacteria.

Modifications or changes made in the structure of the myomaker nucleic acid molecules and/or myomaker polypeptides can be used in the present invention. In certain embodiments, a myomaker polypeptide can be modified (e.g., by one or more insertions, one or more deletions, or one or more substitutions (e.g., conservative substitutions)). In some embodiments, the myomaker polypeptide which was modified does not have an appreciable loss (e.g., a decrease in a function of less than about 1%, less than about 5%, less than about 10%, less than about 25%, less than about 50%, less than about 75%, less than about 90%, less than about 95%, less than about 99%, or less than about 100%) of one or more functions of the unmodified myomaker polypeptide such as, for example, the ability to activate fusion of two cells, the ability to make a cell fusion capable (e.g., a protein confers fusion capable properties to a cell if upon adding the protein, the cell is capable of fusing to another cell if that other cell comprises myomaker and myomerger), the ability to confer fusogenicity to a cell (e.g., a protein confers fusogenic properties to a cell if upon adding the protein, the cell will fuse with another cell if that other cell comprises myomaker), the level of expression during embryonic development, the level of expression during myogenesis in adult organisms (e.g., older than embryonic), the level of induction of myogenesis in adult organisms (e.g., older than embryonic), the affinity for membranes, or the level of association with membrane compartment. In some embodiments, the myomaker polypeptide which was modified retains desired levels (e.g., at least about 20%, at least about 40%, at least about 50%, at least about 75%, at least about 80%, at least about 90%, at least about 95%, or at least about 99%) of one or more functions of the unmodified myomaker polypeptide, such as, for example, the ability to activate fusion of two cells, the ability to make a cell fusion capable (e.g., a protein confers fusion capable properties to a cell if upon adding the protein, the cell is capable of fusing to another cell if that other cell comprises myomaker and myomerger), the ability to confer fusogenicity to a cell (e.g., a protein confers fusogenic properties to a cell if upon adding the protein, the cell will fuse with another cell if that other cell comprises myomaker), the level of expression during embryonic development, the level of expression during myogenesis in adult organisms (e.g., older than embryonic), the level of induction of myogenesis in adult organisms (e.g., older than embryonic), the affinity for membranes, or the level of association with membrane compartment. In some embodiments, the myomaker polypeptide after modification has an increased level of one or more functions as compared to the unmodified myomaker polypeptide. Nucleic acid molecules can be designed to encode for such a modified myomaker polypeptide, and such nucleic acid molecules can be used in the present invention.

A "functional myomaker polypeptide" is defined as a myomaker polypeptide (e.g., a modified polypeptide) that has desired levels (e.g., at least about 20%, at least about 40%, at least about 50%, at least about 75%, at least about 80%, at least about 90%, at least about 95%, or at least about 99%, as compared to another myomaker polypeptide, such as a naturally occurring myomaker polypeptide) of one or more functions such as, for example, the ability to activate fusion of two cells, the ability to make a cell fusion capable (e.g., a protein confers fusion capable properties to a cell if upon adding the protein, the cell is capable of fusing to another cell if that other cell comprises myomaker and myomerger), the ability to confer fusogenicity to a cell (e.g., a protein confers fusogenic properties to a cell if upon adding the protein, the cell will fuse with another cell if that other cell comprises myomaker), the level of expression during embryonic development, the level of expression during myogenesis in adult organisms (e.g., older than embryonic), the level of induction of myogenesis in adult organisms (e.g., older than embryonic), the affinity for membranes, or the level of association with membrane compartment. In some embodiments, the function myomaker polypeptide has an increased level of one or more functions as compared to another myomaker polypeptide (e.g., a naturally occurring myomaker polypeptide). Nucleic acid molecules can be designed to encode for functional myomaker polypeptides, and such nucleic acid molecules can be used in the present invention.

A "functionally equivalent myomaker polypeptide" is defined as a myomaker polypeptide that has been modified (e.g., by one or more insertions, one or more deletions, or one or more substitutions (e.g., conservative substitutions)) from an original myomaker polypeptide and that modified myomaker polypeptide retains desired levels (e.g., at least about 20%, at least about 40%, at least about 50%, at least about 75%, at least about 80%, at least about 90%, at least about 95%, or at least about 99%) of one or more functions of the original myomaker polypeptide, such as, for example, the ability to activate fusion of two cells, the ability to make a cell fusion capable (e.g., a protein confers fusion capable properties to a cell if upon adding the protein, the cell is capable of fusing to another cell if that other cell comprises myomaker and myomerger), the ability to confer fusogenicity to a cell (e.g., a protein confers fusogenic properties to a cell if upon adding the protein, the cell will fuse with another cell if that other cell comprises myomaker), the level of expression during embryonic development, the level of expression during myogenesis in adult organisms (e.g., older than embryonic), the level of induction of myogenesis in adult organisms (e.g., older than embryonic), the affinity for membranes, or the level of association with membrane compartment. In some embodiments, the functionally equivalent myomaker polypeptide can have an increased level of one or more functions compared to the original myomaker polypeptide. Nucleic acid molecules can be designed to encode for functionally equivalent myomaker polypeptides, and such nucleic acid molecules can be used in the present invention.

In certain embodiments, the shorter the length of a myomerger polypeptide, the fewer the modifications (e.g., substitutions) that can be made within the polypeptide while retaining, for example, a desired level of a chosen function. In some instances, longer domains can have a greater number of such changes while retaining, for example, a desired level of a chosen function. In other embodiments, a full-length polypeptide can have more tolerance for a fixed number of changes while retaining, for example, a desired level of a chosen function, as compared to a shorter length of that polypeptide.

The design of substitutions can take many forms, including but not limited to those described herein. In some embodiments, the hydropathic index of amino acids may be considered in designing substitutions. In the hydropathic index, each amino acid is assigned a hydropathic index on the basis of their hydrophobicity or charge characteristics, as follows: isoleucine (+4.5); valine (+4.2); Leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); or arginine (-4.5). In some instances, certain amino acids may be substituted for other amino acids having a similar hydropathic index. In making changes based upon the hydropathic index, the substitution of amino acids with hydropathic indices can be made with amino acids that have an index difference of no more than ±2, no more than ±1, or no more than ±0.5.

In some embodiments, substitutions can also be made based on hydrophilicity values. As detailed in U.S. Patent 4,554,101, the following hydrophilicity values have been assigned to amino acid residues: arginine (+3.0); lysine (+3.0); aspartate (+3.0 ± 1); glutamate (+3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). In making changes based upon similar hydrophilicity values, the substitution of amino acids with hydrophilicity values can be made with amino acids that have a value of no more than ±2, no more than ±1, or no more than ±0.5.

A "conservative substitution" in an amino acid sequence or polypeptide indicates that a given amino acid residue is replaced by a residue having similar physiochemical characteristics (e.g., no more than ±1 when based on hydropathic index or no more than ±1 when base on hydrophilicity values). Examples of conservative substitutions include (a) substitution of one aliphatic residue for another with an aliphatic residue, (b) substitution of one of Ile, Val, Leu, or Ala for one another of Ile, Val, Leu, or Ala, (c) substitution of one of Gly, Ile, Val, Leu, or Ala for one another of Gly, Ile, Val, Leu, or Ala, (d) substitution of one polar residue for another polar residue, (e) substitution of one of Lys and Arg with another of Lys and Arg, (f) substitution of one of Glu and Asp with another of Glu and Asp, (g) substitution of one of Gln and Asn with another of Gln and Asn, (h) substitution of one hydroxyl or sulfur containing residue with another hydroxyl or sulfur containing residue, (i) substitution of one of Ser, Cys, Thr, or Met with another of Ser, Cys, Thr, or Met, (j) substitution of one aromatic residue for another with an aromatic residue, (k) substitution of one of Phe, Tyr, or Trp with another of Phe, Tyr, or Trp, (l) substitution of one basic residue for another basic residue, (m) substitution of one of His, Lys, or Arg with another of His, Lys, or Arg, (n) substitution of an acidic/amide residue with another acidic/amide residue, (o) substitution of one of Asp, Glu, Asn, or Gln with another of Asp, Glu, Asn, or Gln, (p) substitution of a residue with another residue of a similar size, and (q) substitution of one of Ala, Gly, or Ser with another of Ala, Gly, or Ser. In some embodiments, each amino acid in a hydrophobic region of a polypeptide can be substituted with conservative substitutions (e.g., any combination of conservative substitutions relating to hydrophobic residues).

While discussion has focused on amino acid changes, it will be appreciated that these changes may occur by alteration of the encoding DNA; taking into consideration also that the genetic code is degenerate and that two or more codons may code for the same amino acid. Tables A and B of amino acids and their codons are presented herein for use in such embodiments, as well as for other uses, such as in the design of probes and primers and the like.

The term "functionally equivalent codon" is used herein to refer to codons that encode the same amino acid, such as the six codons for arginine or serine.

In certain instances, the nucleic acid molecule can be engineered to contain distinct sequences while at the same time retaining the capacity to encode a desired inventive polypeptide. In some embodiments, this can be accomplished owing to the degeneracy of the genetic code (i.e., the presence of multiple codons) which encode for the same amino acids. In other instances, it can be accomplished by including, adding, or excluding introns in the nucleic acid molecule.

In certain embodiments, a restriction enzyme recognition sequence can be introduced into a nucleic acid sequence while maintaining the ability of that nucleic acid molecule to encode a desired polypeptide. In other embodiments, a CRISPR system (e.g., a CRISPR system comprising one or more of guide RNA, crRNA, tracrRNA, sgRNA, DNA repair template, and Cas protein, such as but not limited to CRISPR/Cas9) can be used to introduce a nucleic acid molecule while maintaining the ability of that nucleic acid molecule to encode a desired polypeptide.

It will also be understood that amino acid sequences (e.g., polypeptides) and nucleic acid sequences may include additional residues, such as additional N- or C-terminal amino acids or 5' or 3' sequences, and yet still be essentially as set forth in one of the sequences disclosed herein, so long as the sequence meets the criteria set forth above, including the maintenance of biological activity where polypeptide expression is concerned. The addition of terminal sequences particularly applies to nucleic acid sequences that may, for example, include various non-coding sequences flanking either of the 5' or 3' portions of the coding region or may include various internal sequences, (i.e., introns) which can occur within genes.

Some embodiments use synthesis of polypeptides *in cyto*, via transcription and translation of appropriate nucleic acid molecules (e.g., nucleic acid sequences as discussed herein). These polypeptides will include the twenty "natural" amino acids, and post-translational modifications thereof. *In vitro* peptide synthesis permits the use of modified or unusual amino acids. In some embodiments, the myomaker polypeptide encompasses modifications (e.g., one or more substitutions or one or more insertions) that include one or more modified or unusual amino acids. A table of exemplary, but not limiting, modified or unusual amino acids is provided in Table C (disclosed herein).

The presently disclosed subject matter further includes a method of producing a myomaker polypeptide (e.g., a mutant myomaker polypeptide or a wt-myomaker polypeptide). Any suitable method can used to make the myomaker polypeptides including but not limited to expression through any suitable molecular biological technique (e.g., using a prokaryotic or eukaryotic expression system), isolation from a source in nature, or chemical synthesis. Eukaryotic expression systems include plant-based systems; insect cell systems via recombinant baculoviruses; whole insect systems via recombinant baculoviruses; genetically engineered yeast systems, including but not limited to *Saccharomyces sp.* and *Picchia spp.* ; and mammalian cell systems, including but not limited to C2C12 cells, 10T 1/2 fibroblasts, NIH/3T3 fibroblasts, mesenchymal stem cells (MSCs), hematopoietic stem cells, Chinese hamster ovary cells or other cell lines commonly used for industrial scale expression of recombinant proteins. In some embodiments, useful plant-based expression systems can include transgenic plant systems. In some embodiments, useful plant-based expression systems can include transplastomic plant systems.

In some embodiments, a method of producing the myomaker polypeptide includes providing a host cell comprising a myomaker nucleic acid molecule, as disclosed herein, operatively linked to a promoter operable under conditions whereby the encoded myomaker polypeptide is expressed; and recovering the myomaker polypeptide from the host cell.

### Cells Including Modified Cells

Some embodiments of the invention include cells such as modified cells. In certain embodiments, a modified cell is a cell that comprises one or more modifications of a cell, where at least one of the one or more modifications was implemented by a human (e.g., by human activity, either directly or indirectly). In some embodiments, the cell to be modified can be an unmodified cell or can be a cell that has been previously modified (e.g. modified as disclosed herein). A cell can be modified in any desired manner, including but not limited to (a) adding a nucleic acid molecule such as but not limited to one or more nucleic acid molecules disclosed herein, (b) diminishing the effect of one or more nucleic acid molecules (e.g., a naturally occurring nucleic acid molecule or an added nucleic acid molecule) such as a gene, (c) adding one or more polypeptides, including but not limited to polypeptides disclosed herein, (d) diminishing the effect of one or more polypeptides (e.g., a naturally occurring polypeptide or an added polypeptide), or (e) a combination thereof. In some instances, a modified cell can result from a further modification of another modified cell.

Adding a nucleic acid molecule to modify a cell can be accomplished using any suitable method including but not limited to one or more of transformation (as used herein transfection methods are encompassed by the term transformation), viral transformation (e.g., using a viral vector, a retroviral vector, a lentiviral vector, an adenoviral vector, an adeno-associated viral vector, a herpesviral vector, a chimeric viral vector, a plasmid, a cosmid, an artificial chromosome, a bacteriophage, a virus, an animal virus, a plant virus, an expression vector, a conjugative vector, or a nonconjugative vector), injection, microinjection, electroporation, sonication, calcium ion treatment, calcium phosphate precipitation, PEG-DMSO treatment, DE-Dextran treatment, liposome mediated transformation, or a receptor mediated transformation. Adding a polypeptide to modify a cell can be accomplished using any suitable method including but not limited to one or more of injection, microinjection, electroporation, sonication, calcium ion treatment, calcium phosphate precipitation, PEG-DMSO treatment, DE-Dextran treatment, or liposome mediated. The added nucleic acid molecule can be part of a vector (e.g., a viral vector, a retroviral vector, a lentiviral vector, an adenoviral vector, an adeno-associated viral vector, a herpesviral vector, a chimeric viral vector, a plasmid, a cosmid, an artificial chromosome, a bacteriophage, an animal virus, a plant virus, an expression vector, a conjugative vector, or a nonconjugative vector), a plasmid, a cosmid, an artificial chromosome, a bacteriophage, a virus, an animal virus, or a plant virus. In some embodiments, the added nucleic acid molecule is exogenous; "exogenous" means (a) that the added nucleic acid molecule originates from outside of the cell (e.g., is foreign to the cell) or (b) that the added nucleic acid molecule can be found inside the cell, but the added nucleic acid molecule is placed in the cell where it is not normally found (e.g., a different part of the chromosome or on an added plasmid). In some embodiments, the added polypeptide is exogenous; "exogenous" in this context means that the added polypeptide originates from outside of the cell (e.g., is foreign to the cell).

In some embodiments, the modification to the cell can be the diminution of the effect of a nucleic acid molecule in the cell; the nucleic acid molecule can be added to the cell or not, or can be exogenous. Diminishing the effect of a nucleic acid molecule includes but is not limited to decreasing (e.g., stopping) the expression of a polypeptide (e.g., myomaker, myomerger, or both) or expressing a less active form of a polypeptide (e.g., by changing the polypeptide's amino acid sequence or expressing only a fragment of a polypeptide). Diminishing the effect of a nucleic acid molecule can be accomplished using any suitable method including but not limited to removal of the nucleic acid molecule (e.g., from a chromosome or from a plasmid), removal of one or more parts of the nucleic acid molecule (e.g., from a chromosome or from a plasmid), altering the nucleic acid sequence of the nucleic acid molecule, diminishing or preventing transcription of the nucleic acid molecule (e.g., via a repressor, inhibitor, blocker (e.g., via a molecule that blocks part of the transcription), or stabilization of a non-transcribing form), or diminishing or preventing translation (e.g., via an inhibitor or an RNA inhibitor).

The cell to be modified can be any suitable cell including but not limited to an insect cell (e.g., an Sf9 cell), a vertebrate cell, or a mammalian cell (e.g., a human cell, a rat cell a mouse cell, a muscle cell, a non-muscle cell, a myoblast, a fibroblast, a C2C12 cell, a 10T ½ fibroblast, a NIH/3T3 cell, a CHO cell, a mesenchymal stem cell (MSC), a hematopoietic stem cell, a blood cell, a bone marrow cell, a stem cell, or an adipose stem cell). In certain embodiments, an unmodified cell can be any suitable cell including but not limited insect cell, a vertebrate cell, or a mammalian cell (e.g., a human cell, a rat cell a mouse cell, a muscle cell, a non-muscle cell, a myoblast, a fibroblast, a NIH/3T3 cell, a CHO cell, a mesenchymal stem cell (MSC), a hematopoietic stem cell, a blood cell, a bone marrow cell, a stem cell, or an adipose stem cell).

In some embodiments, a modified cell can be but is not limited to a modified animal cell, a modified vertebrate cell, a modified mammalian cell, a modified human cell, a modified rat cell, a modified mouse cell, a modified muscle cell, a modified non-muscle cell, a modified myoblast, a modified fibroblast, a C2C12 cell, a modified C2C12 cell, a 10T ½ fibroblast, a modified 10T ½ fibroblast, a modified NIH/3T3 cell, a modified CHO cell, a modified mesenchymal stem cell (MSC), a modified hematopoietic stem cell, a modified blood cell, a modified bone marrow cell, a modified stem cell, or a modified adipose stem cell. In other embodiments, the modified cell is a modified non-muscle cell (e.g., a modified fibroblast, a 10T ½ fibroblast, a modified 10T ½ fibroblast, a modified NIH/3T3 cell, a modified CHO cell, a modified mesenchymal stem cell (MSC), a modified hematopoietic stem cell, a modified blood cell, a modified bone marrow cell, a modified stem cell, or a modified adipose stem cell).

In some embodiments, the modified cell is a non-muscle cell with a myomerger nucleic acid molecule added (e.g., where the myomerger nucleic acid molecule is exogenous), a stem cell with a myomerger nucleic acid molecule added (e.g., where the myomerger nucleic acid molecule is exogenous), a fibroblast with a myomerger nucleic acid molecule added (e.g., where the myomerger nucleic acid molecule is exogenous), a muscle cell with a myomerger nucleic acid molecule added (e.g., where the myomerger nucleic acid molecule is exogenous), or a myoblast cell with a myomerger nucleic acid molecule added (e.g., where the myomerger nucleic acid molecule is exogenous).

In other embodiments, the modified cell is a non-muscle cell with a myomaker nucleic acid molecule added (e.g., where the myomaker nucleic acid molecule is exogenous), a stem cell with a myomaker nucleic acid molecule added (e.g., where the myomaker nucleic acid molecule is exogenous), a fibroblast with a myomaker nucleic acid molecule added (e.g., where the myomaker nucleic acid molecule is exogenous), a muscle cell with a myomaker nucleic acid molecule added (e.g., where the myomaker nucleic acid molecule is exogenous), or a myoblast cell with a myomaker nucleic acid molecule added (e.g., where the myomaker nucleic acid molecule is exogenous),

In still other embodiments, the modified cell is a non-muscle cell with a myomerger nucleic acid molecule added (e.g., where the myomerger nucleic acid molecule is exogenous) and a myomaker nucleic acid molecule added (e.g., where the myomaker nucleic acid molecule is exogenous), a stem cell with a myomerger nucleic acid molecule added (e.g., where the myomerger nucleic acid molecule is exogenous) and a myomaker nucleic acid molecule added (e.g., where the myomaker nucleic acid molecule is exogenous), a fibroblast with a myomerger nucleic acid molecule added (e.g., where the myomerger nucleic acid molecule is exogenous) and a myomaker nucleic acid molecule added (e.g., where the myomaker nucleic acid molecule is exogenous), a muscle cell with a myomerger nucleic acid molecule added (e.g., where the myomerger nucleic acid molecule is exogenous) and a myomaker nucleic acid molecule added (e.g., where the myomaker nucleic acid molecule is exogenous), or a myoblast cell with a myomerger nucleic acid molecule added (e.g., where the myomerger nucleic acid molecule is exogenous) and a myomaker nucleic acid molecule added (e.g., where the myomaker nucleic acid molecule is exogenous).

The modified cell can be prepared using any suitable method including but not limited to those disclosed herein.

### Compositions including Pharmaceutical Compositions

One or more inventive polypeptides (e.g., a wt-myomerger polypeptide or mutant myomerger polypeptide) or one or more myomerger nucleic acid molecules (e.g., in the form of a bare nucleic acid molecule, a vector, a virus, a plasmid or any suitable form) can be part of a composition and can be in an amount (by weight of the total composition) of at least about 0.0001%, at least about 0.001%, at least about 0.10%, at least about 0.15%, at least about 0.20%, at least about 0.25%, at least about 0.50%, at least about 0.75%, at least about 1%, at least about 10%, at least about 25%, at least about 50%, at least about 75%, at least about 90%, at least about 95%, at least about 99%, at least about 99.99%, no more than about 75%, no more than about 90%, no more than about 95%, no more than about 99%, or no more than about 99.99%, from about 0.0001% to about 99%, from about 0.0001% to about 50%, from about 0.01% to about 95%, from about 1% to about 95%, from about 10% to about 90%, or from about 25% to about 75%. In certain embodiments, a myomaker polypeptide, myomaker nucleic acid molecule (e.g., added as another vector or as part of the vector comprising myomerger nucleic acid), or both can be part of the composition (e.g., together with a myomerger polypeptide in the composition or a myomerger nucleic acid molecule in the composition) at any amount indicated herein (e.g., indicated above). In certain embodiments, cells, such as modified cells (e.g., as disclosed herein) can be part of the composition at any amount indicated herein (e.g., indicated above).

One or more inventive polypeptides (e.g., a wt-myomerger polypeptide or mutant myomerger polypeptide) or one or more myomerger nucleic acid molecules (e.g., in the form of a bare nucleic acid molecule, a vector, a virus, a plasmid or any suitable form) can be purified or isolated in an amount (by weight of the total composition) of at least about 0.0001%, at least about 0.001%, at least about 0.10%, at least about 0.15%, at least about 0.20%, at least about 0.25%, at least about 0.50%, at least about 0.75%, at least about 1%, at least about 10%, at least about 25%, at least about 50%, at least about 75%, at least about 90%, at least about 95%, at least about 99%, at least about 99.99%, no more than about 75%, no more than about 90%, no more than about 95%, no more than about 99%, no more than about 99.99%, from about 0.0001% to about 99%, from about 0.0001% to about 50%, from about 0.01% to about 95%, from about 1% to about 95%, from about 10% to about 90%, or from about 25% to about 75%. In some embodiments, isolated or purified means that impurities (e.g., cell components or unwanted solution components if chemically synthesized) were removed by one or more of any suitable technique (e.g., column chromatography, HPLC, centrifugation, fractionation, gel, precipitation, or salting out).

Some embodiments of the present invention include compositions comprising one or more inventive polypeptides (e.g., a wt-myomerger polypeptide or mutant myomerger polypeptide) or one or more myomerger nucleic acid molecules (e.g., in the form of a bare nucleic acid molecule, a vector, a virus, a plasmid or any suitable form). In certain embodiments, the composition is a pharmaceutical composition, such as compositions that are suitable for administration to animals (e.g., mammals, primates, monkeys, humans, canine, porcine, mice, rabbits, or rats). In some embodiments, there may be inherent side effects (e.g., it may harm the patient or may be toxic or harmful to some degree in some patients).

In some embodiments, one or more inventive polypeptides (e.g., a wt-myomerger polypeptide or mutant myomerger polypeptide) or one or more myomerger nucleic acid molecules (e.g., in the form of a bare nucleic acid molecule, a vector, a virus, a plasmid or any suitable form) can be part of a pharmaceutical composition and can be in an amount (by weight of the total composition) of at least about 0.0001%, at least about 0.001%, at least about 0.10%, at least about 0.15%, at least about 0.20%, at least about 0.25%, at least about 0.50%, at least about 0.75%, at least about 1%, at least about 10%, at least about 25%, at least about 50%, at least about 75%, at least about 90%, at least about 95%, at least about 99%, at least about 99.99%, no more than about 75%, no more than about 90%, no more than about 95%, no more than about 99%, no more than about 99.99%, from about 0.001% to about 99%, from about 0.001% to about 50%, from about 0.1% to about 99%, from about 1% to about 95%, from about 10% to about 90%, or from about 25% to about 75%. In certain embodiments, a myomaker polypeptide, myomaker nucleic acid molecule (e.g., added as another vector or as part of the vector comprising myomerger nucleic acid), or both can be part of the pharmaceutical composition (e.g., together with a myomerger polypeptide in the pharmaceutical composition or a myomerger nucleic acid molecule in the pharmaceutical composition) at any amount indicated herein (e.g., indicated above). In some embodiments, cells, such as modified cells (e.g., as disclosed herein) can be part of the pharmaceutical composition at any amount indicated herein (e.g., indicated above).

In some embodiments, the pharmaceutical composition can be presented in a dosage form which is suitable for the topical, subcutaneous, intrathecal, intraperitoneal, oral, parenteral, rectal, cutaneous, nasal, vaginal, or ocular administration route. In other embodiments, the pharmaceutical composition can be presented in a dosage form which is suitable for parenteral administration, a mucosal administration, intravenous administration, subcutaneous administration, topical administration, intradermal administration, oral administration, sublingual administration, intranasal administration, or intramuscular administration. The pharmaceutical composition can be in the form of, for example, tablets, capsules, pills, powders granulates, suspensions, emulsions, solutions, gels (including hydrogels), pastes, ointments, creams, plasters, drenches, delivery devices, suppositories, enemas, injectables, implants, sprays, aerosols or other suitable forms.

In some embodiments, the pharmaceutical composition can include one or more formulary ingredients. A "formulary ingredient" can be any suitable ingredient (e.g., suitable for the drug(s), for the dosage of the drug(s), for the timing of release of the drugs(s), for the disease, for the disease state, for the organ, or for the delivery route) including, but not limited to, water (e.g., boiled water, distilled water, filtered water, pyrogen-free water, or water with chloroform), sugar (e.g., sucrose, glucose, mannitol, sorbitol, xylitol, or syrups made therefrom), ethanol, glycerol, glycols (e.g., propylene glycol), acetone, ethers, DMSO, surfactants (e.g., anionic surfactants, cationic surfactants, zwitterionic surfactants, or nonionic surfactants (e.g., polysorbates)), oils (e.g., animal oils, plant oils (e.g., coconut oil or arachis oil), or mineral oils), oil derivatives (e.g., ethyl oleate , glyceryl monostearate, or hydrogenated glycerides), excipients, preservatives (e.g., cysteine, methionine, antioxidants (e.g., vitamins (e.g., A, E, or C), selenium, retinyl palmitate, sodium citrate, citric acid, chloroform, or parabens, (e.g., methyl paraben or propyl paraben)), or combinations thereof. In some embodiments, the concentration of any individual formulary ingredient in a composition (e.g., pharmaceutical composition) can be in an amount (by weight of the total composition) of at least about 0.0001%, at least about 0.001%, at least about 0.10%, at least about 0.15%, at least about 0.20%, at least about 0.25%, at least about 0.50%, at least about 0.75%, at least about 1%, at least about 10%, at least about 25%, at least about 50%, at least about 75%, at least about 90%, at least about 95%, at least about 99%, at least about 99.99%, no more than about 75%, no more than about 90%, no more than about 95%, no more than about 99%, no more than about 99.99%, from about 0.001% to about 99%, from about 0.001% to about 50%, from about 0.1% to about 99%, from about 1% to about 95%, from about 10% to about 90%, or from about 25% to about 75%. In some embodiments, the concentration of at least one formulary ingredient is not that same as that found in the natural system in which inventive polypeptide (e.g., wt-myomerger polypeptide) is found. In some embodiments, the concentration of at least one formulary ingredient is not that same as that found in one or more natural systems (e.g., any natural system found in nature) in which the nucleic acid molecule which encodes an inventive polypeptide (e.g., wt-myomerger polypeptide) is found.

In certain embodiments, pharmaceutical compositions can be formulated to release the active ingredient (e.g., one or more compounds of Formula (I)) substantially immediately upon the administration or any substantially predetermined time or time after administration. Such formulations can include, for example, controlled release formulations such as various controlled release compositions and coatings.

Other formulations (e.g., formulations of a pharmaceutical composition) can, in certain embodiments, include those incorporating the drug (or control release formulation) into food, food stuffs, feed, or drink.

### Methods of Using Cells Including Modified Cells

Some embodiments of the invention include methods of using cells, such as modified cells.

Some embodiments of the invention include methods for fusing two or more cells comprising contacting a first cell with a second cell to form a third cell, where the first cell is a modified cell (e.g., as disclosed herein). In certain embodiments, the term "fuse" (and related terms such as "fusing", "fusion" etc.) means to combine two cells to form a a third cell. In certain embodiments of fusion, fusion results in a multinuclear cell (e.g., syncytium). In certain embodiments of fusion, fusion does not result in a multinuclear cell. In some embodiments, the first cell comprises a first myomerger polypeptide, a first myomaker polypeptide, or both. In some embodiments, the first cell comprises a first myomerger polypeptide and a first myomaker polypeptide. In other embodiments, the second cell comprises a second myomaker polypeptide, a second myomerger polypeptide, or both. In some embodiments, the second cell comprises a second myomaker polypeptide and a second myomerger polypeptide. In other embodiments, the third cell is a multinucleated cell. In still other embodiments, the third cell is not a multinucleated cell.

In some embodiments, the first cell, the second cell, or both can be any suitable cell including but not limited to an insect cell (e.g., an Sf9 cell), a vertebrate cell, or a mammalian cell (e.g., a human cell, a rat cell a mouse cell, a muscle cell, a non-muscle cell, a myoblast, a fibroblast, a C2C12 cell, a 10T ½ fibroblast, a NIH/3T3 cell, a CHO cell, a dendritic cell, a cancer cell, a mesenchymal stem cell (MSC), a hematopoietic stem cell, a blood cell, a bone marrow cell, a stem cell, or an adipose stem cell). In some embodiments, the first cell and the second cell are the same type of cell (e.g., homotypic cell fusion which can in certain instances form syncytium). In other embodiments, the first cell and the second cell are different types of cell (e.g., heterotypic cell fusion). Cell fusion can, in some instances, result in nuclear fusion. In other instances, cell fusion does not result in nuclear fusion.

In some embodiments, the first cell, the second, cell or both can be a modified cell that can be but is not limited to a modified animal cell, a modified vertebrate cell, a modified mammalian cell, a modified human cell, a modified rat cell, a modified mouse cell, a modified muscle cell, a modified non-muscle cell, a modified myoblast, a modified fibroblast, a C2C12 cell, a modified C2C12 cell, a 10T ½ fibroblast, a modified 10T ½ fibroblast, a modified NIH/3T3 cell, a modified CHO cell, a modified dendritic cell, a modified cancer cell, a modified mesenchymal stem cell (MSC), a modified hematopoietic stem cell, a modified blood cell, a modified bone marrow cell, a modified stem cell, or a modified adipose stem cell. In other embodiments, the first cell, the second, cell or both can be a modified cell that is a modified non-muscle cell (e.g., a modified fibroblast, a 10T ½ fibroblast, a modified 10T ½ fibroblast, a modified NIH/3T3 cell, a modified CHO cell, a modified dendritic cell, a modified cancer cell, a modified mesenchymal stem cell (MSC), a modified hematopoietic stem cell, a modified blood cell, a modified bone marrow cell, a modified stem cell, or a modified adipose stem cell).

In certain embodiments of the method, the first cell is a non-muscle cell, the second cell is a non-muscle cell, or both. In other embodiments of the method, the first cell is a non-muscle cell and the second cell is a muscle cell.

In some instances of the method, the second cell is an isolated muscle cell (e.g., myoblast). In still other embodiments of the method, the second cell is a muscle cell or is a cell that is part of a muscle, muscle tissue, or non-muscle tissue. In some embodiments, the muscle, muscle tissue, or non-muscle tissue is diseased. In other embodiments, the muscle, muscle tissue, or non-muscle tissue (e.g., diseased or not diseased) is part of the circulation system (e.g., heart), respiratory system (e.g., diaphragm), head, neck, gastrointestinal system (e.g., tongue, esophageal muscles, or intestinal muscles), skeletal muscles, or genitourinary tract.

The contacting of the cells in the method can occur by any suitable manner, such as but not limited to those disclosed herein. For example, the contacting can occur *in vitro* or the contacting can occur *in vivo.*

Some embodiments of the invention include methods for delivering a gene of interest comprising contacting a first cell with a second cell, which fuse to form a third cell. In certain embodiments, the term "fuse" (and related terms such as "fusing", "fusion" etc.) means to combine two cells to form a a third cell. In certain embodiments of fusion, fusion results in a multinuclear cell (e.g., syncytium). In certain embodiments of fusion, fusion does not result in a multinuclear cell. In certain embodiments, the first cell is a modified cell (e.g., as disclosed here) and can comprise (a) a gene of interest and (b) a first myomerger polypeptide, a first myomaker polypeptide or both. In certain embodiments, the first cell can be a modified cell (e.g., as disclosed here) and can comprise a first myomerger polypeptide, a first myomaker polypeptide, and a gene of interest. In other embodiments, the second cell can comprise a second myomaker polypeptide, a second myomerger polypeptide, or both. In other embodiments, the second cell can comprise a second myomaker polypeptide and a second myomerger polypeptide. In other embodiments, the third cell is a multinucleated cell. In other embodiments, the third cell is not a multinucleated cell. In some embodiments, the first cell and the second cell are the same type of cell (e.g., homotypic cell fusion which can in certain instances form syncytium). In yet other embodiments, the first cell and the second cell are different types of cell (e.g., heterotypic cell fusion). Cell fusion can, in some instances, result in nuclear fusion. In other instances, cell fusion does not result in nuclear fusion.

In some embodiments, the gene of interest is a nucleic acid sequence that encodes a polypeptide, a protein, or an oligopeptide (e.g., is not a myomerger polypeptide and is not a myomaker polypeptide). In certain embodiments, the gene of interest encodes for a therapeutic polypeptide, a therapeutic protein, or a therapeutic oligopeptide, where the gene of interest can be part of a treatment of a disease. In other embodiments, the gene of interest can be genomic DNA or can be cDNA.

In certain embodiments, the second cell can underexpress the gene of interest, does not express the gene of interest, expresses a defective version of the gene of interest, or a combination thereof. In other embodiments, the second cell does not express the gene of interest.

In some embodiments, the first cell, the second cell, or both can be any suitable cell including but not limited to an insect cell (e.g., an Sf9 cell), a vertebrate cell, or a mammalian cell (e.g., a human cell, a rat cell a mouse cell, a muscle cell, a non-muscle cell, a myoblast, a fibroblast, a C2C12 cell, a 10T ½ fibroblast, a NIH/3T3 cell, a CHO cell, a dendritic cell, a cancer cell, a mesenchymal stem cell (MSC), a hematopoietic stem cell, a blood cell, a bone marrow cell, a stem cell, or an adipose stem cell).

In some embodiments, the first cell, the second, cell or both can be a modified cell that can be but is not limited to a modified animal cell, a modified vertebrate cell, a modified mammalian cell, a modified human cell, a modified rat cell, a modified mouse cell, a modified muscle cell, a modified non-muscle cell, a modified myoblast, a modified fibroblast, a C2C12 cell, a modified C2C12 cell, a 10T ½ fibroblast, a modified 10T ½ fibroblast, a modified NIH/3T3 cell, a modified CHO cell, a modified dendritic cell, a modified cancer cell, a modified mesenchymal stem cell (MSC), a modified hematopoietic stem cell, a modified blood cell, a modified bone marrow cell, a modified stem cell, or a modified adipose stem cell. In other embodiments, the first cell, the second, cell or both can be a modified cell that is a modified non-muscle cell (e.g., a modified fibroblast, a 10T ½ fibroblast, a modified 10T ½ fibroblast, a modified NIH/3T3 cell, a modified CHO cell, a modified dendritic cell, a modified cancer cell, a modified mesenchymal stem cell (MSC), a modified hematopoietic stem cell, a modified blood cell, a modified bone marrow cell, a modified stem cell, or a modified adipose stem cell).

In certain embodiments of the method, the first cell is a non-muscle cell, the second cell is a non-muscle cell, or both. In other embodiments of the method, wherein the first cell is a non-muscle cell and the second cell is a muscle cell.

In some instances of the method, the second cell is an isolated muscle cell (e.g., myoblast). In still other embodiments of the method, the second cell is a muscle cell or is a cell that is part of a muscle, muscle tissue, or non-muscle tissue. In some embodiments, the muscle, muscle tissue, or non-muscle tissue is diseased. In other embodiments, the muscle, muscle tissue, or non-muscle tissue (e.g., diseased or not diseased) is part of the circulation system (e.g., heart), respiratory system (e.g., diaphragm), head, neck, gastrointestinal system (e.g., tongue, esophageal muscles, or intestinal muscles), skeletal muscles, or genitourinary tract.

The contacting of the cells in the method can occur by any suitable manner such as but not limited to those disclosed herein. For example, the contacting can occur *in vitro* or the contacting can occur *in vivo.* In other exemplary embodiments, contacting can occur *ex vivo* and the method can further comprise placing (e.g., implanting, injecting, or grafting) the third cell in an animal. The placing can be done using any suitable mechanism, such as by any suitable administration route.

Animals include but are not limited to mammals, primates, monkeys (e.g., macaque, rhesus macaque, or pig tail macaque), humans, canine, feline, bovine, porcine, avian (e.g., chicken), mice, rabbits, and rats. As used herein, the term "subject" refers to both human and animal subjects.

In certain embodiments, the method to delivery of a gene of interest can be part of a treatment of a disease. In some embodiments, the disease can be a disease, such as but not limited to, diseases where cells underexpress the gene of interest, do not express the gene of interest, express a defective version of the gene of interest, or a combination thereof. In some embodiments, the disease can be a non-muscle-related disease, such as but not limited to, non-muscle diseases where cells underexpress the gene of interest, do not express the gene of interest, express a defective version of the gene of interest, or a combination thereof. In some embodiments, the disease can be a muscle-related disease, such as but not limited to, muscle diseases where cells underexpress the gene of interest, do not express the gene of interest, express a defective version of the gene of interest, or a combination thereof. In certain embodiments, the treated disease can be a myopathy, muscular dystrophy, amyotrophic lateral sclerosis (ALS or also called Lou Gehrig's disease), glycogen storage disease type II (also called Pompe disease), rhabdomyosarcoma (RMS), sarcopenia, or a combination thereof. In some embodiments, the disease can be cancer. As used herein, the term "treating" (and its variations, such as "treatment") is to be considered in its broadest context. In particular, the term "treating" does not necessarily imply that an animal is treated until total recovery. Accordingly, "treating" includes amelioration of the symptoms, relief from the symptoms or effects associated with a condition, decrease in severity of a condition, or preventing, preventively ameliorating symptoms, or otherwise reducing the risk of developing a particular condition. As used herein, reference to "treating" an animal includes but is not limited to prophylactic treatment and therapeutic treatment. Any of the methods or compositions (e.g., pharmaceutical compositions) described herein can be used to treat an animal.

In yet other embodiments, the delivery of the gene of interest can occur by any suitable administration route. Administration routes can be, but are not limited to the oral route, the parenteral route, the cutaneous route, the nasal route, the rectal route, the vaginal route, and the ocular route. In other embodiments, administration routes can be parenteral administration, a mucosal administration, intravenous administration, depot injection, subcutaneous administration, topical administration, intradermal administration, oral administration, sublingual administration, intranasal administration, or intramuscular administration (e.g., intramuscular injection). In certain embodiments, the delivery comprises an injection or an intramuscular injection. In certain embodiments, the delivery comprises an injection comprising the first cell, the second cell, or both (e.g., in a composition or in a pharmaceutical composition). In other embodiments, the delivery comprises an intramuscular injection comprising the first cell, the second cell, or both (e.g., in a composition or in a pharmaceutical composition).

In still other embodiments, the delivery can further comprise one or more of the contacting steps.

The presently-disclosed subject matter is further illustrated by the following specific but non-limiting examples. The following examples may include compilations of data that are representative of data gathered at various times during the course of development and experimentation related to the present invention.

### EXAMPLES

### Materials and Methods

### Cell Culture

C2C12 cells, 10T 1/2 fibroblasts, and NIH/3T3 fibroblasts were purchased from American Type Culture Collection and propagated in DMEM (Gibco) containing 10% heat-inactivated bovine growth serum (BGS) and supplemented with antibiotics. C2C12 cells were differentiated by switching to media containing 2% heat-inactivated horse serum (HS) and antibiotics. MSCs were a gift from Jose Cancelas. GONZALEZ-NIETO et al., "Connexin-43 in the osteogenic bm niche regulates its cellular composition and the bidirectional traffic of hematopoietic stem cells and progenitors" Blood (2012) Vol. 119, pp. 5144-5154.

### Bioinformatic Analysis

Microarray data from the GEO DataSet GSE34907³⁴ was interrogated using GEO2R analysis to identify 1826 genes displaying an increase greater than 1 log fold-change in MyoD-expressing fibroblasts. In parallel, a transcriptional profile of 10T 1/2 fibroblasts transduced with empty virus was generated using RNA-seq analysis (paired-end library layout using Illumina sequencing platform) and a list of all genes with RPKM values below 1.5 compiled using Strand NGS software (Ver. 2.6; Build: Mouse mm10 (UCSC) using Ensembl transcript annotations). These two gene lists were then compared to generate a final tally comprised of 531 genes that were both upregulated in MyoD-expressing fibroblasts and had low or no detectable expression in 10T 1/2 fibroblasts. Finally, the top 100 genes were interrogated for genes that contain transmembrane domains and not previously studied for their role during myoblast fusion.

### Animals

We used a dual sgRNA targeting strategy to create *Gm7325*^{*-*/*-*} mice. We selected the sgRNAs according to the on- and off-target scores from the web tool CRISPOR. (HAEUSSLER et al. "Evaluation of off-target and on-target scoring algorithms and integration into the guide rna selection tool crispor" Genome Biol (2016) Vol. 17, article 148 (12 pages).) The selected gRNAs were 5'-GCAGCGATCGAAGCACCATC-3' (SEQ ID NO: 1) and 5'-GAGGCCTCTCCAGAATCCGG-3' (SEQ ID NO: 2) that target exon 3 of *Gm 7325.* The sgRNAs were *in vitro* synthesized using the MEGAshortscript T7 kit (ThermoFisher) and purified by the MEGAclear Kit (ThermoFisher). sgRNAs (50 ng/ul of each) were mixed with 100 ng/ul Cas9 protein (ThermoFisher) and incubated at 37°C for 15 min to form a ribonucleoprotein complex. We then injected the mix into the cytoplasm of one-cell-stage embryos of the C57BL/6 genetic background using a piezo-driven microinjection technique. (YANG et al., "Generating genetically modified mice using crispr/cas-mediated genome engineering" Nat Protoc (2014) Vol. 9, pp. 1956-1968) Injected embryos were immediately transferred into the oviducal ampulla of pseudopregnant CD-1 females. Live born pups were genotyped by PCR with primers spanning the mutated region (Table E1). The edited allele was further confirmed by Sanger sequencing. One heterozygous founder was obtained and mated with WT C57B16 mice to eventually generate KO mice. The gender of analyzed embryos was not determined. *Mdx^{4cv}* mice were purchased from Jackson Laboratory (#002378) and male mice were used. Muscle overload of the plantaris muscle was achieved through bilateral synergistic ablation of soleus and gastrocnemius muscles. Specifically, the soleus and gastrocnemius muscles were exposed by making an incision on the posterior-lateral aspect of the lower limb. The distal and proximal tendons of the soleus, lateral and medial gastrocnemius were subsequently cut and carefully excised. All animal procedures were approved by Cincinnati Children's Hospital Medical Center's Institutional Animal Care and Use Committee.

**Table E1**

| Description | Forward Primer | Reverse Primer |
|---|---|---|
| 1. Genotyping for *Gm7325* mutation | GAAGGGAGGACTCCACA CCC (SEQ ID NO: 3) | CGCCTGGACTAACCGGC TCC (SEQ ID NO: 4) |
| 2. Cloning Gm7325 locus containing short and long isoforms | AGTGATGCTGAATCCAC CGCA (SEQ ID NO: 5) | CCAATAACAACACACTG TCCT (SEQ ID NO: 6) |
| 3. Cloning of *Gm7325*- long | ATGCCAGAAGAAAGCTG CACTG (SEQ ID NO: 7) | TCACTTCTGGGGGCCCA ATCTC (SEQ ID NO: 8) |
| 4. Cloning of *Gm7325*- short | ATGCCCGTTCCATTGCTC CCGA (SEQ ID NO: 9) | TCACTTCTGGGGGCCCA ATCTC (SEQ ID NO: 10) |
| 5. myomerger-short SYBR | CAGGAGGGCAAGAAGTT CAG (SEQ ID NO: 11) | ATGTCTTGGGAGCTCAG TCG (SEQ ID NO: 12) |
| 6. myomerger-long SYBR | ACCAGCTTTCATGCCAGA AG (SEQ ID NO: 13) | ATGTCTTGGGAGCTCAG TCG (SEQ ID NO: 14) |
| 7. myomaker SYBR | ATCGCTACCAAGAGGCG TT (SEQ ID NO: 15) | CACAGCACAGACAAACC AGG (SEQ ID NO: 16) |
| 8. Tm6sf1 SYBR | TTAGTGGTCCCTGGATGC TC (SEQ ID NO: 17) | GACGCACCAATGTGAGA AAA (SEQ ID NO: 18) |
| 9. Tspan33 SYBR | GGGGACGAGTTCTCCTTC G (SEQ ID NO: 19) | TGCTTCTGCGTGCTTCAT TAG (SEQ ID NO: 20) |
| 10. Tmem182 SYBR | GGCTCTCTTCGGAGCTTT GG (SEQ ID NO: 21) | GGTGGCTGATTGGTGTA CCAG (SEQ ID NO: 22) |
| 11. Myogenin SYBR | CTACAGGCCTTGCTCAGC TC (SEQ ID NO: 23) | GTGGGAGTTGCATTCAC TGG (SEQ ID NO: 24) |
| 12. Ckm SYBR | ACCTCCACAGCACAGAC AGA (SEQ ID NO: 25) | CAGCTTGAACTTGTTGT GGG (SEQ ID NO: 26) |
| 13. Myh4 SYBR | GCAGGACTTGGTGGACA AAC (SEQ ID NO: 27) | ACTTGGCCAGGTTGACA TTG (SEQ ID NO: 28) |
| 14. GAPDH SYBR | TGCGACTTCAACAGCAA CTC (SEQ ID NO: 29) | GCCTCTCTTGCTCAGTGT CC (SEQ ID NO: 30) |

### CRISPR-Mediated Genome Editing in C2C12 Cells

Freshly plated low passage C2C12 cells were transfected with 4µg of a modified pX458 plasmid (Addgene #48138, gift from Yueh-Chiang Hu), which contained a high fidelity Cas9, an optimized sgRNA scaffold, and an IRES-GFP cassette. The same gRNAs used to generate KO animals were used for C2C12 cells. 16 µL of Lipofectamine 2000 was used for this transfection. 5 × 10⁵ C2C12 cells were transfected in a 60 mm culture dish. Forty-eight hours after transfection GFP⁺ cells were sorted into 96 well plates using FACS. These cells were maintained in DMEM containing 20% FBS with antibiotics at subconfluent densities. The cell lines were genotyped by amplifying a 420 bp region surrounding the site of Cas9 activity using the primers used to genotype *Gm7325*^{*-*/*-*} animals.

### Cloning and viral infection

We initially cloned a region of the *Gm7325* locus, containing all genomic information for expression of myomerger-short and myomerger-long, from C57Bl6 mouse genomic DNA. We cloned myomerger-short and long coding sequences from cDNA of differentiating C2C12 cells. Cloning primers are listed in Table E1. Myomerger cDNA and genomic DNA was cloned into the retroviral vector pBabe-X using EcoRI. Myomaker and GFP retroviral plasmids have been described previously. (MILLAY et al. "Myomaker is a membrane activator of myoblast fusion and muscle formation" Nature (2013) Vol. 499, pp. 301-305.) NLS-TdTomato was subcloned from pQC-NLS-TdTomato (Addgene #37347) into the retroviral vector pMX (Cell Biolabs). Plasmids containing cDNA for Tmem182, Tspan33, and Tm6sf1 from the Mammalian Gene Collection were purchased from Open Biosystems and subcloned into pBabe-X. Ten micrograms of retroviral plasmid DNA were transfected with FuGENE 6 (Roche) into Platinum E cells (Cell Biolabs), which were plated 24 hours before transfection on a 10 cm culture dish at a density of 3-4×10⁶ cells per dish. Forty-eight hours after transfection, viral media were collected, filtered through a 0.45 µm cellulose syringe filter and mixed with polybrene (Sigma) at a final concentration of 6 µg/ml. Target cells were plated on 10 cm culture dishes at a density of 4×10⁵ cells per dish 16-18 hours before infection. Eighteen hours after infection, virus was removed, cells were washed with PBS and split into new 10 cm dishes.

### Cell fusion assays

Cells were split 18 hours after retroviral infection and split again 24-48 hours later. At the second split, cells were seeded for the fusion assay on 35-mm dishes (3-4×10⁵ cells per dish) or on 8-well Ibidi slides (2×10⁴ cells/well). Fusion was assessed 24-48 hours after seeding. For heterologous fusion, cultures of fibroblasts and myoblasts mixed at a ratio of 1:1 (1.5×10⁵ cells for each) were induced to differentiate 24 hours after seeding and fusion was assessed on day 4 of differentiation.

### RNA extraction and quantitative RT-PCR (qRT-PCR)

Total RNA was extracted from either mouse tissue or cultured cells with TRIZOL (Life Technologies) according to manufacturer's protocol. cDNA was synthesized using High-Capacity cDNA Reverse Transcription Kit (Applied Biosystems) with random primers. Gene expression was assessed using standard quantitative PCR approach with Power Sybr^{®} Green PCR mastermix (Applied Biosystems). Analysis was performed on StepOnePlus Real-Time PCR system (Applied Biosystems) with gene-specific primers (Table E1).

### Western blot analysis

Cultured cells were washed two times with ice cold PBS, scraped into a conical tube, pelleted, resuspended in lysis buffer (50 mM Tris-HCl, pH 6.8, 1mM EDTA, 2% SDS) and sonicated for a total of 15 seconds (three 5 second pulses). Skeletal muscle tissues from mice were homogenized with a bead homogenizer (TissueLyser II; Qiagen) in lysis buffer (10 mM Tris (pH 7.4), 1 mM EDTA, 1mM dithiothreitol, 0.5% Triton X-100, 2.1 mg/ml NaF) containing protease and phosphatase inhibitor cocktails (5 µl/ml; Sigma-Aldrich). Both cells and tissue lysates were centrifuged to pellet insoluble material and protein concentration was determined using Bradford protein assay. Equal amounts of protein (5 µg for cells and 20 µg for tissues) were prepared with loading buffer (1x Laemmli (Bio-Rad) with reducing agent (5% β-mercaptoethanol for cells and 100 mM DTT for tissues). Samples were heated at 37°C for 30 minutes and separated on a 20% SDS-PAGE. The gels were subsequently transferred to a PVDF membrane (Millipore), blocked in 5% milk in Tris-buffered saline/0.1% Tween-20 (TBS-T) and incubated with anti-sheep ESGP antibody (1 mg/µl; R&D Systems) overnight at 4°C. Membranes were then washed with TBS-T and incubated with Alexa-Fluor 647 donkey anti-sheep secondary antibody (1:5,000; Invitrogen). Bands were visualized using the Odyssey^{®} infrared detection system (LI-COR Biosciences). GAPDH (1:5,000; Millipore) was used as a loading control.

### Subcellular fractionation

C2C12 cells were harvested on day 2 of differentiation in ice cold hypotonic buffer (10 mM Tris-HCl pH 8, 2 mM EDTA) and lysed using a dounce homogenizer. Lysates were then centrifuged at 800 x g for 5 minutes at 4°C to separate nuclei and cell debris. That supernatant was then centrifuged at 5000 x g for 10 minutes to pellet mitochondria and ER. ER and heavy vesicles were further pelleted through centrifugation at 17,000 x g for 10 minutes. Finally, plasma membrane, light vesicles, and organelles were pelleted at 100,000 x g for 20 minutes and the supernatant from this spin was collected as the cytosolic fraction. All pellets were resuspended in lysis buffer (50 mM Tris-HCl, pH 6.8, 1mM EDTA, 2% SDS) at volumes equal to the supernatant. Eight µl of each fraction was separated by SDS-PAGE and analyzed for presence of myomerger, caveolin-3, and tubulin. Caveolin-3 antibody (BD Transduction Laboratories #610421) was used at 1:6700 and tubulin (Santa Cruz #SC-8035) at 1:50.

### Immunocytochemistry

Cultured cells were rinsed with PBS and fixed in 4% paraformaldehyde (PFA)/PBS for 15 minutes at room temperature. Cells were subsequently permeabilized and blocked in 0.01% Triton X-100/5% donkey serum/PBS for one hour at room temperature. Primary antibody diluted in permeabilization/blocking buffer was incubated overnight. Cells were then washed with PBS and incubated with secondary Alexa-Fluor antibodies (1:250) for 1 hour. A myomaker custom antibody was generated through YenZym Antibodies LLC. Rabbits were immunized with amino acids #137-152 of mouse myomaker (MKEKKGLYPDKSIYTQ (SEQ ID NO: 31)) after conjugation to KLH. We used antigen-specific affinity purified products at a concentration of 4.3 µg/mL for immunostaining. Esgp (myomerger) antibody was used at a concentration of 1 µg/mL. Anti-mouse myosin (my32, MA5-11748, ThermoFisher Scientific) antibody was used at 1:100. Hoechst 33342 solution (ThermoFisher Scientific) was used to stain nuclei. Cells were imaged using Nikon A1R+ confocal on a FN1 microscope (35 mm dishes) or Nikon A1R confocal on Eclipse T1 inverted microscope (Ibidi slides).

### Histology and immunohistochemistry

For cryosections, embryos were dissected, fixed in 4% PFA/PBS overnight at 4°C, washed in PBS, incubated in 30% sucrose/PBS overnight and then in 1:1 mix of optimal cutting temperature (O.C.T.) formulation and 30% sucrose prior to embedding in O.C.T. Sections were cut at 10 µm and then permeabilized with 0.2% Triton X-100/PBS, blocked with 1% BSA/1% heat inactivated goat serum/0.025% Tween20/PBS and incubated with primary antibody overnight. Anti-mouse myosin (my32, MA5-11748, ThermoFisher Scientific) antibody was used at 1:100, whereas myogenin (F5D, Developmental Hybridomas) was used at a concentration of 2.56 µg/mL. Secondary goat anti-mouse IgG1-488 Alexa-Fluor antibody (Invitrogen) was incubated at a dilution of 1:250 for 1 hour. Slides were mounted with VectaShield containing DAPI (Vector Laboratories) and visualized using Nikon A1R confocal on Eclipse T1 inverted microscope. Images were analyzed with Fiji.

### Statistical analysis

For quantitation of cell fusion in FIGs. 1H and 2B, cells with 3 or more nuclei were considered syncytial cells. The number of nuclei in syncytial cells and total number of nuclei were manually counted. To quantify fusion between myomaker⁺ myomerger⁺ GFP⁺ fibroblasts with either myomaker⁺ NLS-Tom⁺ or myomerger⁺ NLS-Tom⁺ fibroblasts (FIG. 2A), we calculated the percentage of GFP⁺NLS-Tom⁺ syncytial cells. In FIG. 2D, the number of myosin⁺ myotubes (myosin structures with 3 or more nuclei) and GFP⁺ myosin⁺ myotubes were manually counted. The differentiation index (FIG. 4E) was calculated as the percentage of nuclei in myosin⁺ cells, and the fusion index (FIG. 4F) as the percentage of myosin⁺ cells with the indicated number of nuclei. For FIG. 4E, fusion was expressed as the percentage of myosin⁺ cells with ≥ 3 nuclei. Quantitative data sets are presented as means ± SEM. For each quantitation, at least 3 independent experiments were performed in duplicate and 4-6 fields were randomly chosen for imaging. Histological analysis of embryos was performed on 3-4 embryos per genotype per time point. Multiple histological levels within each muscle were examined. The data were analyzed using an unpaired Student's t-test (two-tailed) with GraphPad Prism 6 software. A value of *P* < 0.05 was considered statistically significant.

### Results

### Identification and fusogenic activity of myomerger.

To uncover potential fusion factors, we compared genes induced by expression of MyoD to their level of expression in 10T ½ fibroblasts. Of the top 100 MyoD-regulated genes not expressed in fibroblasts (data not shown), we eliminated genes not likely to be directly involved in fusion (transcription factors, sarcomeric and metabolic genes) and focused on genes with transmembrane domains. This analysis yielded the following five genes: *Tmem182*, *Gm7325*, *Cdh15*, *Tspan33*, and *Tm6sf1*, however *Cdh15* was omitted from further analysis because it has previously been shown as not necessary for myoblast fusion or muscle formation. We retrovirally expressed each gene in myomaker⁺ GFP⁺ fibroblasts and assayed for fusion. Appropriate expression in fibroblasts was verified through quantitative reverse transcription polymerase chain reaction (qRT-PCR) analysis (FIG. 1A). We observed mainly mono-nucleated GFP⁺ cells in all cultures except when *Gm7325* was expressed where widespread multi-nucleated cells were present (FIG. 1B). Based on the ability of *Gm7325* to induce fusion of myomaker⁺ fibroblasts and the observations described below we named the protein myomerger.

Multiple *Gm7325* transcripts are annotated in the University of California, Santa Cruz, mouse genome. The shorter transcript contains a single exon and yields a protein with 84 amino acids. In contrast, the longer transcript utilizes an upstream exon with an alternative start site and results in a protein of 108 amino acids (FIG. 1C). The single coding exon of the short transcript is conserved in other mammalian genomes, including humans, while the upstream alternative exon leading to the longer transcript is not highly conserved (FIG. 1D). For the initial screen, we cloned the *Gm7325* locus into a retroviral vector, allowing normal splicing and expression of both short and long transcripts. Transduction of myomaker⁺ fibroblasts with either myomerger-short (S) or myomerger-long (L) induced formation of multi-nucleated cells, indicating both proteins are sufficient for fusion (FIG. 1E). Additionally, myomerger and myomaker together induced fusion of 3T3 fibroblasts and MSCs (FIG. 1F), suggesting these two genes could activate fusion in a multitude of cell types.

Given that multi-nucleated cells could arise from fusion or replication associated with incomplete cytokinesis, we designed a system to validate that multi-nucleated cells observed in fibroblasts expressing both myomerger and myomaker were generated through fusion. We engineered two fibroblast cell lines that both express myomaker, with one expressing GFP and the other expressing nuclear-localized TdTomato (NLS-Tom). Myomaker⁺ GFP⁺ and myomaker⁺ NLS-Tom⁺ fibroblasts were infected with a myomerger retrovirus or a control empty retrovirus, mixed, and fusion was assessed (FIG. 1G). We observed cells with multiple nuclei containing both GFP and NLS-Tom in fibroblasts expressing myomaker and myomerger indicating fusion (FIG. 1G). Quantification of fusion revealed approximately 20% of nuclei were contained in syncytia in cultures where fibroblasts were expressing both myomaker and myomerger (FIG. 1H). These results confirm that the observed syncytial cells are formed through fusion and that expression of myomaker and myomerger is sufficient to confer fusogenicity in non-fusogenic fibroblasts.

We also sought to determine the cell biology of fusion induced by myomaker and myomerger. We mixed myomaker⁺ myomerger⁺ GFP⁺ fibroblasts with NLS-Tom⁺ fibroblasts expressing myomaker or myomerger (FIG. 2A). Here we observed fusion of myomaker⁺ myomerger⁺ GFP⁺ fibroblasts with myomaker⁺ NLS-Tom⁺ but not myomerger⁺ NLS-Tom⁺ fibroblasts (FIG. 2A). We detected 10% of nuclei in syncytia (FIG. 2A), lower than the fusion observed when both cells express myomaker and myomerger (FIG. 1H) suggesting an enhanced fusogenic efficiency when cells express both proteins. Nonetheless, these data indicate that myomerger does not appear to be sufficient for fusion in the absence of myomaker. This heterotypic nature of fibroblast fusion (where myomaker appears to be required on both cells and myomerger only appears to be required on one cell) are consistent with our previously reported heterologous fusion system between myoblasts and fibroblasts. In that system, myomaker⁺ fibroblasts that do not express myomerger fused with muscle cells, which express both myomaker and myomerger. To confirm this concept, we utilized our heterologous fusion system where fibroblasts were infected with GFP and either empty, myomaker, or myomerger retrovirus, and then mixed with C2C12 myoblasts (FIG. 2C). In this assay, fusion is detected through co-localization of GFP (fibroblasts) with myosin⁺ myotubes. Compared to empty-infected GFP⁺ fibroblasts, we detected an increase in fusion between myosin⁺ cells with either myomaker⁺ GFP⁺ fibroblasts or myomerger⁺ GFP⁺ fibroblasts (FIG. 2C and FIG. 2D). However, quantification of myosin⁺ GFP⁺ cells revealed that myomerger did not drive the fusion of fibroblasts with muscle cells to the levels observed with myomaker (FIG. 2D). These data confirm that myomaker appears to be required in both fusing cells for *in vitro* fusion, while myomerger is appears to be required in one fusing cell.

### Myomerger is muscle-specific and associates with membranes

We interrogated *Gm7325* expression pattern more thoroughly. We performed qRT-PCR on multiple tissues from postnatal (P) day 5 mice with primers to distinguish the two potential mouse transcripts (FIG. 3A). In neonatal tissues, we detected expression of both myomerger transcripts only in skeletal muscle (FIG. 3B). Despite the evidence of two myomerger transcripts, immunoblot analysis of skeletal muscle lysates from P5 mice using a commercially available antibody identified a single band at approximately 12 kDa. This band was absent in P28 lysates indicating that myomerger is downregulated after neonatal development (FIG. 3C). Skeletal muscle exhibits a robust ability to regenerate due to the presence of muscle stem cells, also known as satellite cells. We analyzed expression of myomerger in *mdx*^{4cv} mice, which is a mouse model of muscular dystrophy that leads to chronic cycles of degeneration and regeneration. (DURBEEJ et al. "Muscular dystrophies involving the dystrophin-glycoprotein complex: An overview of current mouse models" (2002) Curr Opin Genet Dev, Vol. 12, pp. 349-361.) Myomerger expression was detected in diaphragm lysates from *mdx*^{4cv} mice, but not control diaphragms (FIG. 3D). Finally, we analyzed expression of myomerger in a model of skeletal muscle overload-induced (MOV) hypertrophy and observed up-regulation (FIG. 3E). Collectively, these data demonstrate that myomerger is expressed during development and is induced during adult myogenesis.

We next sought to determine if myomerger is regulated as myoblasts differentiate. In C2C12 cells, both *Gm7325* transcripts were significantly elevated during differentiation (FIG. 3F). Similarly, myomerger protein levels were low in proliferating myoblasts (day 0), but increased upon differentiation with expression maintained during myoblast differentiation and fusion into myotubes (Fig. 3G). The short mouse myomerger protein, but not the long form, is highly conserved among vertebrate species (FIG. 3H). After transduction of C2C12 cells with empty, myomerger-S, or myomerger-L, we detected an increased upper band in cells expressing either myomerger-S or myomerger-L that co-migrated with the 12 kDa endogenous protein in the empty-infected C2C12 cells (FIG. 3I). A lower band was identified exclusively in myomerger-S lysates suggesting that complex mRNA or post-translational processing results in the endogenous single 12 kDa band observed in WT C2C12 cells and muscle homogenates. Both myomerger proteins harbor a hydrophobic region close to the N-terminus, where computational analysis of this region appears to indicate a signal peptide or transmembrane domain (FIG. 3J). Both variants were found to confer fusogenicity. To understand subcellular localization, we fractionated C2C12 cells on day 2 of differentiation and identified myomerger in membrane fractions containing caveolin-3, a protein known to associate with both heavy and light vesicles (FIG. 3K). Immunostaining of fibroblasts expressing myomerger-S or myomerger-L shows that both proteins exhibit similar diffuse and vesicular localization (FIG. 3L). Thus, myomerger associates with membrane compartments which is consistent with its ability to induce fusion.

### Role of myomerger in myoblast fusion

The ability of myomerger to induce fusion of myomaker-fibroblasts, and its muscle-specific expression in the mouse, suggests that myomerger may play a role during myogenesis. To begin to decipher the role of myomerger in myogenesis, we evaluated its function during myoblast differentiation. We utilized CRISPR/Cas9 genome editing to disrupt myomerger in C2C12 myoblasts. Two guide RNAs (gRNA) were designed to target the largest exon of *Gm7325,* which resulted in a 166 base pair deletion thereby disrupting both mouse transcripts (FIG. 4A). C2C12 cells were transfected with a plasmid containing Cas9 with an IRES-GFP and myomerger gRNAs, or transfected with only Cas9-IRES-GFP as a control. Flow cytometry of GFP⁺ cells followed by genotyping through PCR analysis revealed disruption of the myomerger locus (FIG. 4B). Myomerger was not detectable in myomerger KO C2C12 cells confirming efficient disruption of the locus (FIG. 4C). Control and myomerger KO C2C12 cells were then analyzed for their ability to differentiate and form myotubes. WT myoblasts differentiated, as indicated by myosin⁺ cells, and fused to form multi-nucleated myotubes (FIG. 4D). In contrast, myomerger KO C2C12 cells exhibited the ability to differentiate but lacked fusogenic activity to form myotubes (FIG. 4D). Indeed, quantification of the differentiation index revealed no difference in the percentage of myosin⁺ cells between WT and myomerger KO cultures (FIG. 4E). Additionally, quantification of fusion demonstrated that myomerger KO myosin⁺ cells remain mono-nucleated while WT cells fuse (FIG. 4F). qRT-PCR analysis for the myogenic genes *Myogenin*, *Myh4*, *Ckm*, and *Tmem8c* (myomaker) further indicated that myomerger KO myoblasts activate the differentiation program (FIG. 4G). Interestingly, myogenic transcripts were elevated in myomerger KO cells potentially suggesting a feedback mechanism by which non-fusogenic cells attempt to further differentiate (FIG. 4G). Infection of myomerger KO C2C12 cells with either myomerger-S or myomerger-L rescued the fusion defect demonstrating that the phenotype in these cells is specifically due to the loss of myomerger and not an off-target effect of Cas9 (FIG. 4H). Western blot analysis from these lysates shows re-expression of myomerger in KO cells (FIG. 4I). As a potential mechanism for the lack of fusion in myomerger KO myocytes, we examined expression and localization of myomaker. On day 2 of differentiation, myomerger KO cells exhibited normal expression and localization of myomaker (FIG. 5A). Moreover, we did not detect widespread co-localization between myomaker and myomerger suggesting that myomerger does not directly regulate myomaker distribution (FIG. 5B). These data reveal that myomerger is used in myoblast fusion *in vitro* through a mechanism that does not involve regulation of myomaker levels or localization.

### Role of myomerger in muscle formation in vivo

To examine the function of myomerger *in vivo*, we disrupted exon 3 using the same CRISPR/Cas9 strategy described for C2C12 myoblasts. Injection of Cas9 and myomerger gRNAs into blastocysts resulted in lethality of 9 of the 10 F₀ pups, suggesting that the high efficiency of Cas9 lead to homozygous deletion of myomerger. The one remaining pup was heterozygous for myomerger (FIG. 6A) and sequencing of the mutant PCR product revealed the presence of the same mutation as was achieved in C2C12 cells. The heterozygous founder was mated to WT mice for multiple generations, which controlled for potential off-target effects given that we only selected pups with the *Gm7325* mutation. Heterozygous mice from these litters were then crossed to generate *Gm7325*^{*-*/*-*} mice. We failed to observe any *Gm7325*^{*-*/*-*} mice upon genotyping at P7 suggesting that myomerger is essential for life. Indeed, E17.5 *Gm7325*^{*-*/*-*} embryos exhibited minimal skeletal muscle upon gross examination (Fig. 6B). Specifically, bones of the limbs and rib cage were noticeable due to a scarcity of surrounding muscle as observed in WT embryos. Myomerger KO mice also displayed a hunched appearance with elongated snouts, hallmark characteristics of embryos with improper muscle formation (FIG. 6B). Detection of myomerger by western blot of WT and *Gm7325*^{*-*/*-*} tongues showed elimination of myomerger protein in KO samples (FIG. 6C). E15.5 forelimb sections showed that myomerger KO myoblasts express myogenin indicating that specification and differentiation were activated despite loss of myomerger (FIG. 6D). Moreover, histological analysis of multiple muscle groups at E15.5 revealed the presence of myosin⁺ muscle cells and sarcomeric structures in myomerger KO mice, (FIG. 6E and FIG. 6F). While multi-nucleated myofibers were present in WT mice, these structures were not readily detected in myomerger KO mice indicating that genetic loss of myomerger renders myocytes non-fusogenic (FIG. 6E and FIG. 6F). Analysis of forelimbs from E17.5 WT and myomerger KO embryos confirm that myomerger KO myoblasts are unable to properly fuse, although we did detect myocytes with two nuclei (FIG. 6G, FIG. 6H, and FIG. 6I). These results, together with our *in vitro* analysis, reveal that myomerger is used in muscle formation during mammalian development through regulation of myoblast fusion.

### Discussion

In summary, we report the discovery of an additional muscle-specific factor used in myoblast fusion and developmental myogenesis. While myomaker and myomerger appear to be used in muscle formation, E17.5 myomerger KO embryos grossly exhibit more myocytes compared to embryos lacking myomaker suggesting that these two myoblast fusion proteins may have distinct functions. We did not detect robust co-localization indicating that there is no physical interaction between myomaker and myomerger or that any potential interaction is transient or at discrete cellular locations.

Our data from the cell mixing experiments reveal that myomaker appears to be necessary in both fusing cells while myomerger only appears to be required in one fusing cell. This indicates that, for reconstitution of cell fusion, both fusing cells must become fusion competent (amenable to fuse), while only one cell needs to become fusogenic to allow syncytial formation. With this concept in mind, our data suggest that myomaker allows the cell to become fusion competent, whereas myomerger confers fusogencity. The cell mixing experiments also indicate that myomerger may require myomaker activity for fusogenic function because myomerger-expressing fibroblasts do not fuse to fibroblasts expressing both myomaker and myomerger. Without wishing to bound by theory, these data potentially suggest that myomerger acts downstream of myomaker, where myomaker acts as an initiator of fusion and myomerger executes the final steps to drive syncytial formation.

Without wishing to bound by theory, the distinct consequences of myomaker and myomerger expression in fibroblasts are consistent with the idea that the two proteins regulate different aspects of fusion. Membrane fusion appears to be a complex process that often includes membrane apposition and tethering, mixing of the outer membranes (hemifusion), pore formation, and pore expansion. Classical viral fusogens, as well as Eff-1, are large proteins with long ectodomains that are able to accomplish all of the steps necessary for fusion. The discovery of myomerger as an additional myoblast fusion factor could indicate that in higher organisms these multiple functions of viral proteins have been delegated to different myocyte proteins. Without wishing to bound by theory, this evolutionary strategy, at least in the case of muscle fusion, could provide more regulatory control to ascertain that cells are compatible for fusion.

Without wishing to bound by theory, the myomerger association with membranes could indicate that it functions to alter membrane dynamics that overcomes the thermodynamic barriers for fusion. Without wishing to bound by theory, myomerger activation of fusion through cytoskeletal alterations would be consistent with induction of fusogencity, as cytoskeletal alterations provide the necessary tension to induce membrane fusion in various systems.

The headings used in the disclosure are not meant to suggest that all disclosure relating to the heading is found within the section that starts with that heading. Disclosure for any subject may be found throughout the specification.

It is noted that terms like "preferably," "commonly," and "typically" are not used herein to limit the scope of the claimed invention or to imply that certain features are critical, essential, or even important to the structure or function of the claimed invention. Rather, these terms are merely intended to highlight alternative or additional features that may or may not be utilized in a particular embodiment of the present invention.

As used in the disclosure, "a" or "an" means one or more than one, unless otherwise specified. As used in the claims, when used in conjunction with the word "comprising" the words "a" or "an" means one or more than one, unless otherwise specified. As used in the disclosure or claims, "another" means at least a second or more, unless otherwise specified. As used in the disclosure, the phrases "such as", "for example", and "e.g." mean "for example, but not limited to" in that the list following the term ("such as", "for example", or "e.g.") provides some examples but the list is not necessarily a fully inclusive list. The word "comprising" means that the items following the word "comprising" may include additional unrecited elements or steps; that is, "comprising" does not exclude additional unrecited steps or elements.

Unless otherwise indicated, all numbers expressing quantities of ingredients, properties such as reaction conditions, and so forth used in the specification and claims are to be understood as being modified in all instances by the term "about". Accordingly, unless indicated to the contrary, the numerical parameters set forth in this specification and claims are approximations that can vary depending upon the desired properties sought to be obtained by the presently-disclosed subject matter.

As used herein, the term "about," when referring to a value or to an amount of mass, weight, time, volume, concentration or percentage is meant to encompass variations of in some embodiments ±20%, in some embodiments ±10%, in some embodiments ±5%, in some embodiments ±1%, in some embodiments ±0.5%, and in some embodiments ±0.1% from the specified amount, as such variations are appropriate to perform the disclosed method.

Detailed descriptions of one or more embodiments are provided herein. It is to be understood, however, that the present invention may be embodied in various forms. Therefore, specific details disclosed herein (even if designated as preferred or advantageous) are not to be interpreted as limiting, but rather are to be used as an illustrative basis for the claims and as a representative basis for teaching one skilled in the art to employ the present invention in any appropriate manner. Indeed, various modifications of the invention in addition to those described herein will become apparent to those skilled in the art from the foregoing description and the accompanying figures. Such modifications are intended to fall within the scope of the appended claims.

Aspects of the invention will now be described with reference to the following numbered clauses:
1. A polypeptide comprising a myomerger polypeptide.
2. The polypeptide of clause 1, wherein the polypeptide is not a wt-myomerger polypeptide.
3. The polypeptide of clause 1 or clause 2, wherein the polypeptide is a mutant-myomerger polypeptide.
4. The polypeptide of any of clauses 1-3, wherein the polypeptide comprises at least one amino acid modification relative to a wt-myomerger polypeptide.
5. The polypeptide of any of clauses 1-4, wherein the polypeptide comprises at least one amino acid modification relative to a wt-myomerger polypeptide and the at least one amino acid modification is an insertion, a deletion, or a substitution.
6. The polypeptide of any of clauses 1-5, wherein the polypeptide is selected from the group consisting of SEQ ID NO: 32, SEQ ID NO: 33, SEQ ID NO: 34, SEQ ID NO: 35, SEQ ID NO: 36, SEQ ID NO: 37, and SEQ ID NO: 38.
7. The polypeptide of any of clauses 1-6, wherein the polypeptide is not SEQ ID NO: 32, SEQ ID NO: 33, SEQ ID NO: 34, SEQ ID NO: 35, SEQ ID NO: 36, SEQ ID NO: 37, or SEQ ID NO: 38.
8. The polypeptide of any of clauses 1-7, wherein the wt-myomerger polypeptide is selected from the group consisting of SEQ ID NO: 32, SEQ ID NO: 33, SEQ ID NO: 34, SEQ ID NO: 35, SEQ ID NO: 36, SEQ ID NO: 37, and SEQ ID NO: 38.
9. The polypeptide of any of clauses 1-8, wherein the polypeptide sequence has at least an 80% sequence identity to a wt-myomerger polypeptide.
10. The polypeptide of any of clauses 1-9, wherein the polypeptide sequence has at least a 90% sequence identity to a wt-myomerger polypeptide.
11. A myomerger nucleic acid molecule encoding the polypeptide of any of clauses 1-10.
12. The myomerger nucleic acid molecule of clause 11, wherein the myomerger nucleic acid sequence has at least an 80% identity to one or more of SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, or SEQ ID NO: 49.
13. The myomerger nucleic acid molecule of clause 11 or clause 12, wherein the myomerger nucleic acid sequence encoding the polypeptide is selected from the group consisting of SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, and SEQ ID NO: 49.
14. The myomerger nucleic acid molecule of any of clauses 11-13, wherein the myomerger nucleic acid sequence is a cDNA, or the myomerger nucleic acid sequence is not SEQ ID NO: 46, SEQ ID NO: 47, SEQ ID NO: 48, or SEQ ID NO: 49.
15. The myomerger nucleic acid molecule of any of clauses 11-14, wherein the myomerger nucleic acid molecule is in a cell, an insect cell, a mammalian cell, a human cell, or an sf9 insect cell.
16. The myomerger nucleic acid molecule of any of clauses 11-15, wherein the myomerger nucleic acid molecule is in a non-muscle cell, a muscle cell, a fibroblast, a mesenchymal stem cell (MSC), a hematopoietic stem cell, a blood cell, a bone marrow cell, or an adipose stem cell.
17. The myomerger nucleic acid molecule of any of clauses 11-16, wherein the myomerger nucleic acid molecule is in a modified cell.
18. The myomerger nucleic acid molecule of any of clauses 11-17, wherein the myomerger nucleic acid molecule is included in a vector, a viral vector, or a plasmid.
19. A myomerger vector comprising any of myomerger nucleic acid molecules of clauses 11-18.
20. A modified cell comprising the myomerger nucleic acid molecule of any of clauses 11-18 or the myomerger vector of clause 19.
21. The modified cell of clause 20, wherein the myomerger nucleic acid molecule is exogenous.
22. The modified cell of clause 20 or clause 21, wherein the modified cell further comprises a myomaker nucleic acid molecule, where optionally at least one modification of the modified cell was the addition of the myomaker nucleic acid molecule.
23. The modified cell of any of clauses 20-22, wherein the modified cell is an insect cell, a mammalian cell, or a human cell.
24. The modified cell of any of clauses 20-23, wherein the modified cell is a non-muscle cell, a muscle cell, a fibroblast, a mesenchymal stem cell (MSC), a hematopoietic stem cell, a blood cell, a bone marrow cell, or an adipose stem cell.
25. The modified cell of any of clauses 20-24, wherein at least part of the myomerger nucleic acid molecule is under control of a promoter.
26. The modified cell of clause 25, wherein the promotor is a constitutive promoter, a synthetic promoter, an inducible promotor, a tissue specific promoter, a chemically regulated promotor, or a physically regulated promoter.
27. The modified cell of any of clauses 20-26, wherein the modified cell comprises a myomerger polypeptide, a myomaker polypeptide, or both, and prior to modification the modified cell did not comprise a myomaker polypeptide, a myomerger polypeptide, or both.
28. The modified cell of any of clauses 20-27, wherein the modified cell comprises a myomaker nucleic acid molecule.
29. The modified cell of clause 28, wherein the myomaker nucleic acid molecule is exogenous.
30. The modified cell of any of clauses 20-29, wherein the modification to the modified cell comprises one or more of (a) diminishing the effect of a first nucleic acid molecule, (b) addition of a second nucleic acid molecule encoding a myomaker polypeptide, or (c) addition of a third nucleic acid molecule encoding a myomerger polypeptide.
31. The modified cell of any of clauses 20-30, wherein the modified cell (a) is a cell that has a diminished effect of a first nucleic acid molecule, (b) a cell that has a diminished effect of a first myomerger nucleic acid molecule, (c) a cell that has a diminished effect of a first myomaker nucleic acid molecule, (d) a cell that has an addition of a second myomerger nucleic acid molecule, or (e) a cell that has an addition of a third myomerger nucleic acid molecule and that has an addition of a second myomaker nucleic acid molecule, or (f) combinations thereof.
32. A method of preparing the modified cell of any of clauses 20-31 comprising adding a myomerger nucleic acid molecule to a first cell.
33. The method of clause 32, wherein the first cell is a cell that has been previously modified.
34. A composition comprising a polypeptide of any of clauses 1-10, a myomerger nucleic acid molecule of any of clauses 11-18, a myomerger vector of clause 19, or a modified cell of any of clauses 20-31.
35. The composition of clause 34, wherein the amount of the polypeptide, the myomerger nucleic acid molecule, the myomerger vector, or the modified cell is from about 0.0001% (by weight total composition) to about 99%.
36. A pharmaceutical composition comprising a polypeptide of any of clauses 1-10, a myomerger nucleic acid molecule of any of clauses 11-18, a myomerger vector of clause 19, or a modified cell of any of clauses 20-31.
37. The pharmaceutical composition of clause 36, wherein the amount of the polypeptide, the myomerger nucleic acid molecule, the myomerger vector, or the modified cell is from about 0.0001% (by weight total composition) to about 50%.
38. A method for fusing two or more cells comprising
   - contacting a first cell with a second cell to form a third cell;
   wherein
   - the first cell is a modified cell of any of clauses 20-31 comprising a first myomerger polypeptide and a first myomaker polypeptide;
   - the second cell comprises a second myomaker polypeptide and optionally comprises a second myomerger polypeptide; and
   - the third cell is a multinucleated cell.
39. The method of clause 38, wherein the second cell comprises the second myomerger polypeptide.
40. The method of clause 38 or clause 39, wherein the first cell is a non-muscle cell, the second cell is a non-muscle cell, or both.
41. The method of any of clauses 38-40, wherein the first cell is a non-muscle cell and the second cell is a muscle cell.
42. The method of any of clauses 38-41, wherein the second cell is an isolated muscle cell.
43. The method of any of clauses 38-42, wherein the second cell is a myoblast.
44. The method of any of clauses 38-43, wherein the second cell is a muscle cell and is part of a muscle or muscle tissue.
45. The method of any of clauses 38-44, wherein the contacting occurs *in vitro.*
46. The method of any of clauses 38-45, wherein the contacting occurs *in vivo.*
47. A method for delivering a gene of interest comprising
   - contacting a first cell with a second cell, which fuse to form a third cell;
   wherein
   - the first cell is a modified cell of any of clauses 20-31 comprising a first myomerger polypeptide, a first myomaker polypeptide, and a gene of interest;
   - the second cell comprises a second myomaker polypeptide and optionally comprises a second myomerger polypeptide; and
   - the third cell is a multinucleated cell and the gene of interest is delivered to the third cell upon fusion of the first cell with the second cell.
48. The method of clause 47, wherein the second cell comprises the second myomerger polypeptide.
49. The method of clause 47 or clause 48, wherein the first cell is a non-muscle cell, the second cell is a non-muscle cell, or both.
50. The method of any of clauses 47-49, wherein the first cell is a non-muscle cell and the second cell is a muscle cell.
51. The method of any of clauses 47-50, wherein the second cell is an isolated muscle cell.
52. The method of any of clauses 47-51, wherein the second cell is a myoblast.
53. The method of any of clauses 47-52, wherein the second cell is a muscle cell and is part of a muscle or muscle tissue.
54. The method of any of clauses 47-53, wherein the contacting occurs *in vitro.*
55. The method of any of clauses 47-54, wherein the contacting occurs *in vivo.*
56. The method of any of clauses 47-55, wherein the contacting occurs *ex vivo* and the method further comprises implanting the third cell in an animal.
57. The method of any of clauses 47-56, wherein the second cell underexpresses the gene of interest, does not express the gene of interest, or expresses a defective version of the gene of interest.
58. The method of any of clauses 47-57, wherein the delivery comprises an injection comprising the first cell, the second cell, or both, or the delivery comprises an intramuscular injection comprising the first cell, the second cell, or both.
59. The method of any of clauses 47-58, wherein the delivery further comprises one or more of the contacting steps.

## Claims

1. A third cell for use in a method for delivering a gene of interest to an animal for treatment of a disease, the method comprising:
- contacting *ex vivo* a first cell with a second cell, which fuse to form the third cell;
wherein
- the first cell is a modified non-muscle cell comprising a myomerger nucleic acid, a first myomerger polypeptide, a first myomaker polypeptide, and a gene of interest;
- the second cell comprises a second myomaker polypeptide and optionally comprises a second myomerger polypeptide;
- the third cell is a multinucleated cell and the gene of interest is delivered to the third cell upon fusion of the first cell with the second cell;
- the myomerger nucleic acid encodes a polypeptide comprising the first myomerger polypeptide and has at least 80% identity to one or more of SEQ ID NOs: 39 to 49; and
- the first myomerger polypeptide sequence and the second myomerger polypeptide sequence each have at least an 80%, optionally at least a 90%, identity to one or more of SEQ ID NOs: 32 to 38; and
- implanting, injecting, or grafting the third cell in the animal.

2. A pharmaceutical composition for use in the treatment of a disease, the composition comprising:
- a first cell that is a modified non-muscle cell comprising a myomerger nucleic acid, a first myomerger polypeptide, a first myomaker polypeptide, and a gene of interest; and
- a second cell comprising a second myomaker polypeptide and optionally comprising a second myomerger polypeptide;
wherein
- the myomerger nucleic acid encodes a polypeptide comprising the first myomerger polypeptide and has at least 80% identity to one or more of SEQ ID NOs: 39 to 49; and
- the first myomerger polypeptide sequence and the second myomerger polypeptide sequence each have at least an 80%, optionally at least a 90%, identity to one or more of SEQ ID NOs: 32 to 38.

3. The pharmaceutical composition for use of claim 2, wherein the first cell is present in an amount of from about 0.0001% to about 50% (by weight of the total composition).

4. The pharmaceutical composition for use of claim 2 or 3, further comprising boiled water, distilled water, filtered water, pyrogen-free water, or water with chloroform.

5. The third cell for use of claim 1 or the pharmaceutical composition for use of any of claims 2-4, wherein the non-muscle cell is a stem cell selected from the group consisting of mesenchymal stem cells (MSCs), hematopoietic stem cells, and adipose stem cells.

6. The third cell for use of claim 1 or 5 or the pharmaceutical composition for use of any of claims 2-5, wherein the disease is selected from the group consisting of myopathy, muscular dystrophy, amyotropic lateral sclerosis, glycogen storage disease type II, rhabdomyosarcoma, sarcopenia, and cancer.

7. An *in vitro* method for fusing two or more cells, the method comprising:
- contacting *in vitro* a first cell with a second cell to form a third cell;
wherein
- the first cell is a modified non-muscle cell comprising a myomerger nucleic acid, a first myomerger polypeptide, and a first myomaker polypeptide;
- the second cell comprises a second myomaker polypeptide and optionally comprises a second myomerger polypeptide;
- the third cell is a multinucleated cell;
- the myomerger nucleic acid encodes a polypeptide comprising the first myomerger polypeptide and has at least 80% identity to one or more of SEQ ID NOs: 39 to 49; and
- the first myomerger polypeptide sequence and the second myomerger polypeptide sequence each have at least an 80%, optionally at least a 90%, identity to one or more of SEQ ID NOs: 32 to 38.

8. The *in vitro* method of claim 7, wherein the non-muscle cell is a stem cell selected from the group consisting of mesenchymal stem cells (MSCs), hematopoietic stem cells, and adipose stem cells.

9. The *in vitro* method of claim 7 or 8, wherein the second cell (i) comprises the second myomerger polypeptide and/or (ii) is a muscle cell or a myoblast.

10. An *in vitro* method for delivering a gene of interest, the method comprising:
- contacting *in vitro* a first cell with a second cell, which fuse to form a third cell;
wherein
- the first cell is a modified non-muscle cell comprising a myomerger nucleic acid, a first myomerger polypeptide, a first myomaker polypeptide, and a gene of interest;
- the second cell comprises a second myomaker polypeptide and optionally comprises a second myomerger polypeptide;
- the third cell is a multinucleated cell and the gene of interest is delivered to the third cell upon fusion of the first cell with the second cell;
- the myomerger nucleic acid encodes a polypeptide comprising the first myomerger polypeptide and has at least 80% identity to one or more of SEQ ID NOs: 39 to 49; and
- the first myomerger polypeptide sequence and the second myomerger polypeptide sequence each have at least an 80%, optionally at least a 90%, identity to one or more of SEQ ID NOs: 32 to 38.

11. The *in vitro* method of claim 10, wherein the non-muscle cell is a stem cell selected from the group consisting of mesenchymal stem cells (MSCs), hematopoietic stem cells, and adipose stem cells.

12. The *in vitro* method of claim 10 or 11, wherein the second cell (i) comprises the second myomerger polypeptide and/or (ii) is a muscle cell or a myoblast.

13. The *in vitro* method of any of claims 10-12, wherein the contacting occurs *ex vivo.*

14. The *in vitro* method of any of claims 10-13, wherein the second cell underexpresses the gene of interest, does not express the gene of interest, or expresses a defective version of the gene of interest.

15. The third cell for use of claim 1, 5, or 6, the pharmaceutical composition for use of any of claims 2-6, or the *in vitro* method of any of claims 10-14, wherein the gene of interest encodes a therapeutic polypeptide, a therapeutic protein, or a therapeutic oligopeptide, or wherein the gene of interest is a genomic DNA or cDNA.
